(19) Europäisches Patentamt / European Patent Office / Office européen des brevets

(11) **EP 4 549 418 A1**

(12) **EUROPEAN PATENT APPLICATION**
published in accordance with Art. 153(4) EPC

(43) Date of publication:
07.05.2025   Bulletin 2025/19

(21) Application number: 23831277.1

(22) Date of filing: 22.06.2023

(51) International Patent Classification (IPC):
$C07C\ 4/22^{(2006.01)}$       $B29B\ 17/02^{(2006.01)}$
$C07C\ 1/20^{(2006.01)}$       $C07C\ 11/02^{(2006.01)}$
$C07C\ 11/12^{(2006.01)}$      $C08F\ 10/00^{(2006.01)}$
$C08F\ 22/00^{(2006.01)}$      $C08F\ 36/00^{(2006.01)}$
$C08J\ 11/12^{(2006.01)}$      $C08J\ 11/16^{(2006.01)}$

(52) Cooperative Patent Classification (CPC):
B29B 17/02; C07C 1/20; C07C 4/22; C07C 11/02;
C07C 11/12; C08F 10/00; C08F 22/00; C08F 36/00;
C08J 11/12; C08J 11/16; Y02W 30/62

(86) International application number:
PCT/JP2023/023215

(87) International publication number:
WO 2024/004833 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.06.2022  JP 2022106346

(71) Applicant: Sumitomo Chemical Company, Limited
Tokyo 103-6020 (JP)

(72) Inventor: KAWABATA, Tomonori
Ichihara-shi, Chiba 299-0195 (JP)

(74) Representative: Plasseraud IP
104 Rue de Richelieu
CS92104
75080 Paris Cedex 02 (FR)

(54) **METHOD FOR PRODUCING C2-C8 UNSATURATED HYDROCARBON, METHOD FOR PRODUCING C2-C8 UNSATURATED HYDROCARBON MIXTURE, METHOD FOR PRODUCING OLEFIN-BASED POLYMER, METHOD FOR PRODUCING COMPOUND, METHOD FOR PRODUCING POLYMER, OLEFIN-BASED POLYMER, AND POLYMER**

(57)   A method for producing a C2-C8 unsaturated hydrocarbon according to the present invention includes: step (1) of sorting, from a waste plastic, a plastic mixture (A) having a polyolefin-based plastic content of 50% by mass or more and a residue (B); step (2) of decomposing the plastic mixture (A) to obtain a reaction product containing a C2-C8 unsaturated hydrocarbon; and step (3) of cleaning and purifying the reaction product to separate the C2-C8 unsaturated hydrocarbon.

EP 4 549 418 A1

【Ｆｉｇ．６】

Waste plastic

Step (1) Sorting

Residue (B)

Plastic mixture (A) having a polyolefin-based plastic content of 50 mass% or more

Step (2) Decomposition

Step (3) Cleaning/Purification

C2-C8 unsaturated hydrocarbon

**Description**

CROSS-REFERENCE TO RELATED APPLICATIONS

This application claims priority from the Japanese Patent Application No.2022-106346, which is incorporated herein by reference.

TECHNICAL FIELD

The present invention relates to a method for producing a C2-C8 unsaturated hydrocarbon, a method for producing a C2-C8 unsaturated hydrocarbon mixture, a method for producing an olefin-based polymer, a method for producing a compound, a method for producing a polymer, an olefin-based polymer, and a polymer.

BACKGROUND ART

Plastics are largely manufactured because they are easily available, excellent in durability, and relatively inexpensive. Since many plastics are disposable and discarded as waste, for example, environmental pollution due to a microplastic in which a plastic is miniaturized is a problem.

In addition, it is important to regenerate and reuse a plastic from a viewpoint of recycling. Therefore, in order to recycle a waste plastic, treatments such as reuse, material recycling, and chemical recycling are performed. In particular, chemical recycling has a possibility of being able to overcome a limit of performance degradation due to recycling because chemical recycling can chemically decompose a plastic and regenerate the plastic into petrochemical raw materials. For example, Non-Patent Document 1 discloses a catalytic cracking method for converting a polyolefin into a lower olefin using a catalyst as one of chemical recycling techniques.

PRIOR ART DOCUMENTS

NON-PATENT DOCUMENT

Non Patent Document 1: Monthly fine chemicals, Vol 46 (No. 12), p 44-50 (2017)

SUMMARY OF THE INVENTION

PROBLEMS TO BE SOLVED BY THE INVENTION

In recent years, as active development of a chemical recycling technique suggests, there is a demand for a technique of more efficiently obtaining a compound such as a petrochemical raw material. In particular, since products produced from a C2-C8 unsaturated hydrocarbon are diverse, it is desired to efficiently obtain the C2-C8 unsaturated hydrocarbon.

The present invention has been made in view of such a circumstance, and an object of the present invention is to provide a method for producing a C2-C8 unsaturated hydrocarbon capable of efficiently obtaining a compound such as a petrochemical raw material from a waste plastic, a method for producing a C2-C8 unsaturated hydrocarbon mixture using the production method, a method for producing an olefin-based polymer, a method for producing a compound, a method for producing a polymer, an olefin-based polymer obtained by the method for producing an olefin-based polymer, and a polymer obtained by the method for producing a polymer.

MEANS FOR SOLVING THE PROBLEMS

A method for producing a C2-C8 unsaturated hydrocarbon according to a first aspect of the present invention includes:

step (1) of sorting, from a waste plastic, a plastic mixture (A) having a polyolefin-based plastic content of 50 mass% or more and a residue (B);
step (2) of decomposing the plastic mixture (A) to obtain a reaction product containing a C2-C8 unsaturated hydrocarbon; and
step (3) of cleaning and purifying the reaction product to separate the C2-C8 unsaturated hydrocarbon.

A method for producing a C2-C8 unsaturated hydrocarbon according to a second aspect of the present invention includes:

step (1) of sorting, from a waste plastic, a plastic mixture (A) having a polyolefin-based plastic content of 50 mass% or more and a residue (B);
step (12) of heating the residue (B) to obtain a mixed gas containing carbon monoxide, carbon dioxide, and hydrogen;
step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0;
step (14) of causing carbon monoxide, carbon dioxide, and hydrogen contained in the adjustment gas to react with each other to obtain an alcohol; and
step (15) of obtaining a C2-C8 unsaturated hydrocarbon using the alcohol as a raw material.

A method for producing a C2-C8 unsaturated hydrocarbon according to a third aspect of the present invention includes:

step (1) of sorting, from a waste plastic, a plastic mixture (A) having a polyolefin-based plastic content of 50 mass% or more and a residue (B);
step (2) of decomposing the plastic mixture (A) to obtain a reaction product containing a C2-C8 unsaturated hydrocarbon;
step (3) of cleaning and purifying the reaction product to separate the C2-C8 unsaturated hydrocarbon;
step (12) of heating the residue (B) to obtain a mixed gas containing carbon monoxide, carbon dioxide, and hydrogen;
step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0;
step (14) of causing carbon monoxide, carbon dioxide, and hydrogen contained in the adjustment gas to react with each other to obtain an alcohol; and
step (15) of obtaining a C2-C8 unsaturated hydrocarbon using the alcohol as a raw material.

A method for producing a C2-C8 unsaturated hydrocarbon according to a fourth aspect of the present invention includes:

step (21) of causing carbon dioxide and hydrogen to react with each other to obtain a reaction mixture containing an alcohol, carbon dioxide, and carbon monoxide; and
step (22) of obtaining a C2-C8 unsaturated hydrocarbon from the alcohol and/or carbon monoxide contained in the reaction mixture.

A method for producing a C2-C8 unsaturated hydrocarbon according to a fifth aspect of the present invention includes the method for producing a C2-C8 unsaturated hydrocarbon according to any one of the first to third aspects and the method for producing a C2-C8 unsaturated hydrocarbon according to the fourth aspect.
A method for producing a C2-C8 unsaturated hydrocarbon according to a sixth aspect of the present invention includes:

the method for producing a C2-C8 unsaturated hydrocarbon according to any one of the first to fourth aspects; and
a method for producing a C2-C8 unsaturated hydrocarbon, including step (31) of subjecting at least one alcohol selected from the group consisting of ethanol, propanol, and butanol to a dehydration reaction to obtain a C2-C8 unsaturated hydrocarbon.

A method for producing a C2-C8 unsaturated hydrocarbon according to a seventh aspect of the present invention includes:

the method for producing a C2-C8 unsaturated hydrocarbon according to the fifth aspect; and
a method for producing a C2-C8 unsaturated hydrocarbon, including step (31) of subjecting at least one alcohol selected from the group consisting of ethanol, propanol, and butanol to a dehydration reaction to obtain a C2-C8 unsaturated hydrocarbon.

A method for producing a C2-C8 unsaturated hydrocarbon mixture according to the present invention includes step (41) of bringing a C2-C8 unsaturated hydrocarbon obtained by the method for producing a C2-C8 unsaturated hydrocarbon according to any one of the first to seventh aspects into contact with a C2-C8 unsaturated hydrocarbon derived from fossil resources to obtain a mixture.

A method for producing an olefin-based polymer according to the present invention includes a step of polymerizing a monomer containing a C2-C8 unsaturated hydrocarbon obtained by the method for producing a C2-C8 unsaturated hydrocarbon according to any one of the first to seventh aspects or a C2-C8 unsaturated hydrocarbon mixture obtained by the above-described method for producing a C2-C8 unsaturated hydrocarbon mixture.

A method for producing a compound according to the present invention includes a step of synthesizing at least one compound selected from the compound group consisting of methyl methacrylate, propylene oxide, ethylene oxide, ethylene glycol, phenol, acetone, and isopropyl alcohol using, as a raw material, a C2-C8 unsaturated hydrocarbon obtained by the method for producing a C2-C8 unsaturated hydrocarbon according to any one of the first to seventh aspects or a C2-C8 unsaturated hydrocarbon mixture obtained by the above-described method for producing a C2-C8 unsaturated hydrocarbon mixture.

A method for producing a polymer according to the present invention includes a step of polymerizing a monomer containing at least one compound selected from the compound group consisting of methyl methacrylate, propylene oxide, ethylene oxide, and ethylene glycol, the monomer being produced by using, as a raw material, a C2-C8 unsaturated hydrocarbon obtained by the method for producing a C2-C8 unsaturated hydrocarbon according to any one of the first to seventh aspects or a C2-C8 unsaturated hydrocarbon mixture obtained by the above-described method for producing a C2-C8 unsaturated hydrocarbon mixture.

An olefin-based polymer according to the present invention is obtained by the above-described method for producing an olefin-based polymer, and has a plant-derived carbon concentration of 0.1 to 99.9 pMC.

A polymer according to the present invention is obtained by the above-described method for producing a polymer, and has a plant-derived carbon concentration of 0.1 to 99.9 pMC.

EFFECT OF THE INVENTION

The present invention can provide a novel and efficient method for producing a C2-C8 unsaturated hydrocarbon capable of efficiently obtaining a C2-C8 unsaturated hydrocarbon from a waste plastic, a method for producing a C2-C8 unsaturated hydrocarbon mixture using the production method, a method for producing an olefin-based polymer, a method for producing a compound, a method for producing a polymer, an olefin-based polymer obtained by the method for producing an olefin-based polymer, and a polymer obtained by the method for producing a polymer.

BRIEF DESCRIPTION OF THE DRAWINGS

Fig. 1 is a diagram illustrating an example of a flow for producing an ethylene derivative using ethylene produced in the present invention as a starting material.
Fig. 2 is a diagram illustrating an example of a flow for producing a propylene derivative using propylene produced in the present invention as a starting material.
Fig. 3 is a diagram illustrating an example of a flow for producing a derivative using a C4 unsaturated hydrocarbon produced in the present invention as a starting material.
Fig. 4 is a diagram illustrating an example of a flow for producing a derivative using a C5 unsaturated hydrocarbon produced in the present invention as a starting material.
Fig. 5 is a diagram illustrating an example of a flow for producing derivatives using benzene, toluene, and xylene produced in the present invention as starting materials.

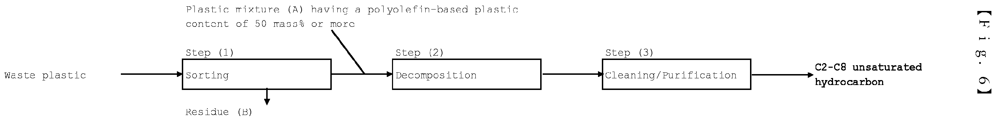

Fig. 6 is a diagram illustrating an example of an invention according to claim 1.

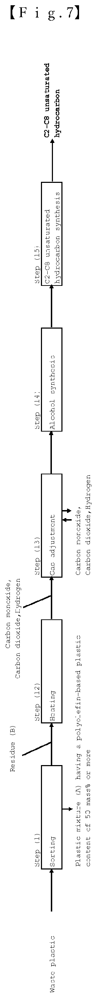

Fig. 7 is a diagram illustrating an example of an invention according to claim 2.

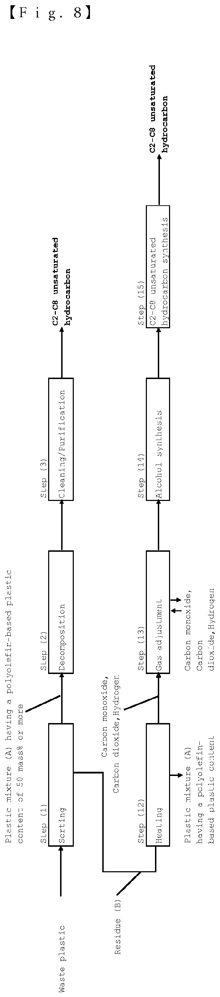

Fig. 8 is a diagram illustrating an example of an invention according to claim 3.

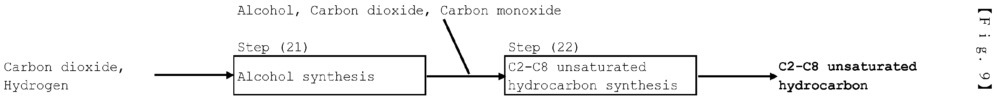

Fig. 9 is a diagram illustrating an example of an invention according to claim 5.

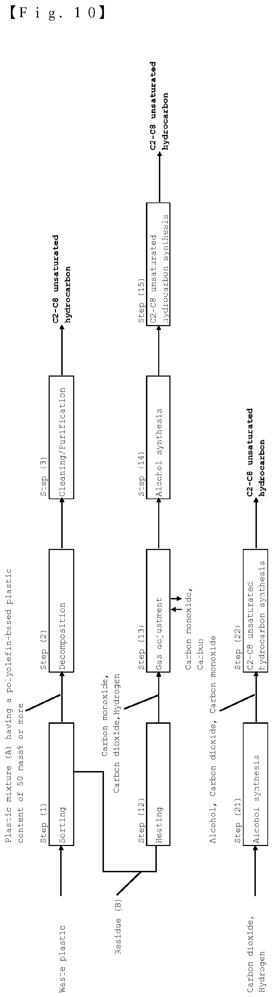

Fig. 10 is a diagram illustrating an example of an invention according to claim 6.

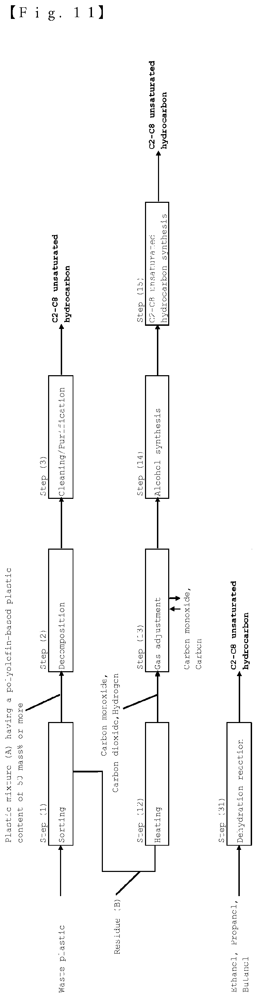

Fig. 11 is a diagram illustrating an example of an invention according to claim 7.

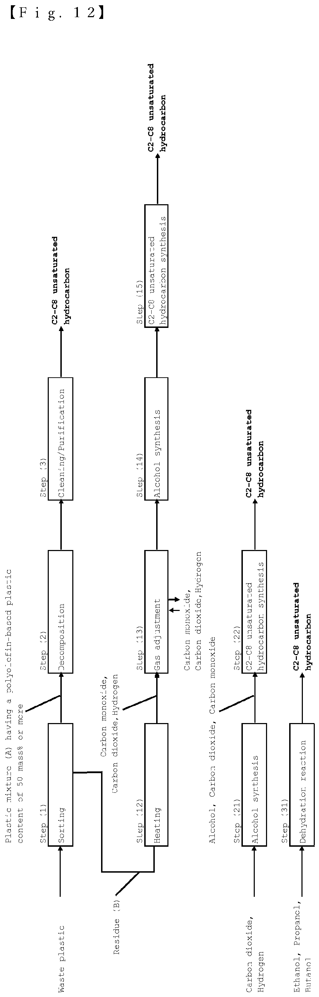

Fig. 12 is a diagram illustrating an example of an invention according to claim 8.

MODE FOR CARRYING OUT THE INVENTION

Hereinafter, embodiments of the present invention will be described, but the present invention is not limited to the following embodiments.

[First embodiment of method for producing C2-C8 unsaturated hydrocarbon]

A method for producing a C2-C8 unsaturated hydrocarbon according to a first embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon; and step (3) of cleaning and purifying the reaction product to separate the C2-C8 unsaturated hydrocarbon.

Step (1) is a step of sorting the plastic mixture (A) and the residue (B) from a waste plastic. The waste plastic used for sorting in step (1) mainly contains two types: a general waste plastic and an industrial waste plastic. The general waste plastic is, for example, a plastic waste mainly discharged from home, such as a plastic bottle after use, a food tray, a plastic bag, a seasoning bottle, or a hanger. On the other hand, the industrial waste plastic is a plastic waste mainly discharged from a business site such as a factory or a store, such as scraps and packaging materials discharged from production, processing, distribution processes of a plastic product, and a plastic bag, a food container, and the like discharged from an office.

Examples of the plastic contained in the waste plastic include a polyolefin-based plastic, polystyrene, polyamide, polycarbonate, polyurethane, polyester, polyethylene terephthalate (PET), polymethyl methacrylate, an acrylonitrile-styrene copolymer, an acrylonitrile-butadiene-styrene copolymer, polyvinyl chloride (PVC), polyvinylidene chloride (PVDC), natural rubber, and synthetic rubber. In addition, usually, the waste plastic contains foreign substances such as paper, glass, stone, wood, and metal as undesirable components other than plastics.

Step (1) is a step of treating a waste plastic to separate the waste plastic into the plastic mixture (A) which is suitable for a decomposition reaction and has an increased polyolefin content and the residue (B) which is another component undesirable for the decomposition reaction. Step (1) may use a plurality of devices that perform different treatments. As such a device, for example, as disclosed in JP-A-2022-2833 and JP-A-2017-170653, one or more devices of a sorting device, a crushing device, a cleaning device, a dewatering device, and a drying device may be included.

The sorting device is a device that selects a polyolefin-based plastic from a waste plastic by using a magnetic force sorting, an optical sorting device, a specific gravity separation device, a swing type sorting machine, a centrifugal separator, or the like.

For example, in the swing type sorting machine, by separating a waste plastic into a heavy object (solid plastic), a small-diameter object (metal pieces, wood chips, and the like), and a light object (film-type plastic), a film-type plastic and a tray-type plastic mainly containing polyolefin may be sorted as the light object, a solid plastic such as a PET bottle, metal, and the like may be removed as the heavy object and the small-diameter object, and furthermore, air separation, magnetic separation, specific gravity separation, and the like may be performed to increase a removal ratio of foreign substances.

In addition, in the centrifugal separator, polyvinyl chloride, polyvinylidene chloride, and the like, which are chlorine-containing plastics that generate a chlorine gas at the time of combustion, are sorted from polypropylene with high accuracy as sorting of a type of plastic material. More specifically, the centrifugal separator separates a light specific gravity plastic and a heavy specific gravity plastic using water as a medium by rotating a container containing, for example, water and a waste plastic for centrifugal separation, and can effectively sort PP having a specific gravity of about 0.9 and a chlorine-containing plastic having a specific gravity of about 1.1 to 1.4.

The crushing device is a device that crushes plastic. The cleaning device is a device that cleans the sorted and crushed plastic. The dewatering device and the drying device are devices that dewater and dry, for example, a cleaned plastic.

By step (1), the plastic mixture (A) having an increased polyolefin-based plastic content can be obtained. The plastic mixture (A) contains a polyolefin suitable for a decomposition reaction. The content of the polyolefin-based plastic is 50 mass% or more with respect to the total mass of the plastic mixture (A). Examples of the polyolefin-based plastic contained in the plastic mixture (A) include polyolefin-based plastics such as polyethylene, polypropylene, polybutene, an ethylene-vinyl acetate copolymer, an ethylene-methyl acrylate copolymer, an ethylene-methyl methacrylate copolymer, an ethylene-propylene copolymer, and an ethylene-$\alpha$-olefin copolymer, and polyethylene, polypropylene, and an ethylene-propylene copolymer are preferable. Note that the polyolefin-based plastic contained in the plastic mixture (A) may be a mixture of two or more thereof.

The total of polyvinyl chloride (PVC) and polyvinylidene chloride (PVDC) in the plastic mixture (A) having an increased polyolefin-based plastic content is preferably 1 mass% or less, polyamide is preferably 4 mass% or less, and polyethylene terephthalate (PET) is preferably 5 mass% or less.

The residue (B) sorted in step (1) is a remaining waste plastic other than the plastic mixture (A). Examples of the residue (B) include a dirty polyolefin, a film in which a polyolefin and a non-polyolefin are bonded, a non-polyolefin resin, paper, glass, wood, stone, and metal. Examples of the non-polyolefin resin include polystyrene, polyamide, polycarbonate, polyurethane, polyester, polyethylene terephthalate (PET), polymethyl methacrylate, an acrylonitrile-styrene copolymer, an acrylonitrile-butadiene-styrene copolymer, polyvinyl chloride (PVC), polyvinylidene chloride (PVDC), natural rubber, and synthetic rubber. The content of the polyolefin-based plastic in the residue (B) is less than the content of the polyolefin-based plastic in the plastic mixture (A).

Step (2) is a step of decomposing the plastic mixture (A) to obtain a reaction product containing a C2-C8 unsaturated hydrocarbon. In one aspect, the decomposition of the plastic mixture (A) in step (2) includes a thermal decomposition step of performing decomposition by heating. In another aspect, the decomposition of the plastic mixture (A) in step (2) includes a catalytic cracking step of performing a reaction while bringing the plastic mixture (A) into contact with a catalyst in addition to heating the plastic mixture (A). In still another aspect, the decomposition of the plastic mixture (A) in step (2) includes a thermal decomposition step and a catalytic cracking step. In addition, the product obtained in the thermal decomposition step and/or the catalytic cracking step may be decomposed again in another thermal decomposition step to obtain a reaction product containing a C2-C8 unsaturated hydrocarbon. When decomposition is performed by combining a plurality of decomposition steps, a reaction product obtained in a decomposition step performed first may be directly supplied to a decomposition step performed later, or at least a part thereof may be purified and supplied to a subsequent decomposition step.

First, an aspect in which the decomposition of the plastic mixture (A) in step (2) includes a thermal decomposition step will be described below.

In the thermal decomposition step, the plastic mixture (A) is decomposed by heating. In the thermal decomposition step, a thermal decomposition device may be used, or a cracker device using naphtha or ethane as a raw material may be used. In the thermal decomposition step, the plastic mixture (A) is decomposed to obtain a reaction product containing a C2-C8 unsaturated hydrocarbon. By the decomposition of the plastic mixture (A), a C2-C8 unsaturated hydrocarbon may be directly obtained, and/or a saturated hydrocarbon and a hydrocarbon component having 9 or more carbon atoms may be obtained. In addition, a mixture of both may be obtained.

As the thermal decomposition device, for example, one or more heating extruders, a heating rotary furnace, a heated vessel type reactor, a fixed bed reactor, a bubbling fluidized bed reactor, an internal circulating fluidized bed reactor, a circulating fluidized bed reactor, a device that causes plastic to flow down along an inner wall of a heating container to decompose the plastic, or a microwave heating device is used. As a heat source in the thermal decomposition step, a thermal decomposition residue generated in the thermal decomposition device, and a gas and/or liquid hydrocarbon containing a hydrocarbon obtained by cleaning and purification in step (3) described later can be burned and used. Alternatively, electricity or microwave can be used, or electricity or microwave can be combined with the combustion. The thermal decomposition step may include a combustion device that burns a thermal decomposition residue generated by the thermal decomposition reaction. In addition, heat necessary for the thermal decomposition device may be supplied from the combustion device using a heat transfer surface or a heat transfer medium.

As conditions of the thermal decomposition device in the thermal decomposition step, low severity or high severity may be used. The low severity thermal decomposition reaction can be performed, for example, at a temperature of 250 to 450°C and may generate a thermal decomposition oil rich in monoolefins and diolefins and significant amounts of aromatics, and the reaction product may contain chloride compounds. The high severity thermal decomposition reaction is a reaction performed, for example, at a temperature of 450 to 750°C, and can generate a thermal decomposition oil rich in aromatic compounds. A liquid product of the high severity thermal decomposition reaction may contain chloride compounds. A residence time of the reaction product in the thermal decomposition step is 0.1 to 100 seconds.

The thermal decomposition device may include a supply device for supplying the plastic mixture (A) sorted in step (1). The supply device may include, for example, a rotary feeder, a screw feeder, or a table feeder as a fixed amount supply device, and may include a double damper, a lock hopper, and the like for sealing a device and the supply device. In addition, the shape of the plastic mixture (A) to be thermally decomposed is not particularly limited, and may be, for example, any of a fluff shape, a compression molded pellet shape, a pellet shape molded by semi-melting, total melting, or the like.

The thermal decomposition device used in the thermal decomposition step may include a devolatilization extruder. The devolatilization extruder introduces a purge gas in the thermal decomposition step, and removes a gaseous chloride from an obtained reaction product. A devolatilization extruder that can be used for mixed plastic thermal decomposition and treatment is described in further detail in U.S. Provisional Patent Application No. 62/369379 filed August 1, 2016.

The reaction product containing a C2-C8 unsaturated hydrocarbon decomposed in the thermal decomposition step may be separated into a gas product and a liquid product. In this case, the gas product contains at least part of a gas phase of the reaction product and the liquid product contains at least part of a liquid phase of the reaction product. At this time, the thermal decomposition device may include a device for separating a gas product and a liquid product. Examples of the device for separating a gas product and a liquid product include a vapor-liquid separator, an oil-gas separator, a gas-liquid separator, a degassing device, a deliquoring device, a scrubber, a trap, a flash drum, a compressor suction drum, a gravity separation device, a centrifugal separator, a filter bearing separator, a mist removing pad, a liquid-gas coalescer, a distillation column, and a combination thereof.

As one aspect, the thermal decomposition device may be a condenser that operates under a condition of condensing a part of a reaction product to form a hydrocarbon liquid (for example, a liquid product) while leaving hydrocarbon gases in a gas phase (for example, a gas product). The gas product of the reaction product may be cooled by a cooler or the like, and may be separated into, for example, a product corresponding to heavy oil, a product corresponding to light oil, a product corresponding to kerosene, and the like depending on a difference in boiling point, and recovered.

The reaction product obtained in the thermal decomposition step may be hydrogenated. The hydrogenation is performed by subjecting a liquid product in the reaction product to a hydrogen treatment. By the liquid product being subjected to the hydrogen treatment, a hydrocarbon product and a C1 to C4 gas product can be generated. The hydrogenation is performed, for example, in a hydrocracker, a contact cracker, a fluid contact cracker, a hydrogen treatment device, or a hydrogen treatment reactor combining these. By the hydrogenation of the reaction product in the thermal decomposition step, a long-chain molecule having a large carbon atom number is hydrogenolyzed and dechlorinated. In the hydrogenation, a liquid product, hydrogen, or a combination thereof is stepwise added to and brought into contact with an ascending stream, a descending stream, a radial stream, or a hydrogen treatment catalyst in combination thereof.

The hydrogen treatment reactor in which hydrogenation is performed includes a hydrogen treatment catalyst and can handle a gas phase, a liquid phase, a gas-liquid phase, a gas-liquid-solid phase, or a slurry phase. The hydrogen treatment reactor may include one or more hydrogen treatment catalyst beds such as a fixed bed, a fluidized bed, a movable bed, an ebullated bed, a slurry bed, or a combination thereof. The hydrogen treatment reactor can be operated adiabatically, isothermally, non-adiabatically, non-isothermally, or by a combination thereof. In one aspect, the hydrogen treatment reactor may include one or more containers. In addition, the hydrogen treatment reactor can facilitate any reaction of components of the liquid product with or in the presence of hydrogen.

The reaction by the hydrogenation is a reaction of adding a hydrogen atom to a double bond of an unsaturated molecule (for example, olefins and aromatic compounds), and as a result, a saturated molecule (for example, paraffins, i-paraffins, and naphthenes) is generated. Furthermore, the reaction by the hydrogenation can break a bond of an organic compound, resulting in cracking of a hydrocarbon molecule to form a smaller hydrocarbon molecule having 2 or more carbon atoms, or in a subsequent reaction and/or replacement of a heteroatom with hydrogen. Examples of a reaction that may occur in the hydrogenation include, but are not limited to, hydrogenation of olefins, removal of a heteroatom from heteroatom-containing hydrocarbons (for example, dechlorination), hydrocracking of large paraffins or i-paraffins into smaller hydrocarbon molecules, hydrocracking of aromatic hydrocarbons into smaller cyclic or acyclic hydrocarbons, conversion of one or more aromatic compounds into one or more cycloparaffins, isomerization of one or more normal paraffins into one or more i-paraffins, selective ring opening of one or more cycloparaffins into one or more i-paraffins, and a combination thereof.

In one aspect, the liquid product is brought into contact with a hydrogen treatment catalyst in the presence of hydrogen to obtain C1 to C4 gas products and liquid hydrocarbons having 5 or more carbon atoms. Here, an advantage of dechlorination using a hydrogen treatment catalyst is that a chlorine sorbent is not required, and an effective amount of $Na_2CO_3$ does not have to be added for the hydrogen treatment catalyst functioning as a dechlorination agent.

The hydrogen treatment catalyst only needs to be any catalyst (for example, a commercially available hydrogen treatment catalyst) to be used in hydrogenation of olefins and aromatic hydrocarbons. Examples of the hydrogen treatment catalyst include an alumina-supported cobalt and molybdenum catalyst (Co-Mo catalyst), an alumina-supported nickel and molybdenum catalyst (Ni-Mo catalyst), an alumina-supported tungsten and molybdenum catalyst (W-Mo catalyst), alumina-supported cobalt oxide and molybdenum oxide, alumina-supported nickel oxide and molybdenum oxide, alumina-supported tungsten oxide and molybdenum oxide, alumina-supported cobalt sulfide and molybdenum sulfide, alumina-supported nickel sulfide and molybdenum sulfide, alumina-supported tungsten sulfide and molybdenum sulfide, a zeolite containing one or more metals, and a combination thereof. Furthermore, examples of other catalysts suitable for use as the hydrogen treatment catalyst include an alumina-supported platinum and palladium catalyst (Pt-Pd catalysts) suitable for slurry treatment, nickel sulfide suitable for slurry treatment, molybdenum sulfide suitable for slurry treatment, and a combination thereof. Examples of the zeolite include ZSM-5, ZSM-11, Y, high silica Y, USY, and a combination thereof. Each of one or more metals of the zeolite can be independently selected from the group consisting of, for example, cobalt, molybdenum, tungsten, Nickel, titanium, copper, magnesium, tin, iron, zinc, tungsten, vanadium, gallium, calcium, manganese, ruthenium, and rhenium.

The reaction product may be introduced into a cracker such as a naphtha cracker or an ethane cracker and cracked. In some aspects, the reaction product can be introduced into a cracker. In another aspect, the reaction product can be introduced into a cracker after C6 to C8 aromatic compounds are recovered in an aromatic compound separation unit. At least a part of the cracked reaction product and/or at least a part of the C1 to C4 gas product is introduced into the separation unit, and an unsaturated hydrocarbon gas, a saturated hydrocarbon gas, an aromatic compound, and a heavy component can be generated. Note that the unsaturated hydrocarbon gas contains ethylene, propylene, butylenes, butadiene, or a combination thereof. The saturated hydrocarbon gas contains methane, ethane, propane, butanes, hydrogen, or a combination thereof. The aromatic compound contains C6-C8 aromatic hydrocarbons. The heavy component contains hydrocarbons having 5 or more carbon atoms other than C6-C8 aromatic hydrocarbons. In one aspect, the separation unit may include a plurality of distillation columns.

Next, an aspect in which the decomposition of the plastic mixture (A) in step (2) includes a catalytic cracking step will be described below.

In the catalytic cracking step, the plastic mixture (A) is caused to react while being heated and further brought into contact with a catalyst. Any known and preferable reaction container can be used for the catalytic cracking step. Examples of a material of the reaction container include quartz glass, carbon steel, stainless steel, Inconel alloy, Hastelloy alloy, Incoloy alloy, and Monel alloy.

Examples of the catalyst used in the catalytic cracking step include a non-bonded (non-supported) zeolite catalyst and a zeolite catalyst combined with a binder or a carrier. Any known and preferable binder and carrier can be used. Examples of the binder include silica, alumina, silica-alumina, silica-titania, silica-thoria, silica-magnesia, silica-dilonia, silica-beryllia, and a ternary composition of silica and other refractory oxides. Examples of the binder or a matrix material include clays such as montmorillonite, kaolin, bentonite, halloysite, dickite, nacrite, and anaxite.

As the catalyst used in the catalytic cracking step, an MFI-type zeolite catalyst is preferably used. The MFI-type zeolite catalyst may contain a silicon atom, an aluminum atom, an oxygen atom, or a hydrogen atom as an atom other than a sodium atom. In addition, the MFI-type zeolite catalyst may contain an atom such as a sodium atom, a titanium atom, a chromium atom, a manganese atom, an iron atom, a cobalt atom, a nickel atom, a copper atom, a ruthenium atom, a rhodium atom, a palladium atom, a silver atom, an iridium atom, a platinum atom, a boron atom, a nitrogen atom, a magnesium atom, a phosphorus atom, a zinc atom, or a gallium atom. The MFI-type zeolite catalyst preferably contains a sodium atom, a silicon atom, or an aluminum atom from a viewpoint of improving a yield of a C2-C8 unsaturated hydrocarbon.

A ratio of the number of moles of silicon atoms to the number of moles of aluminum atoms (Si/Al ratio) of the MFI-type zeolite catalyst is preferably 50 or more, and more preferably 100 or more from a viewpoint of improving a yield of a C2-C8 unsaturated hydrocarbon.

The sodium content and the Si/Al ratio of the zeolite catalyst can be calculated by analyzing the zeolite catalyst according to known ICP emission spectrometry.

Here, the MFI-type zeolite means a crystalline aluminosilicate having an MFI structure in a structure code of International Zeolite Association (IZA). Specific examples of the MFI-type zeolite include $H^+$-ZSM-5, $NH_4^+$-ZSM-**5,** $Na^+$-ZSM-5, and $Ca^{2+}$-ZSM-5. The MFI-type zeolite can be prepared by any known and preferable method, and commercially available $H^+$-ZSM-5 may be used. The MFI-type zeolite can be confirmed by analysis by X-ray diffraction analysis.

Hereinafter, a method for producing the MFI-type zeolite catalyst will be described. The MFI-type zeolite catalyst can be produced by a production method including a step of preparing a mixture containing a silicon source, an aluminum source, a mold agent, and an alkali metal source, and crystallizing the mixture to obtain an MFI-type zeolite. Here, the "mold agent" refers to a substance for imparting a pore structure to zeolite.

As the silicon source, a known silicon source used for production of various zeolites can be used. Examples of the silicon source include tetraethyl orthosilicate, colloidal silica, silica gel dry powder, silica hydrogel, and sodium silicate.

As the aluminum source, a known aluminum source used for production of various zeolites can be used. Examples of the aluminum source include aluminum nitrate, aluminum chloride, sodium aluminate, aluminum hydroxide, and an aluminum alkoxide. Among these aluminum sources, aluminum nitrate or sodium aluminate is preferable.

As the mold agent, a known mold agent used for synthesis of an MFI-type zeolite can be used. Examples of the mold agent include a tetrapropylammonium salt, a tetraethylammonium salt, propanolamine, ethanolamine, n-propylamine, morpholine, 1,5-diaminopentane, 1,6-diaminohexane, dipropylenetetramine, and triethylenetetramine. Among these mold agents, a tetrapropylammonium salt (tetrapropylammonium hydroxide) is preferable.

Examples of the alkali metal source include a hydroxide of an alkali metal, a chloride of an alkali metal, a bromide of an alkali metal, and a sulfide of an alkali metal. Examples of the alkali metal include sodium and potassium.

When the alkali metal is sodium, examples of a sodium source include sodium hydroxide, sodium nitrate, sodium chloride, sodium bromide, sodium sulfate, sodium silicate, sodium aluminate, and a compound containing sodium as a counter cation.

When the alkali metal is potassium, examples of potassium include potassium hydroxide, potassium nitrate, potassium chloride, potassium bromide, potassium sulfate, potassium silicate, potassium aluminate, and a compound containing potassium as a counter cation.

In the mixture containing a silicon source, an aluminum source, a mold agent, and an alkali metal source, a ratio of the number of moles of silicon atoms to the number of moles of aluminum atoms (Si/Al ratio) is preferably 50 or more, and more preferably 100 or more. The ratio may be 10,000 or less, and is preferably 2500 or less.

In addition, a ratio of the number of moles of each component in the mixture containing a silicon source, an aluminum source, a mold agent, and an alkali metal source to the number of moles of silicon atoms preferably satisfies the following requirements.

Mold agent: 0.02 or more and 5.0 or less
Alkali metal source: 0.01 or more and 0.2 or less
Water: 2 or more and 100 or less

Furthermore, the ratio of the number of moles of each component in the mixture containing a silicon source, an aluminum source, a mold agent, and an alkali metal source to the number of moles of silicon atoms more preferably satisfies the following requirements.

Mold agent: 0.05 or more and 2.0 or less
Alkali metal source: 0.04 or more and 0.3 or less
Water: 5 or more and 50 or less

The temperature of catalytic cracking in the catalytic cracking step is 400 to 700°C, and preferably 450 to 600°C. A pressure of the catalytic cracking in the catalytic cracking step is 0 to 5 MPaG, and preferably 0 to 0.5 MPaG. A residence time of gas in the catalytic cracking step is 0.1 to 100 seconds.

In the catalytic cracking step, an inert gas such as steam, nitrogen gas, or carbon dioxide gas may coexist.

In the catalytic cracking step, a reaction is performed while the plastic mixture (A) is heated and further brought into contact with a catalyst. Therefore, the above-described thermal decomposition device may be used in the catalytic cracking step. At this time, the thermal decomposition device may include one or more beds of an inert substance or a thermal decomposition catalyst containing sand, zeolites, alumina, a catalytic cracking catalyst, or a combination thereof. The thermal decomposition catalyst can transfer heat to a component to be subjected to the thermal decomposition step. The thermal decomposition device can be operated adiabatically, isothermally, non-adiabatically, non-isothermally, or by a combination thereof.

The zeolite catalyst used in the catalytic cracking step may be a regenerated catalyst. That is, the first embodiment can be performed by applying the plastic mixture (A) (which may further contain an organic chlorine compound) to the catalytic cracking step in the presence of a regenerated catalyst obtained by regenerating a used zeolite catalyst.

Hereinafter, a method for regenerating a catalyst will be specifically described. The method for regenerating a catalyst includes a step of firing a catalyst in an atmosphere containing 1 vol% to 50 vol% of oxygen to obtain a regenerated catalyst.

The step of obtaining a regenerated catalyst is preferably performed in an atmosphere containing oxygen in a range of 1 vol% to 50 vol%, and more preferably performed in an atmosphere containing oxygen in a range of 5 vol% to 30 vol%.

The step of obtaining a regenerated catalyst can be performed, for example, in an air atmosphere, a nitrogen gas atmosphere, an argon gas atmosphere, a carbon dioxide gas atmosphere, or a gas atmosphere in which these are mixed while an oxygen concentration in the atmosphere is adjusted by any known and preferable method.

A temperature in the step of obtaining a regenerated catalyst is preferably in a range of 400 to 700°C, and more preferably in a range of 450 to 600°C.

A treatment time in the step of obtaining a regenerated catalyst is preferably 30 minutes to 48 hours, and more preferably 1 to 24 hours.

When applied to the first embodiment, a regenerated catalyst obtained by the above method for regenerating a catalyst can be used similarly to a "zeolite catalyst" that is not used in the catalytic cracking step without particularly adjusting conditions such as temperature, treatment time, and atmosphere.

An aspect in which the plastic mixture (A) is decomposed in multiple stages in step (2) will be described. In the aspect performed in multiple stages, a product obtained in the thermal decomposition step and/or the catalytic cracking step is decomposed again in another thermal decomposition step.

When step (2) is performed in multiple stages, for example, thermal decomposition devices may be connected in series. When two thermal decomposition devices are connected to each other, the thermal decomposition step can be divided into a first stage performed in a first thermal decomposition device and a second stage performed in a second thermal decomposition device and fluid-communicating with a downstream of the first stage. As can be understood by those skilled in the art, the second stage can enhance thermal decomposition of an intermediate thermal decomposition product stream flowing from the first stage to the second stage to obtain a reaction product flowing out of the second stage. In one aspect, the first stage can perform thermal decomposition of the plastic mixture (A), and the second stage can obtain a reaction product flowing out of the second stage using thermal decomposition or catalytic cracking of the plastic mixture (A). Alternatively, the first stage can perform catalytic cracking of the plastic mixture (A), and the second stage can obtain a reaction product flowing out of the second stage using thermal decomposition or catalytic decomposition of the plastic mixture (A). In another aspect, the thermal decomposition device may include one or more facilities configured to convert the plastic mixture (A) into gas-phase and liquid-phase products. The one or more facilities may include the inert substance or the thermal decomposition catalyst described above.

Step (3) is a step of cleaning and purifying the reaction product to separate a C2-C8 unsaturated hydrocarbon. The reaction product is cleaned and purified in step (3), and a C2-C8 unsaturated hydrocarbon is separated. The cleaning and purification may be performed by a known method. The purification in step (3) may include the above-described purification by hydrogenation.

The reaction product containing a C2-C8 unsaturated hydrocarbon obtained in step (2) is cleaned in step (3) to remove a chlorine compound, a sulfur compound, a nitrogen compound, and the like. Solvent cleaning, cleaning using an alkaline liquid, absorption and removal of an ammonia component, and adsorption and removal using an adsorbent are performed. In addition, the reaction product is separated and purified into a plurality of products having different boiling point differences by the purification. The cleaning and purification may be disposed in a process of the method for producing a C2-C8 unsaturated hydrocarbon according to the first embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification. In addition, in order to increase a yield of a C2-C8 unsaturated hydrocarbon, a part of the separated and purified hydrocarbons may be recycled to the catalytic cracking step or the cracker.

The C2-C8 unsaturated hydrocarbon obtained in step (3) is a C2-C5 olefin such as ethylene, propylene, butene, or pentene, a C4 or C5 diene such as butadiene or pentadiene, or a C6-C8 aromatic hydrocarbon such as benzene, toluene, or xylene, and is preferably a C2-C4 olefin.

[Second embodiment of method for producing C2-C8 unsaturated hydrocarbon]

A method for producing a C2-C8 unsaturated hydrocarbon according to a second embodiment includes: step (1) of sorting, from a waste plastic, a plastic mixture (A) having a polyolefin-based plastic content of 50 mass% or more and a residue (B); step (12) of heating the residue (B) to obtain a mixed gas containing carbon monoxide, carbon dioxide, and hydrogen;

step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; and step (15) of obtaining a C2-C8 unsaturated hydrocarbon using the alcohol as a raw material.

Step (1) of the second embodiment is similar to the above-described step (1) of the first embodiment. In the second embodiment, the raw material containing the residue (B) sorted in step (1) is gasified in step (12) described later to obtain a mixed gas mainly containing carbon monoxide, carbon dioxide, and hydrogen. The raw material may contain organic substance-containing waste from which a gas containing hydrogen and carbon oxide described below is obtained. Examples of the waste include waste such as garbage, paper waste, fiber waste, plastic waste, biomass waste, food waste, building materials, wood, wood chips, thinning wood, rice straw, wheat straw, rice hulls, waste oil, a rubber tire, or a mixture thereof.

Step (12) is a step of heating the residue (B) to obtain a mixed gas containing carbon monoxide, carbon dioxide, and hydrogen, and is performed using a mixed gas acquisition device. In step (12), the residue (B) is supplied to the mixed gas acquisition device, and the residue (B) reacts in the mixed gas acquisition device. As the mixed gas acquisition device, for example, a known device such as a fixed bed furnace, a fluidized bed furnace, a bubbling fluidized bed furnace, a circulating fluidized bed furnace, or a circulating movable bed furnace can be used.

The residue (B) supplied to the mixed gas acquisition device may further contain a waste plastic such as a general waste plastic or an industrial waste plastic. The general waste plastic is, for example, a plastic waste mainly discharged from home, such as a plastic bottle after use, a food tray, a plastic bag, a seasoning bottle, or a hanger. On the other hand, the industrial waste plastic is a plastic waste mainly discharged from a business site such as a factory or a store, such as scraps and packaging materials discharged from production, processing, distribution processes of a plastic product, and a plastic bag, a food container, and the like discharged from an office.

In the mixed gas acquisition device, it is assumed that, for example, reactions represented by the following formulas (1) to (4) occur by a reaction of a hydrocarbon with steam or oxygen.

$$C_nH_{2n}+nH_2O \rightarrow nCO+2nH_2 \qquad (1)$$

$$C_nH_{2n}+2nH_2O \rightarrow nCO_2+3nH_2 \qquad (2)$$

$$C_nH_{2n}+0.5nO_2 \rightarrow nCO+nH_2 \qquad (3)$$

$$C_nH_{2n}+nO_2 \rightarrow nCO_2+nH_2 \qquad (4)$$

Note that, in the above formulas (1) and (3), carbon monoxide is generated together with hydrogen, but in the second embodiment, a step of obtaining carbon dioxide and hydrogen by a reaction of the following formula (5) using the carbon monoxide and water as raw materials may be performed. In addition, a step of obtaining heat energy necessary for step (12) and carbon dioxide by a reaction of the following formula (6) using the carbon monoxide and oxygen as raw materials may be performed.

$$CO+H_2O \rightarrow CO_2+H_2 \qquad (5)$$

$$CO+0.5O_2 \rightarrow CO_2 \qquad (6)$$

In the reactions of the above formulas (1) to (6), 1 to 3 mol of hydrogen is obtained per mol of carbon atoms.

On the other hand, when the residue (B) contains a waste containing a naturally occurring organic substance, it is considered that, for example, a reaction represented by the following formula (7) and/or a reaction represented by the following formula (8) occurs in the mixed gas acquisition device.

$$(C_6H_{12}O_6)_n \rightarrow 6nCO + 6nH_2 \qquad (7)$$

$$(C_6H_{12}O_6)_n + 6nH_2O \rightarrow 6nCO_2 + 12nH_2 \qquad (8)$$

Note that, in the above formula (7), carbon monoxide is generated together with hydrogen, but carbon dioxide and hydrogen may be obtained by a reaction of the following formula (5) using the carbon monoxide and water as raw materials. In the above formulas (7) and (8), 1 to 2 mol of hydrogen is obtained per mol of carbon atoms.

In the above-described reaction for obtaining carbon monoxide and hydrogen from an organic substance, a reaction at a high temperature may be required as compared with a reaction for obtaining carbon dioxide and hydrogen, and the method for obtaining carbon dioxide and hydrogen is expected to be more advantageous in view of energy cost related to temperature increase.

The second embodiment may optionally additionally include step (12') of cleaning the mixed gas after step (12).

Step (12') is a step of removing impurities from the mixed gas to clean the gas, and is performed using a mixed gas cleaning device. In step (12'), the mixed gas is supplied to the mixed gas cleaning device. The mixed gas may contain, as impurities, solid contents such as soot and fly ash which cause catalyst poisoning. In addition, the mixed gas may contain, as other impurities, a reaction inhibiting component such as a sulfur component, a chlorine component, or a nitrogen component. In such a case, it is preferable to clean the mixed gas before step (14) described later. Step (12') can reduce, for example, concentrations of a chloride and a sulfide of the mixed gas. As a result, each of the concentrations of the chloride and the sulfide in a subsequent step can be reduced to 1 ppm or less, preferably 100 ppb or less. Step (12') is usually performed according to a solid content or a reaction inhibiting component contained in the mixed gas. The cleaning in step (12') is performed by a known cleaning method. As the mixed gas cleaning device, for example, a known device such as a wet cleaning column or an electric dust collector can be used.

By cleaning the mixed gas in step (12') such that each of the concentrations of the chloride and the sulfide in the mixed gas is 1 ppm or less, and preferably 100 ppb or less, a degradation rate of a catalyst used in step (14) described later can be reduced, and a catalyst life can be extended.

Step (13) is a step of mixing the mixed gas with at least one gas selected from the group consisting of carbon monoxide, carbon dioxide, and hydrogen, or removing a part of at least one gas selected from the group consisting of carbon monoxide, carbon dioxide, and hydrogen from the mixed gas to obtain an adjustment gas in which the volume amount of hydrogen to the total volume amount of carbon monoxide and carbon dioxide is 1.5 to 4.0. Step (13) is performed using a gas component adjusting device, and may be performed further using a $CO_2$ separation device as necessary.

Hereinafter, step (13) performed using the gas component adjusting device and the $CO_2$ separation device will be described in detail.

In step (13), the mixed gas cleaned by the mixed gas cleaning device is supplied to the gas component adjusting device. Hydrogen can be supplied to the gas component adjusting device. As the gas component adjusting device, for example, a known device such as a partial oxidation reactor, a steam reforming reactor, or an aqueous gas shift reactor can be used.

Then, the adjustment gas (hereinafter, also referred to as a primary adjustment gas) adjusted by the gas component adjusting device is supplied to the $CO_2$ separation device. In the $CO_2$ separation device, carbon dioxide is selectively separated (removed) from the supplied primary adjustment gas to obtain an adjustment gas (hereinafter, also referred to as a secondary adjustment gas). Examples of the $CO_2$ separation device include a pressure swing adsorption device, an absorption diffusion column, a cryogenic carbon dioxide separation device, and a carbon dioxide separation membrane facility.

Step (13) is a step of adjusting the composition of the mixed gas in the gas component adjusting device and selectively separating $CO_2$ from the adjusted gas in the $CO_2$ separation device. Through step (13), the mixed gas can be adjusted to a composition suitable for alcohol synthesis.

Hydrogen required for the production of an alcohol is 2 moles per mole of carbon monoxide (CO) and 3 moles per mole of carbon dioxide ($CO_2$). Therefore, volume fractions of hydrogen, carbon monoxide, and carbon dioxide in the adjustment gas supplied to a reactor in which a reaction in step (14) described later is performed are ideally adjusted such that an index SN represented by the following formula (9) is 2 or more. In the following formula (9), $yH_2$, $yCO_2$, and yCO are volume fractions of hydrogen, carbon dioxide, and carbon monoxide in the adjustment gas, respectively.

$$SN = (yH_2 - yCO_2) / (yCO + yCO_2) \qquad (9)$$

In the second embodiment, when the mixed gas obtained in step (12) contains hydrogen, carbon dioxide, and carbon monoxide, it is desirable to use an adjustment gas in which the concentration of carbon dioxide is adjusted to preferably 0.1 to 20 vol% and more preferably 1 to 10 vol%. Therefore, when a value of the index SN is low, it is preferable to adjust the SN value in advance before step (14) described later using the mixed gas as an adjustment gas.

Step (13) may be performed by performing at least one of (i) a method for adding hydrogen to the mixed gas, (ii) a partial oxidation reaction of a hydrocarbon contained in the mixed gas, (iii) a steam reforming reaction of a hydrocarbon contained in the mixed gas, and (iV) an aqueous gas shift reaction of carbon monoxide contained in the mixed gas. As a result, in the adjustment gas, the volume amount of hydrogen to the total volume amount of carbon monoxide and carbon dioxide (hereinafter, also referred to as $H_2/(CO+CO_2)$) can be adjusted to preferably 1.5 to 4.0.

In step (13), for example, the hydrocarbon contained in the mixed gas may be reformed into carbon monoxide and hydrogen by a partial oxidation reaction or a steam reforming reaction, and then an aqueous gas shift reaction may be performed. As a result, $H_2/(CO+CO_2)$ can be more easily adjusted to 1.5 or more and 4.0 or less.

Alternatively, in step (13), the hydrocarbon contained in the mixed gas may be reformed into carbon monoxide and hydrogen by a partial oxidation reaction or a steam reforming reaction, and then hydrogen (hydrogen gas) may be added. As a result, $H_2/(CO+CO_2)$ can be more easily adjusted to 1.5 or more and 4.0 or less. As the method for obtaining hydrogen, for example, a known technique such as reforming of fossil resources, a reaction by decomposition, a decomposition reaction or a dehydrogenation reaction of a hydrocarbon, electrolysis of water or saline, decomposition of water using a photocatalyst, or decomposition of ammonia can be used.

Examples of the decomposition reaction of a hydrocarbon include a method for generating a lower olefin or the like by a decomposition reaction of naphtha and a method for obtaining hydrogen by thermal decomposition of methane. Examples of the dehydrogenation reaction of a hydrocarbon include a method for generating ethylene by a dehydrogenation reaction of ethane, a method for generating propylene by a dehydrogenation reaction of propane, a method for generating toluene by a dehydrogenation reaction of methylcyclohexane, and a method for generating cyclohexanone by a dehydrogenation reaction of cyclohexanol.

As another aspect of the method for adjusting a value of the index SN in step (13), a ratio between carbon monoxide and carbon dioxide contained in the mixed gas may be changed by reforming, decomposing, or the like at least a part of carbon oxide contained in the mixed gas.

As still another aspect of the method of adjusting a value of the index SN in step (13), there is a method for removing at least a part of carbon dioxide from the mixed gas. The carbon dioxide removed here may be used for another application. Specifically, for example, by treating the mixed gas by a chemical adsorption method using an amine solution, carbon dioxide as an acidic gas can be removed to obtain a gas containing hydrogen and carbon monoxide. In addition to the chemical adsorption method, by treating the mixed gas by a cryogenic separation method, carbon dioxide having the highest boiling point among the three components can be separated to obtain a gas containing hydrogen and carbon monoxide. In addition, carbon dioxide may be separated by a membrane separation method using a separation membrane that selectively blocks carbon dioxide or more preferably a separation membrane through which carbon dioxide selectively passes.

In the step of selectively separating $CO_2$, for example, by treating a primary adjustment gas by a chemical absorption method using an amine solution, carbon dioxide as an acidic gas can be separated from the primary adjustment gas and obtained. As a result, it is possible to obtain a secondary adjustment gas in which a concentration of carbon dioxide is adjusted to 0.1 to 20 vol%, and preferably 1 to 10 vol%. Alternatively, by treating a primary adjustment gas by a cryogenic separation method, carbon dioxide having the highest boiling point among hydrogen, carbon monoxide, and carbon dioxide may be separated to obtain a secondary adjustment gas. In addition, by a membrane separation method using a separation membrane that selectively blocks carbon dioxide or more preferably a separation membrane through which carbon dioxide selectively passes, carbon dioxide may be separated to obtain a secondary adjustment gas. The separated carbon dioxide may be used for another application.

Note that, in the above description, the case where hydrogen is supplied to the gas component adjusting device has been described, but carbon monoxide, carbon dioxide, and/or hydrogen may be supplied to the gas component adjusting device. In addition, in the above description, the case where carbon dioxide is selectively separated (removed) from the supplied primary adjustment gas in the $CO_2$ separation device has been described, but carbon monoxide, carbon dioxide, and/or hydrogen may be selectively separated (removed) from the supplied primary adjustment gas.

By adjusting the components of the gas in step (13), the raw material composition can be adjusted such that the composition is suitable for a reaction in step (14) described later. As a result, it is possible to improve a conversion ratio to alcohol.

Step (14) is a step of causing carbon monoxide, carbon dioxide, and hydrogen contained in the adjustment gas to react with each other to obtain an alcohol. The alcohol obtained by the reaction in step (14) refers to any compound in which one or more hydrogen atoms of a hydrocarbon are replaced with hydroxy groups (-OH). The alcohol may be linear, branched, or cyclic, but is preferably a linear or branched saturated aliphatic alcohol. In addition, the alcohol is preferably a monohydric alcohol having one hydroxy group. In addition, a C1 to C5 alcohol is preferable, and a C1 to C4 alcohol is more preferable. Examples of the alcohol include methanol, ethanol, 1-propanol, 2-propanol, 1-butanol, 2-butanol, 2-methyl-1-propanol, and 2-methyl-2-propanol. Here, 1-propanol and 2-propanol may be collectively referred to as propanol, and 1-butanol, 2-butanol, 2-methyl-1-propanol, and 2-methyl-2-propanol may be collectively referred to as butanol.

The reaction of carbon monoxide, carbon dioxide, and hydrogen contained in the adjustment gas of step (14) is performed in a reactor filled with a catalyst. When methanol is obtained as one aspect, examples of the reactor include a fixed bed reactor. The fixed bed reactor for methanol synthesis is variously devised for the purpose of size increase and energy saving (Reference Document: Mitsubishi Heavy Industries, Ltd. Technical Method, Vol. 33 No. 5 (1996)), and the reactor may have a condensation surface that condenses a high-boiling component containing generated alcohol and water in the reactor.

The reactor having a condensation surface includes, in a reaction container, a catalyst layer, a transmission wall, and a condensation surface disposed spaced apart from the transmission wall. The reaction container is, for example, a metal container made of stainless steel having pressure resistance. The catalyst layer is a region where the adjustment gas and a catalyst come into contact with each other and a reaction proceeds. The catalyst layer is filled with a catalyst suitable for a reaction. The transmission wall is made of a porous member through which gas can pass. As the transmission wall, a member through which a gas containing an alcohol generated by a reaction in the catalyst layer can pass and through which a catalyst cannot pass is used. The space is a space formed between the transmission wall and the condensation surface. The condensation surface is a surface that cools a gas containing an alcohol that has passed through the transmission wall to a temperature equal to or lower than a dew point thereof to condense a high-boiling component containing methanol and water. Note that the high-boiling component means a component having a high boiling point, and in the present specification, means a component having a high boiling point among components contained in a gas generated in a methanol conversion reaction. That is, in the present specification, the high-boiling component is a component that is condensed at a temperature equal to or lower than a dew point of a gas generated in the methanol conversion reaction, and contains methanol and water.

The reactor may have a first heating medium region in which a first heating medium circulates in order to maintain the condensation surface at a temperature equal to or lower than the dew point. In addition, the reactor may have a second heating medium region in which a second heating medium for recovering heat generated by a reaction in the catalyst layer circulates.

In addition, as another aspect, in a case of obtaining an alcohol having 2 or more carbon atoms, the reactor may have a catalyst in a form other than the fixed bed, for example, a fluidized bed or a movable bed.

In one aspect, the catalyst used in step (14) may be a catalyst for producing methanol. Examples of the catalyst for producing methanol include a catalyst containing copper and zinc. Copper may be in a form of copper oxide (CuO) or in a form of a simple substance (Cu). Constituent components of the catalyst are not limited to only the above-described components, and may include other elements.

In addition, the catalyst for producing methanol may contain an alkali metal. A lower limit of the content of the alkali metal in the catalyst is 0 mass% or more, 0.001 mass% or more, 0.01 mass% or more, or 0.015 mass% or more. An upper limit of the content of the alkali metal in the catalyst is 0.05 mass% or less, 0.04 mass% or less, or 0.03 mass% or less. When the content of the alkali metal is too large, aggregation of Cu crystals is likely to occur, and a decrease in activity may be accelerated. The content of the alkali metal in the catalyst may be 0 mass%.

Examples of the alkali metal include Na, K, and Rb. In one aspect, the alkali metal is Na.

A method for producing the catalyst for producing methanol is not particularly limited, and may be a known method. For example, the catalyst can be produced by precipitating an acidic salt aqueous solution of metal elements constituting the catalyst with a precipitating agent, and then drying and firing the precipitate (see JP-A-2010-194421).

As reaction conditions in the method for producing the catalyst for producing methanol, for example, a reaction temperature: 150 to 300°C and a reaction pressure (gauge pressure): 0.5 to 10 MPa-G can be adopted.

The catalyst for producing methanol may be used as it is, or may be used after being reduced with a reducing gas (hydrogen, a mixed gas of hydrogen and nitrogen, a gas containing carbon monoxide, or the like). For example, when the catalyst is a CuO-ZnO catalyst, preferably, the CuO-ZnO catalyst is brought into contact with a gas containing hydrogen to be changed to a Cu-ZnO catalyst in a reduced state and then brought into contact with the adjustment gas.

In another aspect, the catalyst used in step (14) may be a catalyst for producing an alcohol having 2 or more carbon atoms. The catalyst for producing an alcohol having 2 or more carbon atoms may be an aggregate of catalytic metals, or may be a supported catalyst in which a catalytic metal is supported on a carrier, and the supported catalyst is preferable. By using the supported catalyst, contact efficiency between the adjustment gas and the catalytic metal is enhanced.

As the carrier, a carrier conventionally used for a catalyst can be used, and for example, a porous carrier is preferable. A material of the porous carrier is not particularly limited, and examples thereof include silica, zirconia, titania, magnesia, alumina, activated carbon, and zeolite. As the catalyst, a known catalyst can be used. For example, it is possible to refer to documents such as Catalyst 2022, 64, and 33, Catalyst 2020, 62, and 133, Chem. Soc. Rev. 2017, 46, 1358, and Journal of the Japan Petroleum Institute 1990, 33, 1.

The form of the catalyst is not particularly limited, and may be powder or a compact. The shape of the compact may be a granular shape, a cylindrical shape, a ring shaped, or the like, and is not particularly limited. Examples of a method for molding the catalyst include tablet molding, compression molding, and extrusion molding, but are not particularly limited.

When the catalyst is filled into the reactor, various diluents inert to a source gas and a reaction product may be mixed with the catalyst. Examples of the diluent include copper, alumina, zirconia, quartz, glass, and silicon carbide. Examples of the shape of the diluent include those molded in a granular shape, a spherical shape, and a cylindrical shape, and an amorphous shape.

The amount of the catalyst used is not particularly limited, and only needs to be appropriately adjusted in consideration of other reaction conditions. In addition, the catalyst may be used singly or in combination of two or more types thereof, and in a case of using two or more types in combination, the combination and a ratio thereof only need to be appropriately adjusted according to a purpose.

The adjustment gas in step (14) contains carbon oxide and hydrogen. The carbon oxide is at least one of carbon monoxide and carbon dioxide. When both carbon monoxide and carbon dioxide are used as the carbon oxide, carbon monoxide and carbon dioxide may be contained at any ratio.

The adjustment gas may contain a component other than carbon oxide and hydrogen as long as the component does not affect production of an alcohol. Examples of such a component include saturated and unsaturated hydrocarbons such as methane, ethane, propane, ethylene, and propylene, nitrogen, a rare gas element, and the like.

The adjustment gas may further contain water. Originally, in production of methanol, the smaller the amount of water in a source gas, the more preferable. However, in step (14), stability of the catalyst is maintained even when an adjustment gas containing a certain amount of water is used.

The total content of carbon oxide and hydrogen in the adjustment gas is preferably 50 vol% or more, more preferably 80 vol% or more, and still more preferably 90 vol% or more, and may be 100 vol% from a viewpoint of more efficiently obtaining an alcohol.

$H_2/(CO+CO_2)$ of the adjustment gas in step (14) is preferably 1.5 or more and 4.0 or less from a viewpoint of further increasing a space time yield of an alcohol.

The reaction in step (14) is preferably performed under pressurized conditions, and a pressure during the reaction is preferably 1 to 12 MPa. In addition, the reaction is preferably performed under heating conditions, and the temperature during the reaction is preferably 150 to 500°C, and more preferably 200 to 400°C.

When step (14) is performed using, for example, a fixed bed reactor, it is only required to perform a reaction by supplying the adjustment gas as a raw material from an inlet side of the reactor while adjusting the temperature, and to recover a reaction product and an unreacted raw material from an outlet side of the reactor. A target product and the unreacted raw material are usually recovered as a mixed gas, but for example, an alcohol as the target product can be separated by cooling the gas.

The alcohols obtained in step (14) may be separated from each other and then used in a next step, or may be used as a mixture.

In addition, in step (14), a product (for example, oxygenates such as acetic acid and acetaldehyde, and esters such as ethyl acetate, methyl acetate, and methyl formate) other than an alcohol is also generated. Therefore, the second embodiment may include step (14') (alcoholization step) of hydrogenating a product other than an alcohol to convert the product into an alcohol. Examples of step (14') include a method for converting an oxygenate containing acetaldehyde, acetic acid, or the like into an alcohol by bringing the oxygenate into contact with a hydrogenation catalyst.

As the hydrogenation catalyst in step (14'), a known catalyst can be used. Examples thereof include copper, copper-zinc, copper-chromium, copper-zinc-chromium, iron, rhodium-iron, rhodium-molybdenum, palladium, palladium-iron, palladium-molybdenum, iridium-iron, rhodium-iridium-iron, iridium-molybdenum, rhenium-zinc, platinum, nickel, cobalt, ruthenium, rhodium oxide, palladium oxide, platinum oxide, and ruthenium oxide. These hydrogenation catalysts may be supported catalysts supported on a carrier similar to the carrier used for the above-described catalyst.

In addition, step (14) may include a step of removing an acidic substance by bringing at least a part of the product containing an alcohol into contact with a sodium hydroxide aqueous solution.

Step (15) is a step of obtaining a C2-C8 unsaturated hydrocarbon using the alcohol obtained in step (14) as a raw material. Note that, as described above, the C2-C8 unsaturated hydrocarbon obtained in step (15) is a C2-C5 olefin such as ethylene, propylene, butene, or pentene, a C4 or C5 diene such as butadiene or pentadiene, or a C6-C8 aromatic hydrocarbon such as benzene, toluene, or xylene, and is preferably a C2-C4 olefin.

In step (15), the C2-C8 unsaturated hydrocarbon can be obtained by a known method.

A temperature of the step of obtaining olefins from the raw material containing an alcohol is not particularly limited as long as the temperature is in a range of 300 to 700°C, but is preferably 350 to 600°C. By performing a reaction at a temperature in this range, it is possible to prevent deterioration of selectivity of an olefin. A pressure in the step of obtaining olefins from the raw material containing an alcohol is preferably 50 kPa or more as a gauge pressure, more preferably 150 kPa or more as a gauge pressure, still more preferably 150 to 20,000 kPa as a gauge pressure, and particularly preferably 450 to 1000 kPa as a gauge pressure.

As a first method of the step of obtaining olefins from the raw material containing an alcohol, for example, a method using a non-zeolite-based molecular sieve as a catalyst, described in JP-A-02-000121 can be used. Examples of the non-zeolite-based molecular sieve include SAPO-34, SAPO-17, and a mixture thereof.

As a second method of the step of obtaining olefins from the raw material containing an alcohol, for example, a method using a shape-selective catalyst, described in JP-B2-3844734 can be used. Examples of the shape-selective catalyst include a proton-containing pentasil type catalyst having an alkali content of less than 380 ppm, preferably less than 200 ppm.

As a third method of the step of obtaining olefins from the raw material containing an alcohol, for example, a method using a catalyst obtained through a step of silylating CHA type aluminosilicate, described in JP-A-2014-46273 can be used.

As a fourth method of the step of obtaining olefins from the raw material containing an alcohol, for example, a method using a catalyst in which one or more selected from the group consisting of nickel, aluminum, manganese, iron, and copper are supported on a regular mesoporous porous body, described in Republished Patent No. 2007/083684 can be used. Examples of the regular mesoporous porous body include MCM-41.

As a fifth method of the step of obtaining olefins from the raw material containing an alcohol, for example, a method using a catalyst made of zirconium oxide in which a ratio of zirconium oxide on a surface is 50 area% or more, described in WO-A-2012/077723 can be used.

As a sixth method of the step of obtaining olefins from the raw material containing an alcohol, for example, a method using an olefin-producing catalyst containing an oxide of at least any element selected from the group consisting of metal elements of Groups 2, 3, 5 to 9, 11, and 12 in the periodic table, titanium, nickel, boron, gallium, thallium, germanium, tin, lead, and bismuth, described in JP-A-2012-136516 can be used.

As a seventh method of the step of obtaining olefins from the raw material containing an alcohol, for example, a method using an olefin-producing catalyst containing an oxide of indium, described in WO-A-2012/077724 can be used.

As an eighth method of the step of obtaining olefins from the raw material containing an alcohol, for example, a method using a silico-aluminophosphate molecular sieve, described in JP-A-2013-43794, the method including hydrothermal treatment of a mixture containing (i) a silicon source, (ii) an aluminum source, (iii) a phosphorus source, (iv) a structure directing agent, and (v) 0.001 to 0.1 mol of an inorganic or organic magnesium salt or manganese salt with respect to 1 mol of aluminum source, can be used.

As a ninth method of the step of obtaining olefins from the raw material containing an alcohol, for example, a method using a catalyst in which one or more metals selected from Group 4 metals in the periodic table are supported on an aluminosilicate having an MFI structure, described in JP-A-2010-18556 can be used.

As a tenth method of the step of obtaining olefins from the raw material containing an alcohol, when an alcohol dehydration reaction is performed, the dehydration reaction can be performed in a diluted state by adding a solvent or a gas inert to a catalyst and the alcohol to the reaction system. The alcohol dehydration reaction may be any one of a batch method, a semi-batch method, and a continuous flow method. In addition, any form of a liquid phase, a gas phase, and a gas-liquid mixed phase may be used. As a method for filling the catalyst, various methods such as a fixed bed, a fluidized bed, a suspended bed, and a shelf tier fixed bed are adopted, and any method may be adopted.

The catalyst may be a known catalyst that can be usually used in the dehydration reaction, or may be used in combination with a molding agent (binder) or the like. When the catalyst is used together with the molding agent, the catalyst and the molding agent may be used in a form of a compact in which mechanical strength is enhanced by kneading and extrusion-molding the catalyst and the molding agent. Usually, examples of the catalyst or the molding agent that can be used in the dehydration reaction include silica, alumina, clay, titania, zirconia, zinc oxide, ceria, lanthanum, graphite, and ethyl cellulose.

A temperature during the dehydration reaction of the alcohol is preferably 450°C or lower, more preferably 400°C or lower, still more preferably 300°C or lower, and particularly preferably 200°C or lower. A lower limit of the temperature is not particularly limited as long as the temperature is equal to or higher than the temperature at which the dehydration reaction is performed, but it is preferably 160°C or higher and may be 180°C or higher from a viewpoint of enhancing obtained catalytic activity. A temperature of the catalyst layer can be appropriately adjusted by, for example, changing a temperature of a device used for the dehydration treatment.

When the temperature of the dehydration reaction is in the above range, the dehydration reaction can be performed at a relatively low temperature, and therefore energy required for the reaction can be reduced. In addition, when the catalytic activity decreases, regeneration may be performed by a known method to recover the activity of the dehydration catalyst.

A reaction pressure during the dehydration reaction is not particularly limited, but is preferably 0 to 100,000 KPa (gauge pressure), more preferably 0 to 5000 KPa (gauge pressure), and still more preferably 0 to 4000 KPa (gauge pressure).

In addition, although the dehydration reaction can be performed without pressurization, the C2-C8 unsaturated hydrocarbon obtained by the dehydration reaction is a gas at normal temperature and normal pressure when the number of carbon atoms is low, and therefore needs to be liquefied in order to perform purification such as distillation. Therefore, when the C2-C8 unsaturated hydrocarbon is a gas, it is necessary to liquefy the gas by cooling, pressurization, or the like, and production of the C2-C8 unsaturated hydrocarbon is complicated.

The alcohol in the raw material containing an alcohol described above may be purified, or the alcohol obtained in the step of obtaining an alcohol may be used as it is. In addition, the alcohol may be a mixture of alcohols obtained in a plurality of steps of obtaining an alcohol. The alcohol in the raw material containing an alcohol may contain water and/or another oxygen-containing compound, or may be obtained by intentionally adding water and/or another oxygen-containing compound.

The reaction product containing a C2-C8 unsaturated hydrocarbon is separated and purified into a plurality of products having different boiling point differences by purification. The purification may be disposed in a process of the method for producing a C2-C8 unsaturated hydrocarbon according to the second embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification.

[Third embodiment of method for producing C2-C8 unsaturated hydrocarbon]

A method for producing a C2-C8 unsaturated hydrocarbon according to a third embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon;
step (3) of cleaning and purifying the reaction product to separate the C2-C8 unsaturated hydrocarbon; step (12) of heating the residue (B) to obtain a mixed gas containing carbon monoxide, carbon dioxide, and hydrogen; step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; and step (15) of obtaining a C2-C8 unsaturated hydrocarbon using the alcohol as a raw material.

The steps (1) to (3) and the steps (12) to (15) of the third embodiment are similar to the steps (1) to (3) of the first embodiment and the steps (12) to (15) of the second embodiment described above, respectively. Note that, as described above, the C2-C8 unsaturated hydrocarbon obtained in steps (3) and (15) is a C2-C5 olefin such as ethylene, propylene, butene, or pentene, a C4 or C5 diene such as butadiene or pentadiene, or a C6-C8 aromatic hydrocarbon such as benzene, toluene, or xylene, and is preferably a C2-C4 olefin.

In the method for producing a C2-C8 unsaturated hydrocarbon according to the third embodiment, the reaction product obtained in step (2) preferably further contains a paraffin. As a method of increasing the content of the paraffin, there is a method for performing a thermal decomposition reaction by setting a temperature condition of a thermal decomposition device in a thermal decomposition step to, for example, 450 to 750°C, that is, a method for performing a high severity thermal decomposition reaction. In addition, when step (2) includes a catalytic cracking step, for example, a method for selecting a catalyst having high acidity from among catalysts such as a non-bonded (non-supported) zeolite catalyst and a zeolite catalyst combined with a binder or a carrier is also preferable. Examples of the binder include silica, alumina, silica-alumina, silica-titania, silica-thoria, silica-magnesia, silica-dilonia, silica-beryllia, and a ternary composition of silica and other refractory oxides. Examples of the binder or a matrix material include clays such as montmorillonite, kaolin, bentonite, halloysite, dickite, nacrite, and anaxite.

The reaction product containing a C2-C8 unsaturated hydrocarbon is separated and purified into a plurality of products having different boiling point differences by purification. The purification may be disposed in a process of the method for producing a C2-C8 unsaturated hydrocarbon according to the third embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification.

[Fourth embodiment of method for producing C2-C8 unsaturated hydrocarbon]

A method for producing a C2-C8 unsaturated hydrocarbon according to a fourth embodiment includes: step (21) of causing carbon dioxide and hydrogen to react with each other to obtain a reaction mixture containing an alcohol, carbon dioxide, and carbon monoxide; and step (22) of obtaining a C2-C8 unsaturated hydrocarbon from the alcohol and/or carbon monoxide contained in the reaction mixture.

Step (21) is a step of causing carbon dioxide to react with hydrogen to obtain a reaction mixture containing an alcohol, carbon dioxide, and carbon monoxide. Hydrogen and carbon dioxide used in step (21) are not particularly limited, and those obtained by a known method can be used.

As hydrogen, for example, hydrogen obtained by a known method, such as hydrogen generated by a steam reforming reaction, hydrogen generated by an electrolytic reaction, hydrogen obtained by photolysis of water, hydrogen obtained by decomposition of methane, hydrogen obtained by gasification of coal, or hydrogen obtained by decomposition of ammonia can be used.

Hydrogen may be produced using a hydrogen production system. The hydrogen production system includes, for example, a thermal decomposition reaction device, a solid separation device, and a gas purification device.

A hydrocarbon decomposition reaction device is a device that decomposes a gas containing a hydrocarbon to generate hydrogen and solid carbon. Examples of the hydrocarbon to be decomposed include methane, ethane, butane, hexane, ethylene, propylene, and acetylene, and methane is preferable.

As one aspect, decomposition of methane is mentioned. As a source gas containing methane, a natural gas, a biogas (methane) obtained by anaerobic fermentation of biomass, and an off-gas containing methane by-produced from a hydrocarbon thermal decomposition process such as naphtha cracker or ethane cracker (thermal decomposition) can be used.

Methane is decomposed into a hydrogen gas and solid carbon according to formula (10).

$$CH_4\text{-}C+2H_2 \qquad (10)$$

As a methane decomposition reaction device, a known device such as a tubular reaction device, a fixed bed reaction device, a fluidized bed reaction device, a bubbling fluidized bed reaction device, a circulating fluidized bed reaction device, a movable bed reaction device, or a circulating movable bed reaction device can be used. As a decomposition method used in the methane decomposition reaction device, a known method such as a non-catalytic thermal decomposition method, a decomposition method using a solid catalyst, or a decomposition method using a liquid catalyst such as a molten salt or a molten metal can be used.

The solid catalyst is not particularly limited as long as the solid catalyst is a catalyst capable of decomposing a hydrocarbon to by-produce hydrogen and carbon, and can include any number of metals from various IUPAC groups in the periodic table, such as Group 10 (for example, nickel), Group 8 (for example, iron or ruthenium), Group 9 (for example, cobalt), or Group 6 (for example, chromium or molybdenum). Other metals that may be present include Group 7 metals (for example, manganese) and Group 5 metals (for example, cobalt). The metals listed above are merely examples, and other metals of those groups can also be included. In addition, a mixture of one or more of these catalysts may be used as a catalyst, or may be supported on a catalyst carrier.

Since a decomposition reaction of a hydrocarbon containing methane is an endothermic reaction, the decomposition reaction device includes a heating device that supplies reaction heat. As a heating device for performing decomposition, a known device such as a heating furnace, a heat exchange device using steam, a molten salt, or the like as a heat medium, a microwave heating device, a plasma heating device, an induction heating device, or a resistance heating device can be used. In addition, by using renewable energy as energy required in the hydrogen production step, it is possible to provide a hydrogen production method in which a carbon dioxide discharge amount is reduced.

The solid separation device is a device that separates a solid-gas mixture containing hydrogen, an unreacted hydrocarbon, and/or solid carbon discharged from the decomposition reaction device into a solid component and a gas component. The separation device used as the solid separation device may be any device that can separate a solid and a gas from each other. For example, a known separation means such as a cyclone, a bag filter, a ceramic filter, or a sieve can be used.

The solid carbon to be separated is in a form of carbon black, graphite, carbon nanotube, or the like. The solid carbon can be used for applications such as a battery material, a chemical raw material, a soil improver, and a building material.

The gas purification device is a device for separating hydrogen from a gas containing hydrogen and an unreacted hydrocarbon from which a solid has been removed by the solid separation device, and a known gas separation device such as a pressure swing adsorption (PSA) device, a cryogenic separation device, or a membrane separation device can be used.

At least a part of a gas component containing hydrogen and an unreacted hydrocarbon separated by the solid separation device can be separated into hydrogen and a gas component containing an unreacted hydrocarbon by the gas purification device.

Hydrogen separated by the gas purification device can also be used as a raw material for other chemical products and fuel. The gas component containing an unreacted hydrocarbon separated from hydrogen by the gas purification device may be supplied again to the decomposition reaction device as a raw material. As a result, it is possible to improve a conversion ratio of a hydrocarbon.

Examples of carbon dioxide include carbon dioxide recovered from the atmosphere and carbon dioxide recovered from various plants, incinerators, and the like discharging carbon dioxide. Examples of the carbon dioxide recovered from various plants discharging carbon dioxide include carbon dioxide recovered from a flue gas of a steam reforming furnace by a chemical absorption method, a membrane separation method, or a pressure swing adsorption method (PSA method), carbon dioxide obtained by a carbon dioxide gas recovery step of an ammonia plant, carbon dioxide recovered from a blast furnace gas or a coke oven gas by-produced in a steel mill by a chemical absorption method, a membrane separation method, or a pressure swing adsorption method (PSA method), carbon dioxide recovered from an exhaust gas obtained by combustion of fuel in a thermal power plant by a chemical absorption method, a membrane separation method, or a pressure swing adsorption method (PSA method), and carbon dioxide recovered from an incineration facility of waste or the like by the above-described methods and obtained by a carbon dioxide gas recovery step of an ammonia plant. Note that, when a gas containing carbon dioxide contains impurities such as a chlorine compound, carbon dioxide from which impurities have been removed in advance is used.

In step (21), carbon dioxide is caused to react with hydrogen to obtain a reaction mixture containing an alcohol, carbon dioxide, and carbon monoxide. The alcohol contained in the reaction mixture refers to any compound in which one or more hydrogen atoms of a hydrocarbon are replaced with hydroxy groups (-OH). The alcohol may be linear, branched, or cyclic, but is preferably a linear or branched saturated aliphatic alcohol. In addition, the alcohol is preferably a monohydric alcohol having one hydroxy group. In addition, a C1 to C5 alcohol is preferable, and a C1 to C4 alcohol is more preferable. Examples of the alcohol include methanol, ethanol, 1-propanol, 2-propanol, 1-butanol, 2-butanol, 2-methyl-1-propanol, and 2-methyl-2-propanol.

Raw materials in step (21) are hydrogen and carbon dioxide. Therefore, step (21) is almost similar to step (14) of the second embodiment, but is different from step (14) of the second embodiment in that, in the case where only carbon dioxide is used as a raw material, a reaction product contains a larger amount of water and an equilibrium conversion ratio is lower than in the case where carbon monoxide is contained. When the amount of water in the reaction product is large, a catalyst tends to be deactivated, and therefore it is necessary to use a catalyst having strong water resistance. In order to cope with the problem of low equilibrium conversion ratio, it is preferable to actively remove an alcohol and water generated in the reaction. Therefore, a reactor preferably has a condensation surface that condenses the above-described high-boiling component containing a generated alcohol and water in the reactor. As a result, a reaction mixture containing an alcohol, carbon dioxide, and carbon monoxide can be obtained.

Step (22) is a step of obtaining a C2-C8 unsaturated hydrocarbon from an alcohol and/or carbon monoxide contained in the reaction mixture. Note that, as described above, the C2-C8 unsaturated hydrocarbon obtained in step (22) is a C2-C5 olefin such as ethylene, propylene, butene, or pentene, a C4 or C5 diene such as butadiene or pentadiene, or a C6-C8 aromatic hydrocarbon such as benzene, toluene, or xylene, and is preferably a C2-C4 olefin.

A method for producing a C2-C8 unsaturated hydrocarbon from an alcohol and/or carbon monoxide is not particularly limited, and may be a known method.

A temperature of the step of producing a C2-C8 unsaturated hydrocarbon from an alcohol and/or carbon monoxide is preferably 200 to 400°C, and more preferably 250 to 350°C. In addition, a pressure of the step of producing a C2-C8 unsaturated hydrocarbon from an alcohol and/or carbon monoxide is preferably normal pressure to 10 MPa, more preferably 0.1 to 10 MPa, and particularly preferably 0.3 to 5 MPa.

As a method for producing a C2-C8 unsaturated hydrocarbon from an alcohol and/or carbon monoxide, for example, a method using a catalyst containing iron and cobalt, described in JP-A-2014-55126, a method using a catalyst containing iron, cobalt, and nickel or ruthenium, described in Republished Patent No. 2014/024774, or a method using a catalyst having a core-shell type structure in which a solid containing at least iron and manganese is used as a core and a solid containing at least silicon and a zeolite containing a Group 13 element is used as a shell, described in JP-A-2022-30433 can be used. In addition, a catalyst described in "Carbon Recycling Technology in Catalyst 2021" (CMC Research, published on April 20, 2021) p210 to 214 can also be preferably used.

The reaction product containing a C2-C8 unsaturated hydrocarbon is separated and purified into a plurality of products having different boiling point differences by purification. The purification may be disposed in a process of the method for producing a C2-C8 unsaturated hydrocarbon according to the fourth embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification.

[Fifth embodiment of method for producing C2-C8 unsaturated hydrocarbon]

A method for producing a C2-C8 unsaturated hydrocarbon according to a fifth embodiment is a combination of the method for producing a C2-C8 unsaturated hydrocarbon according to any one of the first to third embodiments and the method for producing a C2-C8 unsaturated hydrocarbon according to the fourth embodiment.
One aspect of the fifth embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon; step (3) of cleaning and purifying the reaction product to separate the C2-C8 unsaturated hydrocarbon; step (21) of causing carbon dioxide and hydrogen to react with each other to obtain a reaction mixture containing an alcohol, carbon dioxide, and carbon monoxide; and step (22) of obtaining a C2-C8 unsaturated hydrocarbon from the alcohol and/or carbon monoxide contained in the reaction mixture.
Another aspect of the fifth embodiment includes: step (1) of sorting, from a waste plastic, a plastic mixture (A) having a polyolefin-based plastic content of 50 mass% or more and a residue (B); step (12) of heating the residue (B) to obtain a mixed gas containing carbon monoxide, carbon dioxide, and hydrogen; step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; step (15) of obtaining a C2-C8 unsaturated hydrocarbon using the alcohol as a raw material; step (21) of causing carbon dioxide and hydrogen to react with each other to obtain a reaction mixture containing an alcohol, carbon dioxide, and carbon monoxide; and step (22) of obtaining a C2-C8 unsaturated hydrocarbon from the alcohol and/or carbon monoxide contained in the reaction mixture.
A still another aspect of the fifth embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon; step (3) of cleaning and purifying the reaction product to separate the C2-C8 unsaturated hydrocarbon; step (12) of heating the residue (B) to obtain a mixed gas containing carbon monoxide, carbon dioxide, and hydrogen; step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; step (15) of obtaining a C2-C8 unsaturated hydrocarbon using the alcohol as a raw material; step (21) of causing carbon dioxide and hydrogen to react with each other to obtain a reaction mixture containing an alcohol, carbon dioxide, and carbon monoxide; and step (22) of obtaining a C2-C8 unsaturated hydrocarbon from the alcohol and/or carbon monoxide contained in the reaction mixture.
The steps (1) to (3), the steps (12) to (15), and the steps (21) and (22) of the fifth embodiment are similar to the steps (1) to (3) of the first embodiment, the steps (12) to (15) of the second embodiment, and the steps (21) and (22) of the fourth embodiment described above, respectively.
The reaction product containing a C2-C8 unsaturated hydrocarbon is separated and purified into a plurality of products having different boiling point differences by purification. The purification may be disposed in a process of the method for producing a C2-C8 unsaturated hydrocarbon according to the fifth embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification.

[Sixth embodiment of method for producing C2-C8 unsaturated hydrocarbon]

A method for producing a C2-C8 unsaturated hydrocarbon according to a sixth embodiment is a combination of the method for producing a C2-C8 unsaturated hydrocarbon according to any one of the first to fourth embodiments, and a method for producing a C2-C8 unsaturated hydrocarbon including step (31) of subjecting at least one alcohol selected from the group consisting of ethanol, propanol, and butanol to a dehydration reaction to obtain a C2-C8 unsaturated hydrocarbon.
One aspect of the sixth embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon; step (3) of cleaning and purifying the reaction product to separate the C2-C8 unsaturated hydrocarbon; and step (31) of subjecting at least one alcohol selected from the group consisting of ethanol, propanol, and butanol to a dehydration reaction to obtain a C2-C8 unsaturated hydrocarbon.

Another aspect of the sixth embodiment includes: step (1) of sorting, from a waste plastic, a plastic mixture (A) having a polyolefin-based plastic content of 50 mass% or more and a residue (B); step (12) of heating the residue (B) to obtain a mixed gas containing carbon monoxide, carbon dioxide, and hydrogen; step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; step (15) of obtaining a C2-C8 unsaturated hydrocarbon using the alcohol as a raw material; and step (31) of subjecting at least one alcohol selected from the group consisting of ethanol, propanol, and butanol to a dehydration reaction to obtain a C2-C8 unsaturated hydrocarbon.

A still another aspect of the sixth embodiment includes: step (1) of sorting, from a waste plastic, a plastic mixture (A) having a polyolefin-based plastic content of 50 mass% or more and a residue (B); step (2) of decomposing the plastic mixture (A) to obtain a reaction product containing a C2-C8 unsaturated hydrocarbon; step (3) of cleaning and purifying the reaction product to separate the C2-C8 unsaturated hydrocarbon; step (12) of heating the residue (B) to obtain a mixed gas containing carbon monoxide, carbon dioxide, and hydrogen; step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; step (15) of obtaining a C2-C8 unsaturated hydrocarbon using the alcohol as a raw material; and step (31) of subjecting at least one alcohol selected from the group consisting of ethanol, propanol, and butanol to a dehydration reaction to obtain a C2-C8 unsaturated hydrocarbon.

A still another aspect of the sixth embodiment includes: step (21) of causing carbon dioxide and hydrogen to react with each other to obtain a reaction mixture containing an alcohol, carbon dioxide, and carbon monoxide; step (22) of obtaining a C2-C8 unsaturated hydrocarbon from the alcohol and/or carbon monoxide contained in the reaction mixture; and step (31) of subjecting at least one alcohol selected from the group consisting of ethanol, propanol, and butanol to a dehydration reaction to obtain a C2-C8 unsaturated hydrocarbon.

The steps (1) to (3), the steps (12) to (15), and the steps (21) and (22) of the sixth embodiment are similar to the steps (1) to (3) of the first embodiment, the steps (12) to (15) of the second embodiment, and the steps (21) and (22) of the fourth embodiment described above, respectively.

Step (31) is a step of subjecting an alcohol to a dehydration reaction to obtain a C2-C8 unsaturated hydrocarbon. The alcohols used in step (31) are ethanol, propanol, and butanol.

Ethanol, propanol, and butanol used in step (31) may be produced from a synthesis gas by a chemical method using a metal catalyst, or may be produced by a fermentation method.

Examples of the metal catalyst include a hydrogenated active metal and an aggregate of a hydrogenated active metal and an auxiliary active metal. The hydrogenated active metal may be any metal that is conventionally known as a metal capable of synthesizing ethanol from a mixed gas, and examples thereof include an alkali metal such as lithium or sodium, an element belonging to Group 7 of the periodic table, such as manganese or rhenium, an element belonging to Group 8 of the periodic table, such as ruthenium, an element belonging to Group 9 of the periodic table, such as cobalt or rhodium, and an element belonging to Group 10 of the periodic table, such as nickel or palladium.

These hydrogenated active metals may be used singly or in combination of two or more types thereof. The hydrogenated active metal is preferably a combination of rhodium or ruthenium, an alkali metal, and another hydrogenated active metal, such as a combination of rhodium, manganese, and lithium or a combination of ruthenium, rhenium, and sodium from a viewpoint of further improving a carbon monoxide conversion ratio and improving an ethanol selectivity.

Examples of the auxiliary active metal include titanium, magnesium, and vanadium. By the auxiliary active metal being supported in addition to the hydrogenated active metal, the carbon monoxide conversion ratio, the ethanol selectivity, and the like can be further increased.

Furthermore, the catalyst used in step (14) is also a catalyst preferable for the present reaction.

In production of an alcohol by a fermentation method, cultivation is typically performed in a bioreactor. Note that the term "bioreactor" includes a fermentation device including one or more containers, columns, piping structures, and the like such as a continuous stirred tank reactor (CSTR), an immobilized cell reactor (ICR), a trickle bed reactor (TBR), a bubble column, a gas lift fermenter, a static mixer, and another container or another device preferable for gas-liquid contact. In one aspect, the bioreactor may include a first growth reactor and a second fermentation reactor. A raw material of an alcohol is supplied to one or both of these reactors.

As the bioreactor, a bioreactor having any shape can be used, and examples thereof include a stirring type, an airlift type, a bubble column type, a loop type, an open bond type, and a photobio type. The bioreactor is preferably a known loop type reactor having a main container portion and a reflux portion.

As a raw material used in the fermentation method, crops such as sugar cane, sugar beet, corn, wheat and barley, rice, sweet potato, and potato, and lignocellulose-based raw materials that are not edible can be used, but a lignocellulose-based raw material is preferable. The lignocellulose-based raw material contains cellulose and hemicellulose (hereinafter, also referred to as celluloses), and an alcohol can be obtained by decomposing celluloses into sugars such as glucose and xylose by a saccharifying enzyme and fermenting the obtained sugars.

Examples of the lignocellulose-based raw material include wood, rice straw, wheat straw, bagasse, bamboo, corn (stem, leaf, core, and the like), pulp, and waste generated therefrom (for example, waste paper). The lignocellulose-based raw material may be used singly or in mixture of two or more types thereof.

Examples of the pulp include a wood pulp obtained from softwood, hardwood, forest residues, construction waste, and the like, a non-wood pulp such as cotton linter or cotton lint, hemp, wheat straw, or bagasse, and a waste paper pulp or a deinked pulp obtained from waste paper as a raw material. As a method for producing a pulp, a method for highly removing lignin, such as a chemical pulp production method such as alkali extraction or alkali digestion, is preferable, and among pulps produced by the chemical pulp production method, a pulp for papermaking is preferable from a viewpoint of availability. Examples of the pulp for papermaking include a chemical pulp such as a hardwood kraft pulp (for example, a bleached kraft pulp (LBKP), an unbleached kraft pulp (LUKP), or an oxygen bleached kraft pulp (LOKP)), a softwood kraft pulp (for example, a bleached kraft pulp (NBKP), an unbleached kraft pulp (NUKP), or an oxygen bleached kraft pulp (NOKP)), a sulfite pulp (SP), or a soda pulp (AP), and a semi-chemical pulp such as a semi-chemical pulp (SCP) or a chemi-ground wood pulp (CGP). The pulp is preferably a hardwood kraft pulp, a softwood kraft pulp, a sulfite pulp (SP), or a semi-chemical pulp (SCP), and more preferably a hardwood bleached kraft pulp (LBKP) or a softwood oxygen bleached kraft pulp (NOKP).

Among lignocellulose-based raw materials other than a pulp, examples of a wood-based lignocellulose-based raw material include chips or barks of trees for papermaking, forest residues, and thinned wood, sprouts generated from tree stumps of woody plants, sawdust generated from a sawmill, pruned branches of street trees, and building waste. As the wood-based lignocellulose-based raw material, a plant of the genus Eucalyptus, a plant of the genus Salix, a plant of the genus Popla, a plant of the genus Acacia, a plant of the genus Cryptomeria, and the like can be used. Among these plants, the plant of the genus Eucalyptus, the plant of the genus Acacia, and the plant of the genus Salix are preferable because they are easily collected in large amounts as raw materials. Examples of a herbaceous lignocellulose-based raw material include agricultural waste such as kenaf, rice straw, wheat straw, corn cob, or bagasse, residues and waste of oil crops and craft crops such as rubber (for example, empty fruit bunch (EFB)), and erianthus, miscanthus, and napier grass of herbaceous energy crops.

The lignocellulose-based raw material other than a pulp may be biomass. Examples of the biomass include wood-derived paper, waste paper, pulp sludge, sludge, sewage sludge, and food waste. These types of biomass can be used singly or in combination of two or more types thereof. In addition, the biomass may be a dry solid, a solid containing water, or a slurry.

In addition, the lignocellulose-based raw material used in the fermentation method may be pretreated. Examples of the pretreatment include a coarse pulverization treatment, a blasting treatment, and hydrolysis. In the coarse pulverization treatment, the lignocellulose-based raw material is coarsely pulverized by a crusher or a pulverizer. At this time, the lignocellulose-based raw material is preferably formed into small pieces having an average diameter of 10 to 100 mm or less. The coarsely pulverized lignocellulose-based raw material is subjected to a digestion treatment using an aqueous solution of a metal hydroxide such as sodium hydroxide. By this digestion treatment, a part of lignin contained in bagasse is removed, and reactivity of cellulose and hemicellulose with an enzyme is improved. The biomass after the digestion treatment is neutralized using an acid.

In the blasting treatment, saccharification of a hemicellulose component of the lignocellulose-based raw material and partial removal of lignin are performed. The lignocellulose-based raw materials treated with a blasting device include a C5 saccharified liquid derived from hemicellulose, a lignin lysate, and a solid residue. When the C5 saccharified liquid is subjected to hemicellulose saccharide fermentation separately from a C6 saccharified liquid, the blasted product is subjected to solid-liquid separation by filter press or the like, and divided into a blasted liquid and a solid residue. By further appropriately cleaning the solid residue with water, saccharides contained in the solid residue can be recovered. Note that, when the C5 saccharide fermentation and the C6 saccharide fermentation are simultaneously performed, solid-liquid separation is performed optionally. Note that conditions of the blasting treatment are, for example, 200 to 240°C, 1.5 to 4 MPa, and 1 to 15 min, and preferably 225 to 230°C, 2.5 to 3 MPa, and 1 to 5 min.

The solid residue obtained by the blasting treatment is cleaned with water and the saccharides and the lignin lysate are removed therefrom. Thereafter, the resulting solid residue is immersed in ethanol water having an ethanol concentration of 30% or more, preferably 50% or more at normal temperature for 0.5 hours or more and 48 hours or less, preferably one hour or more and 24 hours or less, and lignin covering cellulose is dissolved and removed. After the immersion in ethanol, the solid residue is subjected to a deethanol treatment mechanically or subjected to the deethanol treatment by heating, heating under reduced pressure, or the like. When high-concentration ethanol water is used, the deethanol treatment may be performed after the solid residue is cleaned with water once.

Hemicellulose contained in the lignocellulose-based raw material is hydrolyzed by high-temperature and high-pressure water or decomposed by a hemicellulose hydrolase such as hemicellulase. When hemicellulose is hydrolyzed with high-temperature and high-pressure water, the hemicellulose can be decomposed into saccharides (mainly a C5 monosaccharide) at 140°C or higher and 180°C or lower. In a case of biomass having a high hemicellulose content, a C5 monosaccharide is excessively decomposed into an organic acid and the like when treated at a high temperature, and therefore it is preferable to perform the decomposition treatment under relatively mild conditions. Note that, when hemicellulose is hydrolyzed with high-temperature and high-pressure water, it is also preferable to add an acid such as phosphoric acid or hydrochloric acid as a catalyst. When hemicellulose is hydrolyzed by a hemicellulose hydrolase, a commercially available enzyme may be used, or a microorganism or the like that generates hemicellulase may be used.

A carbon source of a fermentation reaction in the fermentation method is a gaseous substrate containing carbon monoxide. The gaseous substrate may be a by-product of an industrial process or a carbon monoxide-containing waste gas obtained from an automotive exhaust gas. Examples of the industrial process include ferrous metal product production such as a steel mill, nonferrous product production, a petroleum refining process, coal gasification, power generation, carbon black generation, ammonia generation, methanol generation, and coke production. The gaseous substrate can be captured from the industrial process before being released to the atmosphere using any convenient method. Depending on the composition of the gaseous substrate, it can also be desirable to treat the gaseous substrate in order to remove any unnecessary impurities such as dust particles before introduction of the gaseous substrate into fermentation. For example, the gaseous substrate can be filtered or cleaned using a known method.

In addition, the gaseous substrate may be supplied from biomass gasification. A process of the gasification includes partial combustion of biomass under limited supply of air or oxygen. The obtained gas typically contains mainly carbon monoxide and hydrogen, with minimal amounts of carbon dioxide, methane, ethylene, and ethane. For example, food such as sugar from sugar cane or starch from corn or cereals, or a biomass by-product obtained during extraction and treatment of non-food biomass waste generated by forestry can be gasified in order to generate a gaseous substrate.

The gaseous substrate contains, for example, at least 20 to 100 vol%, 43 to 95 vol%, 60 to 90 vol%, or 70 to 90 vol% of carbon monoxide. In one aspect, the gaseous substrate contains 25 vol%, 30 vol%, 35 vol%, 40 vol%, 45 vol%, or 50 vol% of carbon monoxide. Particularly when hydrogen and carbon dioxide are also present, a gaseous substrate containing carbon monoxide with a lower concentration, for example, 6 vol% of carbon monoxide can also be suitable.

In addition, an industrial combustion exhaust gas or the like containing carbon dioxide and hydrogen may be applied as the gaseous substrate. The industrial combustion exhaust gas is, for example, an exhaust gas from a hydrogen production plant. The exhaust gas from the hydrogen production plant contains 50 to 60% of carbon dioxide, 20 to 30% of hydrogen, 5 to 15% of carbon monoxide, and 5 to 15% of methane. In addition, the exhaust gas in ammonia production is rich in carbon dioxide and hydrogen. Furthermore, examples of the industrial combustion exhaust gas include gases generated from treatment of any carbon-based raw material such as oil, coal, or biomass.

Processes for generation of ethanol and other alcohols from the gaseous substrate are known. Exemplary processes include those described, for example, in WO-A-2007/117157, WO-A-2008/115080, US-B2-6,340,581, US-B2-6,136,577, US-B2-5,593,886, US-B2-5807,722, and US-B2-5,821,111, each of which is incorporated herein by reference.

The gaseous substrate may be derived from waste. The waste may be industrial waste such as industrial solid waste or general waste such as municipal solid waste (MSW), and examples thereof include combustible substances such as plastic waste, garbage, waste tires, biomass waste, food waste, building materials, wood, wood chips, fibers, and paper. As the waste, municipal solid waste (MSW) is preferable.

The gaseous substrate is preferably a synthesis gas containing carbon monoxide and hydrogen. Hereinafter, an example in which the waste-derived gas is a synthesis gas will be described in detail. The synthesis gas can be obtained by performing a source gas generation step of generating a source gas by gasifying waste, and further performing a synthesis gas purification step of removing or reducing specific substances such as various contaminants, soot particles, impurities, and undesired amounts of compounds from the generated source gas.

In the source gas generation step, for example, a gasification furnace is preferably used for the gasification of waste. The gasification furnace is a furnace that burns (incompletely burns) a carbon source. Examples of the gasification furnace include a shaft furnace, a kiln furnace, a fluidized bed furnace, a gasification reforming furnace, and a plasma gasification furnace. A temperature at which waste is gasified into the source gas is not particularly limited, but is usually 100 to 2500°C and preferably 200 to 2100°C.

The source gas obtained by gasifying waste preferably contains carbon monoxide and hydrogen, but may further contain carbon dioxide, oxygen, and nitrogen. In addition, the source gas may further contain components such as soot, tar, a nitrogen compound, a sulfur compound, a phosphorus-based compound, and an organic compound. The source gas typically contains 0.1 to 80 vol% of carbon monoxide and 0.1 to 80 vol% of hydrogen. In addition, the source gas preferably contains 0.1 to 70 vol% of carbon dioxide.

The source gas may be generated as a gas containing, although not particularly limited, 0.1 vol% or more, preferably 10 vol% or more, more preferably 20 vol% or more of carbon monoxide by performing a heat treatment (so-called gasification) for burning (incompletely burning) waste, that is, partially oxidizing waste.

The source gas is preferably formed into the synthesis gas by removing or reducing specific substances such as various contaminants, soot particles, impurities, and undesired amounts of compounds.

In the synthesis gas purification step, the source gas may be purified by performing the treatment using, for example, one or more of a water separation device such as a gas chiller, a low-temperature separation type (cryogenic type) separation device, a fine particle separation device that separates fine particles such as soot with various filters such as a cyclone and a bag filter, a water-soluble impurity separation device such as a scrubber, a desulfurization device (sulfide separation device), a membrane separation type separation device, a deoxidation device, a pressure swing adsorption type separation device (PSA), a temperature swing adsorption type separation device (TSA), a pressure temperature swing adsorption type separation device (PTSA), a separation device using activated carbon, and a separation device using a deoxidation catalyst, specifically, a copper catalyst or a palladium catalyst to obtain the synthesis gas.

When an alcohol is obtained by a fermentation method, it is preferable to reduce a carbon dioxide gas concentration in the source gas. For example, using a pressure swing adsorption type separation device filled with a regenerated adsorbent containing zeolite, a carbon dioxide gas in the synthesis gas is preferably adsorbed to the regenerated adsorbent to reduce the carbon dioxide gas concentration in the synthesis gas.

The obtained synthesis gas contains at least carbon monoxide and hydrogen, and may further contain carbon dioxide and nitrogen. A carbon monoxide concentration in the synthesis gas is 20 to 80 vol%, preferably 25 to 50 vol%, and more preferably 30 to 45 vol% with respect to the total concentration of carbon monoxide, carbon dioxide, hydrogen, and nitrogen in the synthesis gas.

A hydrogen concentration in the synthesis gas is usually 10 to 80 vol%, preferably 30 to 55 vol%, and more preferably 30 to 50 vol% with respect to the total concentration of carbon monoxide, carbon dioxide, hydrogen, and nitrogen in the synthesis gas.

The carbon dioxide concentration in the synthesis gas is not particularly limited, but is usually 0.1 vol% or more and 40 vol% or less, and preferably 0.3 vol% or more and 30 vol% or less with respect to the total concentration of carbon monoxide, carbon dioxide, hydrogen, and nitrogen in the synthesis gas. The carbon dioxide concentration is particularly preferably reduced when ethanol is generated by microbial fermentation, and is more preferably 0.5 vol% or more and 25 vol% or less from such a viewpoint.

A nitrogen concentration in the synthesis gas is 40 vol% or less, preferably 1 to 20 vol%, and more preferably 5 to 15 vol% with respect to the total concentration of carbon monoxide, carbon dioxide, hydrogen, and nitrogen in the synthesis gas.

The concentrations of carbon monoxide, carbon dioxide, hydrogen, and nitrogen in the synthesis gas can be in predetermined ranges by appropriately changing combustion conditions such as the type of waste, a gasification temperature in the source gas generation step, and an oxygen concentration of a supply gas at the time of gasification. For example, when it is desired to change the concentration of carbon monoxide or hydrogen, there is a method using waste containing a hydrocarbon (carbon and hydrogen) at a high ratio, such as waste plastic, and when it is desired to reduce the nitrogen concentration, there is a method for supplying a gas having a high oxygen concentration in the source gas generation step.

Furthermore, in at least one of the source gas and the synthesis gas, the concentration of each of the components of carbon monoxide, carbon dioxide, hydrogen, and nitrogen may be appropriately adjusted. For the concentration adjustment, at least one of these components is preferably added to the source gas or the synthesis gas. An addition amount is, for example, less than 50 vol%, preferably less than 30 vol%, and more preferably less than 10 vol% with respect to the total amount of the source gas or the synthesis gas.

The alcohol generated by the fermentation method varies depending on a microorganism. Each of ethanol, propanol, and butanol will be described below.

Generation of ethanol in the fermentation method will be described. Examples of a microorganism that generates ethanol by fermentation include yeast and bacteria. The yeast can preferably ferment saccharides (six-carbon sugar and five-carbon sugar). Specific examples of the yeast include a yeast of the genus Saccharomyces such as Saccharomyces cerevisiae, a yeast of the genus Pichia such as Pichia stipitis, a yeast of the genus Candida such as Candida shihatae, a yeast of the genus Pachysolen such as Pachysolen tannophilus, and a yeast of the genus Issatchenkia such as Isatchenkia orientalis. The yeast is preferably a yeast belonging to the genus Saccharomyces or Issatchenkia, and more preferably Saccharomyces cerevisiae or Isatchenkia orientalis. It is also possible to use a genetically modified yeast produced using a genetic recombination technique. As the genetically modified yeast, a yeast capable of simultaneously fermenting a six-carbon sugar and a five-carbon sugar can be used without particular limitation.

Certain anaerobic microorganisms are cultured in a liquid medium. For example, a liquid culture medium and gas-assimilating bacteria may be supplied and stored, and the synthesis gas may be supplied into a bioreactor while the liquid culture medium is stirred in this state. As a result, it is possible to culture the gas-assimilating bacteria in the liquid medium and generate ethanol from the synthesis gas by a fermentation action of the gas-assimilating bacteria.

In the bioreactor, a temperature of the culture medium (culture temperature) may be any temperature, but can be preferably 30 to 45°C, more preferably 33 to 42°C, and still more preferably 36.5 to 37.5°C. In addition, a culture time is preferably 1 hour or more, more preferably 7 days or more, particularly preferably 30 days or more, and most preferably 60 days or more in continuous culture, and an upper limit thereof is not particularly set, but is preferably 720 days or less and more preferably 365 days or less from a viewpoint of regular maintenance of facilities or the like. Note that the culture time means a time from when an inoculum is added to a culture tank to when the entire amount of culture liquid in the culture tank is discharged.

Some anaerobic bacteria are known to perform fermentations from carbon dioxide and hydrogen to an alcohol containing ethanol and acetic acid. Acetogen has ability to convert a gaseous substrate to acetic acid, ethanol, and other fermentation products via a Wood-Ljungdahl pathway. Examples of such bacteria include a strain of Acetobacterium Woodii which is a bacterium belonging to the genus Acetobacterium (Demler, M., Weuster-Botz, "Reaction Engineering Analysis of Hydrogenotrophic Production of Acetic Acid by Acetobacterum Woodii", Biotechnology and Bioengineering, Vol. 108, No. 2, February 2011). It has been indicated that Acetobacterium Woodii generates an acetate by fermentation of a gaseous substrate containing carbon dioxide and hydrogen. Buschhorn et al. demonstrated ability of A Woodii to generate ethanol in glucose fermentation using phosphate restriction.

In addition, some anaerobic bacteria are known to ferment carbon monoxide to ethanol. Such bacteria are preferably bacteria having a metabolic pathway of acetyl COA. Examples of such bacteria include Clostridium autoethanogenum, Clostridium ljungdahlii, Clostridium aceticum, and Clostridium carboxidivorans, which belong to the genus Clostridium, Moorella thermoacetica, and Acetobacterium Woodii. Among these bacteria, Clostridium autoethanogenum is preferable.

The fermentation is desirably performed under suitable conditions for causing the fermentation. Reaction conditions considered include a pressure, a temperature, a gas flow rate, a liquid flow rate, fermentation liquid pH, a fermentation liquid redox potential, a stirring rate (when a continuous stirred tank reactor is used), an inoculum level, a maximum gas substrate concentration to ensure that carbon dioxide in a liquid phase is not limited, and a maximum product concentration to avoid product inhibition. Preferable conditions are described in WO-A-2002/08438, WO-A-2007/117157, and WO-A-2008/115080.

Optimal fermentation conditions partially depend on specific microorganism used. However, in general, the fermentation is preferably performed at a pressure higher than an ambient pressure. By operation at an increased pressure, a large increase in a carbon dioxide transfer rate from a gas phase to a liquid phase is possible, and carbon dioxide can be incorporated into microorganisms as a carbon source for generation of ethanol. On the other hand, this means that a holding time (defined as one obtained by dividing a liquid volume in the bioreactor by an input gas flow rate) can be reduced when the bioreactor is maintained at a pressure higher than atmospheric pressure.

In addition, an introduction rate of a gaseous substrate containing carbon dioxide and hydrogen is preferably such a rate as to ensure that concentrations of carbon dioxide and hydrogen in the liquid phase are not limited. This is because the ethanol product may be consumed by a cultured product as a result of carbon dioxide and hydrogen limiting conditions.

A temperature of a fermentation liquid in the bioreactor is not particularly limited as long as the temperature is in an optimum temperature range at which fermentation is performed, but is preferably 20 to 40°C and more preferably 30 to 40°C. The optimal temperature for the fastest growth of bacteria and the highest generation rate of an acetate can be determined by running the bioreactor at various different temperature points. The bioreactor is initially operated at 30°C, and a temperature thereof is increased to a plurality of different temperatures. An optimal temperature for the fastest growth of bacteria is at least 32°C, at least 33°C, at least 34°C, at least 35°C, or at least 36°C.

pH of the fermentation liquid in the bioreactor is not particularly limited, but is preferably maintained in a range of 3 to 10, and more preferably maintained in a range of 4 to 8.

Fermentation is performed in the bioreactor to obtain a fermentation liquid containing ethanol. The fermentation liquid containing ethanol is then subjected to a separation step. In the separation step, for example, the ethanol-containing fermentation liquid is heated to 23 to 500°C under a condition of 0.01 to 1000 kPa (absolute pressure) to be separated into a liquid or solid component containing microorganisms and an ethanol-containing gas component. By performing such a separation step, foaming does not occur in a distillation device in a distillation operation at the time of separation and purification of ethanol described later. Therefore, the distillation operation can be continuously performed. In addition, ethanol can be efficiently separated and purified at the time of separation and purification described later.

In the separation step, the ethanol-containing culture liquid is heated at a temperature of preferably 50 to 200°C, more preferably 80°C to 180°C, still more preferably 100 to 150°C under a condition of preferably 10 to 200 kPa, more preferably 50 to 150 kPa, still more preferably normal pressure from a viewpoint of efficiently separating the ethanol-containing fermentation liquid into a liquid or solid component containing microorganisms, dead bodies thereof, proteins derived from microorganisms, and the like, and an ethanol-containing gas component.

The ethanol-containing gas component obtained in the separation step is preferably liquefied by condensation to form an ethanol-containing liquid (liquefaction step). A device used in the liquefaction step is not particularly limited, but it is preferable to use a heat exchanger, particularly a condenser. Examples of the condenser include a water cooling type, an air cooling type, and an evaporation type, and a water cooling type is preferable. The condenser may have one stage or a plurality of stages.

In addition, in the separation step, instead of separating the ethanol-containing fermentation liquid into a liquid or solid component containing microorganisms and an ethanol-containing gas component, a solid component containing microorganisms and an ethanol-containing liquid component may be separated by a solid-liquid separation device such as a solid-liquid separation filter device.

After the separation step, a purification step of further purifying the ethanol-containing liquid may be performed. In addition, in a case where components such as microorganisms have already been removed from the ethanol-containing liquid obtained by microbial fermentation, the purification step may be performed without the separation step described above.

The purification step is a step of separating the ethanol-containing liquid into a distillate having an increased ethanol concentration and a column bottom liquid having a decreased ethanol concentration. Examples of a device used in the purification step include a distillation device, a treatment device including a pervaporation membrane, a treatment device including a zeolite membrane, a treatment device for removing a low-boiling-point substance having a boiling point lower than that of ethanol, a treatment device for removing a high-boiling-point substance having a boiling point higher than that of ethanol, and a treatment device including an ion exchange membrane. These devices may be used singly or in combination of two or more types thereof. As a unit operation, a distillation device or membrane separation can be preferably used, and a distillation device is more preferable. As the membrane separation, a zeolite membrane can be preferably used.

When a distillation device is used, heating distillation is performed. In the heating distillation, desired ethanol can be obtained as a distillate with high purity. A temperature in the distillation device during distillation of ethanol is not particularly limited, but is preferably 110°C or lower, and more preferably about 70 to 105°C. By setting the temperature in the distillation device in the above range, separation of ethanol and other components, that is, distillation of ethanol can be performed more reliably.

In the heating distillation, the ethanol-containing liquid may be introduced into a distillation device equipped with a heater using steam at 100°C or higher, ae temperature of a bottom of a distillation column may be raised to 90°C or higher within 30 minutes, and then the ethanol-containing liquid may be introduced from a middle of the distillation column. In addition, in the heating distillation using a distillation device, it is preferable to perform a distillation step within a temperature difference of $\pm 15°C$ among a column bottom, a column middle, and a column top. When the temperature difference is within $\pm 15°C$, high-purity ethanol is easily obtained. The distillation temperature difference is preferably $\pm 13°C$, and more preferably $\pm 11°C$. With these distillation temperature differences, separation from other components, that is, purification of ethanol by distillation can be performed more reliably.

A pressure in the distillation device at the time of distillation of ethanol may be normal pressure, but is preferably lower than atmospheric pressure, and more preferably about 60 to 95 kPa (absolute pressure). By setting the pressure in the distillation device to the above range, it is possible to improve separation efficiency of ethanol and therefore a yield of ethanol.

Ethanol obtained in the purification step is further subjected to a distillation treatment or concentration using various separation membranes such as a zeolite membrane, whereby high-purity ethanol can be generated. In addition, the ethanol-containing liquid may be transferred to a bioreactor after removal of ethanol.

Ethanol obtained through the purification step is used as a raw material for obtaining an ethylene-containing product in generation of ethylene described later. "Ethanol" used as a raw material in the present invention does not mean pure ethanol (ethanol represented by chemical formula: $CH_3CH_2OH$) as a compound, but is a composition containing impurities, and is also referred to as "raw material ethanol". The impurities are contained in the raw material ethanol produced through the above-described steps, and most of the impurities are compounds derived from waste.

Note that the purification step may be omitted. That is, after generation of ethanol, as the purification step, both the above purification step and purification in step (31') described later do not have to be performed, and only the purification in step (31') may be performed. In this case, the ethanol-containing liquid serves as raw material ethanol, and the purification in step (31') is preferably performed by a distillation device or membrane separation, and heating distillation using a distillation device is particularly preferable. Of course, when purification in step (31'') described later is performed, both the purification step and the purification in step (31') may be omitted. Note that it is preferable to perform the purification step, and it is more preferable to perform the purification in step (31') together.

The raw material ethanol has an ethanol purity (that is, ethanol content) of, for example, 85 vol% or more. When the ethanol purity is the lower limit value or more, a polymerization reaction using ethylene as a raw material preferably proceeds through at least one of the purification in step (31') and the purification in step (31''), and quality of a polymer obtained from ethylene is favorable. The ethanol purity of the raw material ethanol is preferably 90 vol% or more, more preferably 95 vol% or more, and still more preferably 99.5 vol% or more. In addition, the raw material ethanol only needs to have an ethanol purity of less than 100 vol%. In addition, as the raw material ethanol, a commercially available product may be used as long as the commercially available product contains ethanol derived from waste.

Next, generation of propanol in the fermentation method will be described. In microbial fermentation of propanol, for example, any recombinant microorganism having ability to produce propanol may be used. The ability to produce propanol in the recombinant microorganism may be obtained by modifying (for example, enhancing) a propanol production pathway originally possessed by the microorganism, or may be obtained by newly introducing a propanol production pathway into the microorganism. Such modification and introduction can be performed by genetic recombination. Specifically, modification or introduction of a gene of an enzyme involved in the propanol production pathway and a DNA of a transcription regulator or the like can be performed using a known means such as a plasmid.

Preferable examples of the recombinant microorganism include E. coli having a propanol producing system for producing propanol. Examples thereof include propanol-producing E. coli described in WO-A-2009/008377, WO-A-2011/034031, WO-A-2011/111638, and WO-A-2012/020833. Since E. coli does not originally have a system for producing propanol, the propanol-producing E. coli is E. coli having propanol producing ability introduced or modified by genetic recombination. Such a propanol producing system may be any system as long as the propanol producing system causes target E. coli to produce propanol. In addition, at least a part of the propanol producing system only needs to be introduced or modified by genetic recombination. A known method can be used for introduction or modification by genetic recombination, and examples thereof include homologous recombination into a genome and introduction by a plasmid.

The propanol-producing E. coli is preferably E. coli having enhanced enzyme activity involved in production of propanol. The term "by genetic recombination" includes all those in which a change in a nucleotide sequence occurs due to insertion of an exogenous nucleotide sequence having a sequence different from a nucleotide sequence of a native gene, substitution or deletion of a certain portion of a gene, or a combination thereof, and includes, for example, those obtained as a result of mutation.

In the propanol-producing E. coli, preferably, four types of enzyme activities: an acetoacetate decarboxylase activity, a propanol dehydrogenase activity, a CoA transferase activity, and a thiolase activity are imparted from outside bacterial cells, expression thereof is enhanced in the bacterial cells, or both of these are performed.

Here, the thiolase is classified into enzyme number 2.3.1.9 in accordance with the report of the International Union of Biochemistry (I.U.B.) enzymatic committee, and refers to a generic term for enzymes that catalyze a reaction for generating acetoacetyl CoA from acetyl CoA. The acetoacetate decarboxylase is classified into enzyme number 4.1.1.4 in accordance with the report of the International Union of Biochemistry (I.U.B.) enzymatic committee, and refers to a generic term for enzymes that catalyze a reaction for generating acetone from acetoacetic acid.

Isopropanol dehydrogenase is classified into enzyme number 1.1.1.80 in accordance with the report of the International Union of Biochemistry (I.U.B.) enzymatic committee, and refers to a generic term for enzymes that catalyze a reaction for generating isopropanol from acetone. The CoA transferase is classified into enzyme number 2.8.3.8 in accordance with the report of the International Union of Biochemistry (I.U.B.) enzymatic committee, and refers to a generic term for enzymes that catalyze a reaction for generating acetoacetic acid from acetoacetyl CoA.

Examples of the propanol-producing E. coli having a propanol producing system include a pIPA/B strain and a pIaaa/B strain described in WO 2009/008377. In addition, examples of the E. coli include a strain (also described as pIa/B:: atoDAB strain) in which, among enzymes involved in production of isopropanol, the CoA transferase activity and the thiolase activity are enhanced by enhancing expression of each gene on a genome of the E. coli, and the isopropanol dehydrogenase activity and the acetoacetate decarboxylase activity are enhanced by enhancing expression of each gene with a plasmid.

Recombinant **E.** coli that has further effectively enhanced propanol productivity may be used, and examples thereof include inactivated GntR activity, inactivated glucose-6-phosphate isomerase (Pgi) activity, inactivated phosphogluconate dehydrogenase (Gnd) activity, and enhanced glucose-6-phosphate-1-dehydrogenase (Zwf) activity. These combinations can improve productivity of propanol as compared with combinations of other factors or enzymes.

The glucose-6-phosphate isomerase (Pgi) is classified into enzyme number 5.3.1.9 in accordance with the report of the International Union of Biochemistry (I.U.B.) enzymatic committee, and refers to a generic term for enzymes that catalyze a reaction for generating D-fructose-6-phosphate from D-glucose-6-phosphate.

The glucose-6-phosphate-1-dehydrogenase (Zwf) is classified into enzyme number 1.1.1.49 in accordance with the report of the International Union of Biochemistry (I.U.B.) enzymatic committee, and refers to a generic term for enzymes that catalyze a reaction for generating D-glucono-1,5-lactone-6-phosphate from D-glucose-6-phosphate.

As a gene of the glucose-6-phosphate-1-dehydrogenase (Zwf), a DNA having a nucleotide sequence of a gene encoding thiolase obtained from each of the above-described derived organisms or a synthetic DNA sequence synthesized on the basis of a known nucleotide sequence thereof can be used.

The phosphogluconate dehydrogenase (Gnd) is classified into enzyme number 1.1.1.44 in accordance with the report of the International Union of Biochemistry (I.U.B.) enzymatic committee, and refers to a generic term for enzymes that catalyze a reaction for generating D-ribulose-5-phosphate and carbon dioxide from 6-phospho-D-gluconic acid.

Examples of the propanol-producing E. coli include a strain in which GntR activity of a pIPA/B strain, a pIaaa/B strain, or a pIa/B:: atoDAB strain is inactivated, a strain in which GntR activity and glucose-6-phosphate isomerase (Pgi) activity of a pIa/B:: atoDAB strain are inactivated and the glucose-6-phosphate-1-dehydrogenase (Zwf) activity is enhanced, and a strain in which GntR activity, glucose-6-phosphate isomerase (Pgi) activity, and phosphogluconate dehydrogenase (Gnd) activity of a pIa/B:: atoDAB strain are inactivated and glucose-6-phosphate-1-dehydrogenase (Zwf) activity is enhanced.

Fermentation is performed in a bioreactor using the above-described microorganisms to obtain a fermentation liquid containing propanol. Thereafter, the fermentation liquid containing propanol is subjected to the separation step and the purification step described in the generation of ethanol by the above-described fermentation method similarly.

In addition to the above, propanol obtained by hydrogenating acetone obtained by microbial fermentation described in JP-A-2021-185862 can also be used as preferable propanol.

Next, generation of butanol in the fermentation method will be described. A microorganism is not particularly limited as long as the microorganism generates butanol by fermentation. Some anaerobic bacteria are known to be able to perform fermentation from carbon monoxide to an alcohol including n-butanol and butanol and acetic acid. Examples of such bacteria include bacteria belonging to the genus Clostridium including Clostridium lugdahlii, Clostridium carboxidivorans (Liou et al., International Journal of Systematic and Evolutionary Microbiology 33: pp 2085-2091), Clostridium lagsdaleae (WO-A-2008/028055), and Clostridium auto-ethanogenam (Abrini et al, Archives of Microbiology 161: pp 345-351), bacteria belonging to the genus Morella including Morela species HUC22-1(Sakai et al, Biotechnology Letters 29: pp 1607-1612), bacteria belonging to the genus Carboxydatherm (Svetlichny, V.A., Sokolova, T.G. et al (1991), Systematic and Applied Microbiology 14:254-260), Moorella thermoacetica, Morrella thermoautotrophica, Ruminococcus productus, Acetobacterium Woodii, Eubacterium limosum, Butyribacterium methylotrophicum, Oxobacter pfennigii, Methanosarcina barkeri, Methanosarcina acetiborans, and Desulfotomaculum kuznetsovii) (Simpa et. al. Critical Reviews in Biotechnology, 2006 Vol. 26. Pp 41-65).

In addition, Clostridium gender bacteria such as Clostridium acetobutylicum species directly generate 1-butanol by fermenting saccharides (including starch and cellulose). Use of these bacteria in the generation of 1-butanol based on saccharide fermentation is known as a Weizmann method. Other well-known 1-butanol generation pathways are disclosed in US-B2-4539293 and US-B2-5753474. In methods disclosed in these patent documents, a saccharide is first used in generation of butyric acid, which is later converted into 1-butanol.

In addition, a method using bacterial fermentation for generating acetone, n-butanol, and ethanol from starch, called acetone-butanol-ethanol (ABE) fermentation, is also known. Industrial development of ABE fermentation began in 1916 when Chaim Weizmann described isolation of Clostridium acetobutylicum in US-B2-1315585. This method produces a mixture containing acetone, n-butanol, and ethanol at a ratio of about 3 : 6 : 1. Raw materials and strains of microorganisms used directly influence components of the mixture taken out of a fermenter. For example, Cobalt Technologies Inc. describes in US patent application US-A1-2010330633 that a yield of butanol with fermentation raw materials and Clostridium saccharubutylium reaches 80%. In addition, Lanzatech New Zealand Co., Ltd. describes in US-B2-8119844 that the yield reaches 63% with glycerol and Clostridium pasteurianum. In order to optimize ABE fermentation, various methods have been developed in order to recover a product from a reaction liquid. This includes distillation, pervaporation separation, membrane separation, adsorption, and reverse osmosis.

Fermentation is performed in a bioreactor using the above-described microorganisms to obtain a fermentation liquid containing butanol. Thereafter, the fermentation liquid containing butanol is subjected to the separation step and the purification step described in the generation of ethanol by the above-described fermentation method similarly.

Step (31) is a step of subjecting an alcohol to a dehydration reaction to obtain a C2-C8 unsaturated hydrocarbon. In one aspect, ethanol is subjected to a dehydration reaction to obtain ethylene. In another aspect, propanol is subjected to a dehydration reaction to obtain propylene. In still another aspect, butanol is subjected to a dehydration reaction to obtain butene. In further still another aspect, two or more of ethanol, propanol, and butanol are subjected to a dehydration reaction to obtain ethylene, propylene, and butene.

Hereinafter, as step (31), a method for obtaining ethylene by subjecting ethanol to a dehydration reaction, which is one aspect, and a method for obtaining propylene by subjecting propanol to a dehydration reaction, which is another aspect, will be described as examples.

The step of obtaining ethylene by subjecting ethanol to a dehydration reaction, which is one aspect of step (31), will be described. Ethanol is converted into ethylene in an ethylene generation step, thereby obtaining an ethylene-containing product. Specifically, it is only required to bring ethanol into contact with a catalyst to convert ethanol into ethylene. Ethanol is converted into ethylene by a dehydration reaction.

When an ethanol dehydration reaction is performed, the dehydration reaction can be performed in a diluted state by adding a solvent or a gas inert to a catalyst and the alcohol to a reaction system. The ethanol dehydration reaction may be any one of a batch method, a semi-batch method, and a continuous flow method. In addition, any form of a liquid phase, a gas phase, and a gas-liquid mixed phase may be used. As a method for filling the catalyst, various methods such as a fixed bed, a fluidized bed, a suspended bed, and a shelf tier fixed bed are adopted, and any method may be adopted.

The catalyst to be used is not limited as long as the catalyst can convert ethanol into ethylene. Examples of the catalyst include acid catalysts such as zeolite, a modified zeolite such as a P-modified zeolite, silica-alumina, alumina, silicated, titanated, zirconated, or fluorinated alumina, and silicoaluminophosphate (Hereinafter, these may be collectively referred to as "zeolite or alumina-based catalyst".). Examples of the catalyst also include a heteropoly acid-supported catalyst.

As the zeolite, a zeolite containing at least one 10-membered ring in a structure thereof is advantageous, and the zeolite contains a microporous material made of silicon, aluminum, oxygen, and boron as an optional component. Examples thereof include MFI (ZSM-5, silicalite-1, boralite C, or TS-1), MEL (ZSM-11, silicalite-2, boralite D, TS-2, or SSZ-46), FER (ferriolite, FU-9, or ZSM-35), MTT (ZSM-23), MWW (MCM-22, PSH-3, ITQ-1, or MCM-49), TON (ZSM-22, Theta-1, or NU-10), EUO (ZSM-50 or EU-1), MFS (ZSM-57), and ZSM-48.

As the zeolite, a zeolite having a Si/Al ratio of 10 or more is preferable. The zeolite having a Si/Al ratio of 10 or more preferably has a Si/Al ratio of 100 or more. In addition, the zeolite preferably contains at least one selected from MFI and MEL.

As the zeolite, a dealuminized zeolite is also preferable. In the dealuminized zeolite, it is advantageous to remove about 10 mass% of aluminum. This dealuminization is advantageously performed by a steam treatment, followed by leaching if necessary.

The zeolite and the dealuminized zeolite are advantageously basically H type. In addition, at least one selected from metal compensation ions such as Na, Mg, Ca, La, Ni, Ce, Zn, and Co can be contained as an accessory component (component of about 50% or less).

The zeolite is mixed with a binder, preferably an inorganic binder, and formed into a desired shape such as a pellet shape. As the binder, a binder having durability against a temperature and other conditions used in the dehydration process of the present invention is selected. The binder is at least one inorganic material selected from clay, silica, a metal silicate, a metal oxide (for example, $ZrO_2$), and a gel containing a mixture of silica and metal oxide.

The P-modified zeolite is a phosphorus modified zeolite. In the ethylene generation step, an aspect using the P-modified zeolite is also preferable. The phosphorus modified zeolite can be produced using, as a base, for example, a zeolite having a microporous whose initial atomic ratio Si/Al is 4 to 500, specifically, MFI, MOR, MEL, clinoptilolite, FER, MWW, TON, EUO, MFS, ZSM-48, or the like. The initial atomic ratio Si/Al is preferably 100 or less, and more preferably 4 to 30. The P-modified zeolite of the present production method can also be obtained using an inexpensive zeolite having a low Si/Al ratio (30 or less) as a base.

In addition, the P-modified zeolite can be further modified with at least one metal selected from Mg, Ca, La, Ni, Ce, Zn, Co, Ag, Fe, and Cu.

The phosphorus atom content in the P-modified zeolite is at least 0.05 mass%, and preferably 0.3 to 7 mass%. In addition, at least 10 mass% of aluminum is advantageously extracted and removed from zeolite as a raw material by leaching with respect to the zeolite.

As the catalyst using the P modified zeolite, the P-modified zeolite itself may be used as the catalyst, or a blended P-modified zeolite obtained by combining the P-modified zeolite with another material may be used. By adopting the blended P-modified zeolite, hardness, catalytic activity, and the like of the catalyst can be improved.

Examples of the material that can be mixed with the P-modified zeolite include various inert or catalytically active materials and various binder materials. Specific examples thereof include compositions such as kaolin and other clays, various forms of rare earth metals, phosphates, alumina or alumina sols, titania, zirconia, quartz, silica or silica sols, and a mixture thereof. These components are effective in increasing compressive strength of the catalyst and the blended catalyst. The catalyst can be formed into pellets or spheres, extruded into other shapes, or formed into spray dried particles. The amount of the P-modified zeolite contained in a final catalyst product is 10 to 90 mass% of the total catalyst, and preferably 20 to 70 mass% of the total catalyst.

A preferable example of the P-modified zeolite is a silicoaluminophosphate, a more preferable example thereof is a silicoaluminophosphate in an AEL group, and a representative example thereof is SAPO-11. SAPO-11 uses ALPO-11 as a base, and basically has an Al/P ratio of 1 atom/atom. By adding a silicon precursor during synthesis and inserting silicon into an ALPO skeleton, an acid site is formed on a surface of a micropore of zeolite having a 10-membered ring. The content of silicon is 0.1 to 10 atom% (Al + P + Si is 100).

An aspect in which alumina (particularly γ-alumina) is used as the catalyst in the ethylene generation step is also preferable. It is also preferable to use silicated, zirconated, titanated, or fluorinated alumina. The alumina is generally characterized by having a wide range of acid strength distribution and Lewis type and Bronsted type acid sites. As the alumina, activated alumina is preferably used.

The alumina also preferably improves selectivity of the catalyst by precipitating silicon, zirconium, titanium, fluorite, and the like on a surface thereof. That is, the selectivity of the catalyst may be improved by silicating, zirconating, and titanating the alumina. For production of such a catalyst, suitable commercially available alumina is used, and eta or gamma-alumina having a surface area of 10 to 500 $m^2$/g and an alkali content of 0.5% or less is preferably used. In addition, silicon, zirconium, titanium, and the like are preferably added in a total amount of 0.05 to 10 mass% to prepare the catalyst. These metals may be added at the time of producing alumina, or may be added to a produced alumina, and these metals may be added in a form of a precursor. Fluorinated alumina itself is known, and can be produced according to a conventional technique.

In the ethylene generation step, an aspect in which a heteropoly acid-supported catalyst is used as the catalyst is also preferable. The heteropoly acid-supported catalyst contains a heteropoly acid supported on a suitable catalyst carrier. Note that the term "heteropoly acid" refers to a heteropoly acid compound in a form of a free acid or in a form of a heteropoly acid salt such as an alkali metal salt, an alkaline earth metal salt, an ammonium salt, a salt of a bulky cation, and/or a metal salt (In these cases, the salt may be either a complete salt or a partial salt.).

An anion of a heteropoly acid typically contains a polyvalent metal atom in which 12 to 18 oxygen atoms are bonded, known as a peripheral atom surrounding one or more center atoms in a symmetrical manner. The peripheral atom is suitably selected from molybdenum, tungsten, vanadium, niobium, tantalum, and a combination thereof. The center atom is preferably silicon or phosphorus. In addition, the center atom can include any one selected from atoms of Group I to VIII in the periodic table of elements, for example, copper, beryllium, zinc, cobalt, nickel, boron, aluminum, gallium, iron, cerium, arsenic, antimony, bismuth, chromium, rhodium, silicon, germanium, tin, titanium, zirconium, vanadium, sulfur, tellurium, manganese nickel, platinum, thorium, hafnium, tellurium, and iodine. Examples of a suitable heteropoly acid include Keggin, Wells-Dawson, and Anderson-Evans-Perloff heteropoly acids.

A heteropoly acid component of the heteropoly acid-supported catalyst is preferably heteropolytungstic acid, which is a heteropoly acid whose peripheral atom is a tungsten atom. A preferable heteropolytungstic acid is any heteropolytungstic acid mainly containing a Keggin or Wells-Dawson structure.

Examples of the heteropolytungstic acid include 18-phosphotungstic acid ($H_6[P_2W_{18}O_{62}]\cdot xH_2O$), 12-phospho-tungstic acid ($H_3[PW_{12}O_{40}]\cdot xH_2O$), 12-silicotungstic acid ($H_4[SiW_{12}O_{40}]\cdot xH_2O$), cesium hydrogen silicotungstic acid ($Cs_3H[SiW_{12}O_{40}]\cdot xH_2O$), monopotassium phosphotungstic acid ($KH5[P_2W_{18}O_{62}]\cdot xH_2O$), monosodium 12-silicotungstic acid ($NaK_3[SiW_{12}O_{40}]\cdot xH_2O$), and potassium phosphotungstic acid ($K_6[P_2W_{18}O_{62}]\cdot xH_2O$). A mixture of two or more different heteropolytungstic acids and salts can also be used.

The heteropoly acid component of the heteropoly acid-supported catalyst is more preferably selected from silicotungstic acid, phosphotungstic acid, and a mixture thereof, for example, from 12-silicotungstic acid ($H_4[SiW_{12}O_{40}]\cdot xH_2O$), 12-phosphotungstic acid ($H_3[PW_{12}O_{40}]\cdot xH_2O$), and a mixture thereof. The heteropoly acid is still more preferably silicotungstic acid, and most preferably 12-silicotungstic acid.

The heteropoly acid has a molecular weight of preferably more than 700 and less than 8500, more preferably more than 2800 and less than 6000. Such a heteropoly acid also includes a dimerization complex thereof.

The catalyst carrier used in the heteropoly acid-supported catalyst may be any known suitable catalyst carrier. Suitable raw materials for the catalyst support include mordenite (for example, montmorillonite), clay, bentonite, diatomaceous earth, titania, activated carbon, alumina, silica, silica-alumina, silica-titania cogel, silica-zirconia cogel, carbon-coated alumina, zeolite, zinc oxide, and thermal decomposition oxide. A catalyst carrier mainly containing silica, such as a silica gel carrier or a carrier produced by flame hydrolysis of $SiCl_4$, is preferable.

The shape of the catalyst carrier is not particularly limited, and may be, for example, a powder form, a granular form, a pelletized form, a spherical form, or an extruded form.

Ethanol is not particularly limited, but is preferably converted into ethylene by being brought into contact with the catalyst in a gas phase. In addition, ethanol may be further mixed with water, and an optional component may be appropriately mixed in addition to ethanol and water. One or both of water and the optional component is preferably brought into contact with the catalyst as a gas together with the raw material ethanol.

For example, the catalyst is filled into a reaction container, ethanol or ethanol and at least one selected from water and other optional components are supplied as a gas to the reaction container filled with the catalyst, and a gas phase dehydration reaction is performed, whereby an ethylene-containing product is preferably discharged from the reaction container in a gas phase. When ethanol remains in a gas discharged from the reaction container, a component containing ethanol may be separated from an ethylene-containing product, and the component containing ethanol may be supplied to the reaction container again.

In a case of a zeolite or alumina-based catalyst, a temperature of the reaction container is, for example, 280 to 600°C, preferably 300 to 550°C, and more preferably 330 to 530°C. A pressure (absolute pressure) of the reaction container is, for example, 0.05 to 3 MPa, preferably 0.05 to 2.5 MPa, and more preferably 0.12 to 2 MPa.

In a case of a heteropoly acid-supported catalyst, a temperature of the reaction container is, for example, 170°C or higher, preferably in a range of 180 to 270°C, more preferably in a range of 190 to 260°C, and still more preferably in a range of 200 to 250°C. The pressure is preferably in a range of 0.1 to 4.5 MPa, more preferably 1.0 to 3.5 MPa, and still more preferably 1.0 to 2.8 MPa.

Note that, in the case of the heteropoly acid-supported catalyst, bonded water may be removed from a heteropoly acid component of the heteropoly acid-supported catalyst by heating the heteropoly acid-supported catalyst to a temperature of 220°C or higher before the heteropoly acid-supported catalyst is brought into contact with ethanol and keeping the temperature for a sufficient time.

In step (31), step (31') of purifying ethanol may be performed before the ethylene generation step, and step (31'') of purifying an ethylene-containing product may be performed after the ethylene generation step.

Note that, when step (31'') is performed, the ethylene-containing product purified by step (31'') is referred to as "ethylene" produced by the production method according to the sixth embodiment, and when step (31'') is omitted, the ethylene-containing product obtained in the ethylene generation step is referred to as" ethylene" produced in the sixth embodiment. The "ethylene" produced in the sixth embodiment may be ethylene alone, or may be a composition containing impurities inevitably mixed through synthesis or purification.

In step (31'), it is preferable to remove at least one organic compound selected from a C3 to C14 aliphatic unsaturated hydrocarbon, a C3 to C14 aliphatic saturated hydrocarbon, a C3 to C10 alcohol, and a C3 to C10 ether from ethanol.

When the raw material is derived from waste, the waste contains various components. Therefore, raw material ethanol generated from waste contains various organic compounds. Among the organic compounds, an organic compound having the above carbon atom number often remains in ethanol obtained through various steps. When such an organic compound remains in ethanol, the dehydration reaction in the ethylene generation step and the polymerization reaction using ethylene in a subsequent stage may be inhibited, or quality of a polymer obtained from ethylene may be deteriorated. Therefore, by removing a hydrocarbon, an alcohol, or an ether having a specific carbon atom number in step (31'), the polymerization reaction of ethylene preferably proceeds, and quality of an obtained polymer is easily improved.

On the other hand, in step (31''), it is preferable to remove at least one selected from a specific organic compound including a C3 to C14 aliphatic unsaturated hydrocarbon, a C3 to C14 aliphatic saturated hydrocarbon, a C3 to C10 alcohol, and a C3 to C10 ether, carbon monoxide, carbon dioxide, and oxygen from the generated ethylene-containing product.

Note that the term "removal" used in steps (31') and (31") includes not only an aspect in which a target substance is completely removed from ethanol or the ethylene-containing product, but also an aspect in which the content of the target substance is reduced.

Also in step (31''), as described in the above description of step (31'), by removing a hydrocarbon, an alcohol, or an ether having a specific carbon atom number, the polymerization reaction of ethylene preferably proceeds, and quality of an obtained polymer is easily improved.

Carbon monoxide, carbon dioxide, and oxygen may inhibit a polymerization reaction in the polymerization reaction of ethylene.

In ethylene produced by the production method according to the sixth embodiment, the content of carbon monoxide is preferably 100 ppm by volume or less by removal of carbon monoxide. The content of carbon monoxide is more preferably 10 ppm by volume or less, and still more preferably 1 ppm by volume or less. In addition, ethylene produced in the present invention may contain no carbon monoxide at all, and therefore a lower limit of the content of carbon monoxide is 0 vol%.

In ethylene produced by the production method according to the sixth embodiment, the content of carbon dioxide is preferably 1 vol% or less by removal of carbon dioxide. The content of carbon dioxide is more preferably 0.5 vol% or less, and still more preferably 0.1 vol% or less. In addition, ethylene produced in the present invention may contain no carbon dioxide at all, and therefore a lower limit of the content of carbon dioxide is 0 vol%.

In ethylene produced by the production method according to the sixth embodiment, the content of oxygen is preferably 1 vol% or less by removal of oxygen. The content of oxygen is more preferably 0.5 vol% or less, and still more preferably 0.1 vol% or less. In addition, ethylene produced in the present invention may contain no oxygen at all, and therefore a lower limit of the content of oxygen is 0 vol%.

The C3 to C14 aliphatic unsaturated hydrocarbon is preferably removed in step (31'), step (31"), or both of step (31') and step (31''). When the C3 to C14 aliphatic unsaturated hydrocarbon is contained in ethylene, when a polymerization reaction is performed using the ethylene, the C3 to C14 aliphatic unsaturated hydrocarbon serves as a branched chain of a polymer, and therefore the number of branched chains can be reduced by removing the C3 to C14 aliphatic unsaturated hydrocarbon. Therefore, as described later, it is particularly preferable to remove the C3 to C14 aliphatic unsaturated hydrocarbon when it is desired to reduce the number of branched chains of a polymer, for example, when high density polyethylene (HDPE) is produced from ethylene.

In ethylene produced by the production method according to the sixth embodiment, by removing the C3 to C14 aliphatic unsaturated hydrocarbon, the content of the C3 to C14 aliphatic unsaturated hydrocarbon is preferably 1 vol% or less. The content of the C3 to C14 aliphatic unsaturated hydrocarbon is more preferably 0.5 vol% or less, and still more preferably 0.1 vol% or less. In addition, ethylene produced in the present invention may contain no C3 to C14 aliphatic unsaturated hydrocarbon at all, and therefore a lower limit of the content of the C3 to C14 aliphatic unsaturated hydrocarbon is 0 vol%.

In addition, the C3 to C14 aliphatic saturated hydrocarbon is preferably removed in step (31'), step (31''), or both of step (31') and step (31''), and the C3 to C14 aliphatic saturated hydrocarbon removed in any step preferably contains a C6 to C14 aliphatic saturated hydrocarbon. The C6 to C14 aliphatic saturated hydrocarbon may be linear or may have at least one of a branched structure and a cyclic structure. As a specific example of the C6 to C14 aliphatic saturated hydrocarbon, at least one selected from n-hexane, n-heptane, n-octane, n-decane, n-dodecane, and n-tetradecane is preferable.

These aliphatic saturated hydrocarbons having a relatively large carbon atom number (carbon atom number: 6 to 14) may be contained in a relatively large amount in the raw material ethanol derived from waste.

In ethylene produced by the production method according to the sixth embodiment, by removing the C6 to C14 aliphatic saturated hydrocarbon, the content of the C6 to C14 aliphatic saturated hydrocarbon is preferably 0.3 vol% or less, more preferably 0.2 vol% or less, and still more preferably 0.1 vol% or less In addition, ethylene produced in the present invention may contain no C6 to C14 aliphatic saturated hydrocarbon at all, and therefore a lower limit of the content of the C6 to C14 aliphatic saturated hydrocarbon is 0 vol%.

In addition, the C3 to C10 alcohol is preferably removed in step (31'), step (31''), or both of step (31') and step (31''), and the C3 to C10 alcohol removed in any step includes, for example, at least one selected from 1-propanol, 2-propanol, 1-butanol, 2-butanol, and tert-butanol. These alcohols may be contained in a relatively large amount in the raw material ethanol derived from waste.

In ethylene produced by the production method according to the sixth embodiment, by removing the C3 to C10 alcohol, the content of the C3 to C10 alcohol is preferably 0.3 vol% or less. The content of the C3 to C10 alcohol is more preferably 0.1 vol% or less, and still more preferably 0.05 vol% or less. In addition, ethylene produced in the present invention may contain no C3 to C10 alcohol at all, and therefore a lower limit of the content of the C3 to C10 alcohol is 0 vol%.

The C3 to C10 ether is preferably removed in step (31'), step (31''), or both of step (31') and step (31''), and the C3 to C10 ether removed in any step includes, for example, at least one selected from diethyl ether and dibutyl ether. These ethers are contained in a relatively large amount in the raw material ethanol derived from waste, and may be removed in step (31'), step (31''), or both of step (31') and step (31'').

In ethylene produced by the production method according to the sixth embodiment, by removing the C3 to C10 ether, the content of the C3 to C10 ether is preferably 0.3 vol% or less. The content of the C3 to C10 ether is more preferably 0.1 vol% or less, and still more preferably 0.05 vol% or less. In addition, ethylene produced in the present invention may contain no C3 to C10 ether at all, and therefore a lower limit of the content of the C3 to C10 ether is 0 vol%.

In the ethylene generation step, water is generated by a dehydration reaction, and therefore at least water is preferably removed in step (31"). In addition, unreacted ethanol generally remains in the ethylene-containing product generated in the ethylene generation step, and therefore the unreacted ethanol is also preferably removed. By removing water and ethanol, the polymerization reaction using ethylene described later preferably proceeds, and quality of an obtained polymer is easily improved.

In ethylene produced by the production method according to the sixth embodiment, the content of water is preferably 0.3 vol% or less by removal of water in step (31''). By setting the content of water to 0.3 vol% or less, the polymerization reaction using ethylene preferably proceeds, and quality of an obtained polymer is easily improved. The content of water is more preferably 0.1 vol% or less, and still more preferably 0.05 vol% or less from such a viewpoint. In addition, ethylene produced in the present invention may contain no water at all, and therefore a lower limit of the content of water is 0 vol%.

In ethylene produced by the production method according to the sixth embodiment, the content of ethanol is preferably 0.3 vol% or less by removal of ethanol in step (31''). By setting the content of ethanol to 0.3 vol% or less, the polymerization reaction using ethylene preferably proceeds, and quality of an obtained polymer is easily improved. The content of ethanol is more preferably 0.1 vol% or less, and still more preferably 0.05 vol% or less from such a viewpoint. In addition, ethylene produced in the present invention may contain no ethanol at all, and therefore a lower limit of the content of ethanol is 0 vol%.

Note that ethylene produced by the production method according to the sixth embodiment is generally a gas, and by analyzing each inorganic gas (oxygen, carbon monoxide, carbon dioxide, or the like) and an organic substance gas in the ethylene which is a gas using GC-TCD and GC-FID, the vol% of each component can be measured.

Examples of the purification method in each of step (31') and step (31'') include a water separation device such as a gas chiller, a separation device using an adsorbent such as activated carbon, a pressure swing adsorption type separation device (PSA), a temperature swing adsorption type separation device (TSA), a pressure temperature swing adsorption type separation device (PTSA), a low-temperature separation type (cryogenic type) separation device, a water-soluble impurity separation device such as a scrubber, a desulfurization device (sulfide separation device), a membrane separation type separation device, a distillation device, a separation device having chromatography, and a solution absorption device.

As the low-temperature separation type, various forms can be used, and examples thereof include a type using a condenser. The solution absorption device is a device including an absorbing solution that selectively absorbs a predetermined gas component by bringing a gas into contact with the absorbing solution, and an alkaline solution such as an amine solution is preferably used as the absorbing solution. For example, when an alkaline solution is used in step (31'), carbon dioxide and the like in the gasified raw material ethanol can be absorbed.

In step (31'), as described above, any one of the C3 to C14 aliphatic unsaturated hydrocarbon, the C3 to C14 aliphatic saturated hydrocarbon, the C3 to C10 alcohol, and the C3 to C10 ether is preferably removed, but among these compounds, a compound having a relatively large carbon atom number is more preferably removed, and a C6 to C14 aliphatic unsaturated hydrocarbon is particularly preferably removed. The organic compound having a large carbon atom number is easily and conveniently removed in step (31 ') due to a difference in molecular weight from ethanol, and by removing the organic compound, the reaction in the ethylene generation step more suitably proceeds.

In step (31'), a method for removing the C6 to C14 aliphatic unsaturated hydrocarbon is not particularly limited, but the C6 to C14 aliphatic unsaturated hydrocarbon is preferably removed by a separation device having chromatography. As the chromatography, reverse phase chromatography or the like is preferably used. Furthermore, the C6 to C14 aliphatic unsaturated hydrocarbon may be removed by a distillation device, activated carbon adsorption, or the like.

In step (31"), either water generated in the ethylene generation step or unreacted ethanol is preferably removed, and both of these are preferably removed. In addition, in step (31''), as described above, at least one selected from a specific organic compound, carbon monoxide, carbon dioxide, and oxygen is preferably removed, and as the specific organic compound, a low molecular weight organic compound (for example, carbon atom number: 3 to 5) having a relatively low molecular weight is more preferably removed. That is, more preferably, in step (31'), a C6 to C14 aliphatic saturated hydrocarbon is removed, and in step (31''), in addition to water and unreacted ethanol, at least a specific low molecular weight organic compound having a relatively low molecular weight is removed, and in step (31''), in addition to these compounds, carbon monoxide, carbon dioxide, and oxygen are further removed preferably.

Specifically, the specific organic compound removed in step (31'') more preferably contains at least one selected from a C3 to C10 alcohol such as 2-propanol and a C3 to C10 ether such as diethyl ether.

The low molecular weight organic compound can be efficiently removed from ethylene together with unreacted ethanol, carbon monoxide, carbon dioxide, oxygen, and the like by using a specific separation device. Diethyl ether can be a raw material for generating ethylene together with ethanol in the ethylene generation step. Therefore, it is preferable to remove diethyl ether at a larger ratio in step (31'') than in step (31'). Note that the ratio is a ratio to ethanol in step (31') and a ratio to an ethylene-containing product in step (31").

In step (31''), although not particularly limited, purification is preferably performed by a low-temperature separation type separation device. Specifically, ethylene is solidified by a condenser using a chiller cooled to a temperature equal to or lower than a melting point of ethylene (-170 °C or lower at normal pressure), and ethylene is separated.

In this case, substances such as water, carbon dioxide, ethanol, and other organic compounds having a melting point higher than that of ethylene are preferably removed using one or more preceding-stage condensers in which a temperature of a chiller is higher than that of the above condenser. As the preceding-stage condenser, for example, a first condenser in which a temperature of a chiller is relatively high (for example, 0 to 25°C at normal pressure) and a second condenser in which a temperature of a chiller is lower than that of the first condenser (for example, -50 to -90°C at normal pressure) may be used in combination. In addition, the condenser may have any form, and a gas phase ethylene-containing product may be brought into contact with a metal pipe or the like through which a chiller passes, or the chiller and the ethylene-containing product may be brought into direct contact with each other.

A step of obtaining propylene by subjecting propanol to a dehydration reaction, which is another aspect of step (31), will be described. Propanol is converted into propylene in a propylene generation step, thereby obtaining a propylene-containing product. Specifically, it is only required to bring propanol into contact with a catalyst to convert propanol into propylene. Propanol is converted into propylene by a dehydration reaction.

When a propanol dehydration reaction is performed, the dehydration reaction can be performed in a diluted state by adding a solvent or a gas inert to a catalyst and propanol to a reaction system. The propanol dehydration reaction method may be any one of a batch method, a semi-batch method, and a continuous flow method. In addition, any form of a liquid phase, a gas phase, and a gas-liquid mixed phase may be used. As a method for filling the catalyst, various methods such as a fixed bed, a fluidized bed, a suspended bed, and a shelf tier fixed bed are adopted, and any method may be adopted.

The catalyst to be used is not limited as long as the catalyst can convert propanol into propylene, and examples thereof include acid catalysts such as zeolite, silica alumina, and alumina. Only the acid catalyst may be used, or the acid catalyst may be used in combination with a catalyst, a molding agent (binder), or the like that can be usually used in a dehydration reaction. When the acid catalyst is used together with the molding agent, the catalyst and the molding agent may be used in a form of a compact in which mechanical strength is enhanced by kneading and extrusion-molding the catalyst and the molding agent. The shape of the compact may be a granular shape, a cylindrical shape, a ring shaped, or the like, and is not particularly limited. Examples of a method for molding the catalyst include tablet molding, compression molding, and extrusion molding, but are not particularly limited. Usually, examples of the catalyst or the molding agent that can be used in the dehydration reaction include silica, alumina, clay, titania, zirconia, zinc oxide, ceria, lanthanum, graphite, and ethyl cellulose.

Regarding a combination of the catalysts to be filled, only the acid catalyst may be filled for use as a dehydration reaction catalyst, or a catalyst that can be usually used in a dehydration reaction may be partially contained. For example, since a water content increases near a reactor outlet as the reaction proceeds, it is also useful to use the catalyst in the latter half of the reactor. When catalytic activity decreases at a certain elapsed time, activity of the dehydration catalyst can be recovered by performing regeneration by a known method.

In order to maintain the production amount of propylene, a merry-go-round method may be adopted in which two or three reactors are arranged in parallel, and while one reactor is regenerated, a reaction is performed in the remaining one or two reactors. Furthermore, when there are three reactors, two other reactors may be connected in series to reduce a fluctuation in the production amount. In addition, when the reaction is performed using a fluidized bed flow reaction type or a movable bed reaction type, fixed activity can be maintained by continuously or intermittently extracting a part or a whole of the catalyst from a reactor and supplying a corresponding amount of the catalyst.

A temperature of the catalyst layer is preferably 450°C or lower, more preferably 400°C or lower, still more preferably 300°C or lower, and particularly preferably 200°C or lower. A lower limit of the temperature is not particularly limited as long as the temperature is equal to or higher than the temperature at which the dehydration reaction is performed, but it is preferably 160°C or higher and may be 180°C or higher from a viewpoint of enhancing obtained catalytic activity. The temperature of the catalyst layer can be appropriately adjusted by, for example, changing the temperature of a device used for the dehydration reaction.

When the temperature of the catalyst layer is in the above range, the dehydration reaction can be performed at a relatively low temperature, and therefore energy required for the reaction can be reduced.

A reaction pressure during the dehydration reaction is not particularly limited, but is preferably 0 to 100 MPa (gauge pressure), more preferably 0 to 5 MPa (gauge pressure), and still more preferably 0 to 4 MPa (gauge pressure).

In addition, although the dehydration reaction can be performed without pressurization, propylene obtained by the dehydration reaction is a gas at normal temperature and normal pressure, and therefore needs to be liquefied in order to perform purification such as distillation. Therefore, when propylene is a gas, it is necessary to liquefy the gas by cooling, pressurization, or the like, and production of propylene is complicated.

A ratio (W/F) of a catalyst weight g to a propanol flow rate $mol \cdot h^{-1}$ in the dehydration reaction step is not particularly limited, but is preferably 0.01 to 10,000 $g \cdot h \cdot mol^{-1}$, more preferably 0.1 to 5000 $g \cdot h \cdot mol^{-1}$, and still more preferably 1 to 200 $g \cdot h \cdot mol^{-1}$.

In order to quickly discharge propylene generated by the dehydration reaction from the reaction system, a gaseous substance inert to the dehydration reaction may be mixed. Examples of the gaseous substance include nitrogen, helium, argon, methane, ethane, propane, and butane. In addition, even when the gaseous substance is in a liquid state before the dehydration reaction of propanol, the gaseous substance only needs to be in a gas state during the dehydration reaction. Examples of such a gaseous substance include aliphatic hydrocarbons such as pentane, hexane, heptane, and cyclopentane, and aromatic hydrocarbons such as benzene, toluene, xylene, ethylbenzene, and cumene. When a gaseous substance is used, the gaseous substance is preferably in a range of 0.05 to 10 mol with respect to 1 mol of propylene.

In addition, when the catalytic activity decreases, regeneration may be performed by a known method to recover the activity of the dehydration catalyst. In order to maintain the production amount of propylene, a switching system may be adopted in which two or more reactors are arranged in parallel, and while one reactor is recovering the activity of the catalyst, a dehydration reaction is performed by another reactor. Furthermore, when there are three or more reactors, two or more reactors that are not recovering the activity of the catalyst may be connected in series to reduce a fluctuation in the production amount. In addition, when the type of the reactor is a fluidized bed flow reaction type or a movable bed reaction type, fixed catalytic activity can be maintained by continuously or intermittently extracting a part or a whole of the catalyst from a reactor and supplying the catalyst in an amount equivalent to the extracted amount. After the dehydration reaction step, for example, a step of taking out propylene as a product in a gas-liquid separation step and then purifying the propylene by distillation or the like may be performed.

Purification facilities may be disposed in a process of the production method according to the sixth embodiment, or may be disposed in common in a complex obtained by combining different techniques. When a naphtha cracker and an ethane cracker are adjacent to each other, the reaction product may be introduced into a distillation column group of the naphtha cracker and the ethane cracker for separation and purification.

[Seventh embodiment of method for producing C2-C8 unsaturated hydrocarbon]

A method for producing a C2-C8 unsaturated hydrocarbon according to a seventh embodiment is a combination of the method for producing a C2-C8 unsaturated hydrocarbon according to the fifth embodiment, and a method for producing a C2-C8 unsaturated hydrocarbon including step (31) of subjecting at least one alcohol selected from the group consisting of ethanol, propanol, and butanol to a dehydration reaction to obtain a C2-C8 unsaturated hydrocarbon.

Step (31) in the method for producing a C2-C8 unsaturated hydrocarbon mixture according to the seventh embodiment is similar to step (31) in the sixth embodiment described above.

[Method for producing C2-C8 unsaturated hydrocarbon mixture]

A method for producing a C2-C8 unsaturated hydrocarbon mixture includes step (41) of bringing a C2-C8 unsaturated hydrocarbon obtained by the method for producing a C2-C8 unsaturated hydrocarbon according to any one of the first to seventh embodiments into contact with a C2-C8 unsaturated hydrocarbon derived from fossil resources to obtain a mixture.

Step (41) is a step of bringing a C2-C8 unsaturated hydrocarbon produced in the present invention into contact with a C2-C8 unsaturated hydrocarbon derived from fossil resources to obtain a C2-C8 unsaturated hydrocarbon mixture.

In step (41), the contact mass of the fossil resource-derived C2-C8 unsaturated hydrocarbon to be brought into contact to the contact mass of the C2-C8 unsaturated hydrocarbon produced by the method for producing a C2-C8 unsaturated hydrocarbon mixture of the present invention (contact mass of the fossil resource-derived C2-C8 unsaturated hydrocarbon/contact mass of the C2-C8 unsaturated hydrocarbon) is preferably 0.001 to 10,000, and more preferably 0.01 to 10,000.

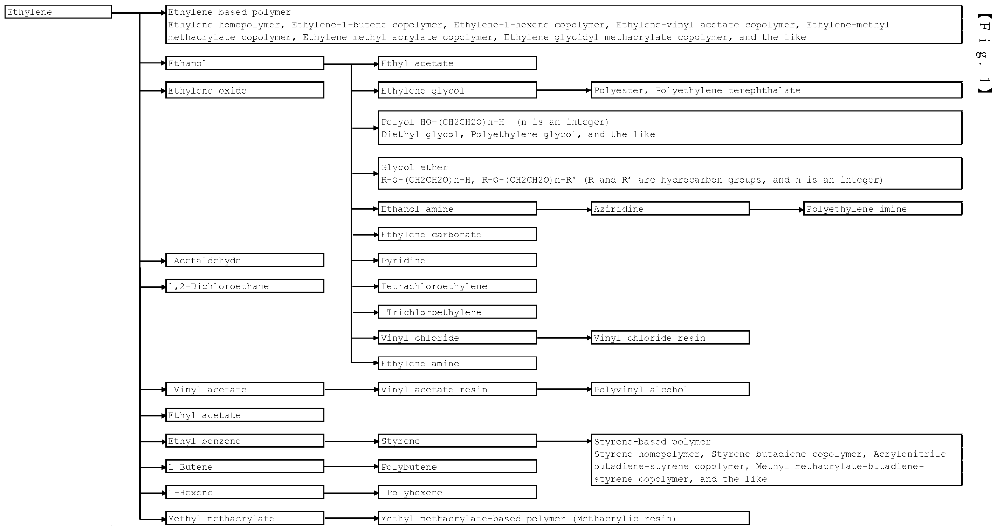

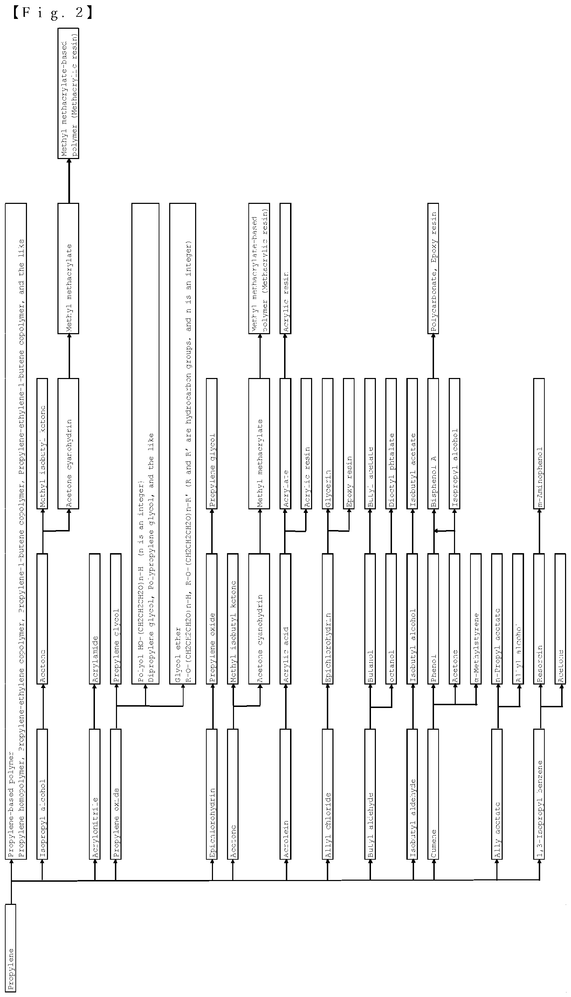

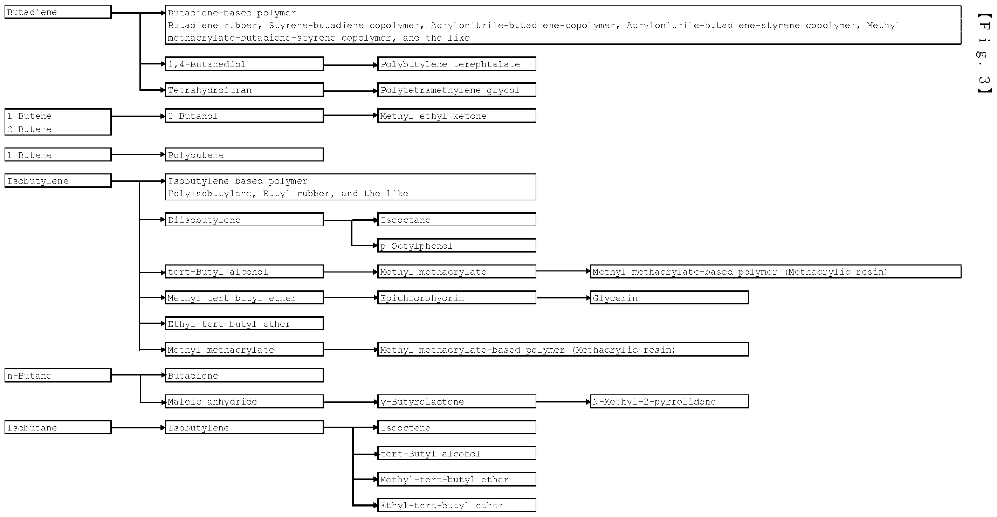

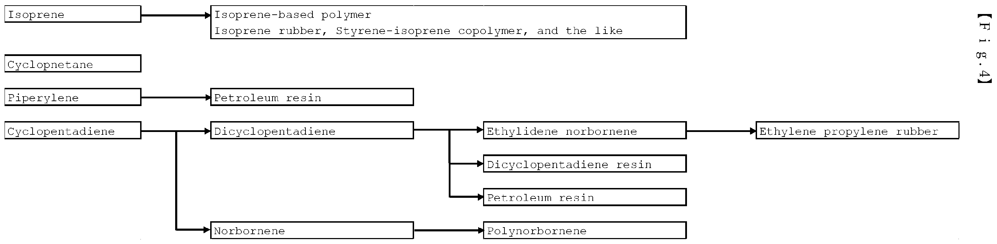

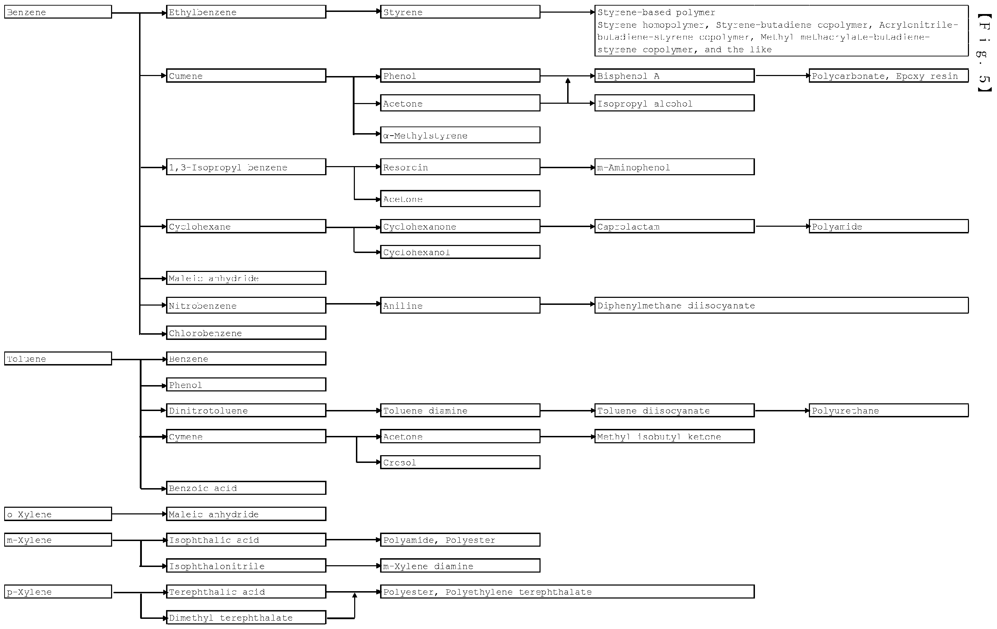

Each compound of the C2-C8 unsaturated hydrocarbon produced in the present invention can be used for various applications. In addition, various derivatives may be produced using each compound as a starting material. An example of a flow of production of each of the derivatives is illustrated in Figs. 1 to 5. As methods for producing these derivatives, known methods can be used. Note that, in each of Figs. 1 to 5, one component used in a synthesis reaction is described.

In addition, in the production of these derivatives, in addition to each compound produced in the present invention, a compound produced using fossil resources such as petroleum, coal, and natural gas as raw materials (fossil resource-derived compound) may be used, a compound produced using plants such as sugar cane and corn as raw materials (biomass-derived compound) may be used, or both a fossil resource-derived compound and a biomass-derived compound may be used.

Hereinafter, as derivatives, a polymer using an olefin as a monomer (olefin-based polymer), a polymer using butadiene as a monomer (butadiene-based polymer), a polymer using methyl methacrylate as a monomer (methyl methacrylate-based polymer), methyl methacrylate, a polymer using propylene oxide as a monomer (propylene oxide-based polymer), propylene oxide, a polymer using ethylene oxide as a monomer (ethylene oxide-based polymer), ethylene oxide, a polymer using ethylene glycol as a monomer (ethylene glycol-based polymer), ethylene glycol, phenol, acetone, and isopropyl alcohol will be described.

[Method for producing olefin-based polymer]

A method for producing an olefin-based polymer includes a step of polymerizing a monomer containing a C2-C8 unsaturated hydrocarbon obtained by the method for producing a C2-C8 unsaturated hydrocarbon according to any one of the first to seventh embodiments or a C2-C8 unsaturated hydrocarbon mixture obtained by the above-described method for producing a C2-C8 unsaturated hydrocarbon mixture.

The C2-C8 unsaturated hydrocarbon and the C2-C8 unsaturated hydrocarbon mixture produced in the present invention are subjected to a polymerization step for producing an olefin-based polymer. In the polymerization step, a monomer containing a C2-C8 unsaturated hydrocarbon is polymerized to obtain a polymer.

In the production of the olefin-based polymer, in addition to the C2-C8 unsaturated hydrocarbon and the C2-C8 unsaturated hydrocarbon mixture produced in the present invention, an olefin produced using fossil resources such as petroleum, coal, and natural gas as raw materials (fossil resource-derived olefin) may be used, an olefin produced using plants such as sugar cane and corn as raw materials (biomass-derived olefin) may be used, or both of a fossil resource-derived olefin and a biomass-derived olefin may be used. Examples of the polymer produced by the method for producing an olefin-based polymer include an ethylene-based polymer, a propylene-based polymer, and a butadiene-based polymer. Hereinafter, an ethylene-based polymer, a propylene-based polymer, and a butadiene-based polymer will be described as examples.

[Olefin-based polymer]

<Ethylene-based polymer>

By polymerizing a monomer containing ethylene produced in the present invention, an ethylene-based polymer such as high-density polyethylene, low-density polyethylene, linear low-density polyethylene, very-low-density polyethylene, or long-chain branched polyethylene can be produced. The polymer may be homopolyethylene obtained by polymerizing ethylene alone, or a copolymer obtained by polymerizing ethylene and a monomer other than ethylene.

Examples of the monomer copolymerizable with ethylene include: a C3 to C20 olefin such as propylene, 1-butene, 2-butene, 1-pentene, 1-hexene, 1-heptene, 1-octene, 1-nonene, 1-decene, 1-dodecene, 4-methyl-1-pentene, or 4-methyl-1-hexene; a cyclic olefin such as norbornene; an alkenyl aromatic hydrocarbyl such as styrene; an unsaturated carboxylic acid such as acrylic acid or methacrylic acid; an unsaturated carboxylate such as methyl acrylate, ethyl acrylate, propyl acrylate, butyl acrylate, methyl methacrylate, ethyl methacrylate, propyl methacrylate, or butyl methacrylate; a vinyl ester compound such as vinyl acetate; a polyene compound such as 5-ethylidene-2-norbornene or dicyclopentadie; vinyl fluoride, vinyl chloride, vinyl bromide, tetrafluoroethylene, diethyl maleate, and diethyl fumarate. These monomers may be used singly or in combination of two or more types thereof.

As ethylene, in addition to ethylene produced in the present invention, fossil resource-derived ethylene may be used, biomass-derived ethylene may be used, or both fossil resource-derived ethylene and biomass-derived ethylene may be used.

In addition, as a monomer other than ethylene, a monomer produced in the present invention (for example, propylene, 1-butene, 2-butene, 1-pentene, 1-hexene, or 1-heptene) may be used, a fossil resource-derived monomer (for example, propylene, 1-butene, 2-butene, 1-pentene, 1-hexene, or 1-heptene) may be used, and a biomass-derived monomer (for example, propylene, 1-butene, 2-butene, 1-pentene, 1-hexene, or 1-heptene) may be used. Two or more of these monomers, such as monomer produced in the present invention/fossil resource-derived monomer, monomer produced in the present invention/biomass-derived monomer, fossil resource-derived monomer/biomass-derived monomer, or monomer produced in the present invention/fossil resource-derived monomer/biomass-derived monomer, may be used in combination.

Specific examples of the polymer include an ethylene homopolymer, an ethylene-propylene copolymer, an ethylene-1-butene copolymer, an ethylene-1-hexene copolymer, an ethylene-1-butene-1-hexene copolymer, an ethylene-1-octene copolymer, an ethylene-4-methyl-1-pentene copolymer, an ethylene-vinyl acetate copolymer, an ethylene-methyl acrylate copolymer, an ethylene-ethyl acrylate copolymer, an ethylene-methyl methacrylate copolymer, an ethylene-ethyl methacrylate copolymer, an ethylene-acrylic acid copolymer, an ethylene-methacrylic acid copolymer, and an ethylene-propylene-5-ethylidene-2-norbornene copolymer.

The content of an ethylene unit in the ethylene-based polymer is usually 50 mass% or more, and preferably 70 mass% or more with respect to 100 mass% of the ethylene-based polymer. Note that the content of the ethylene unit is measured by a $^{13}$C-NMR method.

A density of the ethylene-based polymer depends on the content of the ethylene unit, but is usually 870 to 965 kg/m$^3$.

A melt mass flow rate (MFR) of the ethylene-based polymer is usually 0.01 to 1000 g/10 min, and preferably 0.05 to 100 g/10 min. Note that the MFR is measured at a temperature of 190°C under a load of 2.16 kg defined in JIS K7210-1-2014.

A molecular weight distribution (mass average molecular weight (Mw)/number average molecular weight (Mn)) of the ethylene-based polymer is usually 2 to 15, and preferably 3 to 10.

When the ethylene-based polymer is long-chain branched polyethylene, a branching index (gc') thereof is usually 0.3 to 0.8. Note that the gc' is measured by a method described in JP-A-2012-214780.

Examples of a method for producing an ethylene-based polymer include a method for polymerizing ethylene alone or ethylene and a monomer other than ethylene in the presence of a radical polymerization initiator (high pressure radical polymerization method).

As the radical polymerization initiator, an oxygen-based initiator such as an organic peroxide, a peroxy ester, a dialkyl peroxide, or a combination thereof can be used. Examples of the radical polymerization initiator include t-butyl peroxypivalate, di-t-butyl peroxide (DTBP), t-butyl peroxyacetate (TBPO), t-butyl peroxy-2-ethylhexanoate, t-butyl peroxineodecanoate (PND), t-butyl peroxyoctoate, and a combination of any two or more thereof. In a high pressure radical polymerization method, a polymerization pressure is usually 50 to 400 MPa, and a polymerization temperature is usually 100 to 350°C.

Examples of the method for producing an ethylene-based polymer further include a method for polymerizing ethylene alone or ethylene and a monomer other than ethylene in the presence of a polymerization catalyst such as a Ziegler-Natta catalyst, a metallocene catalyst, a Phillips catalyst, or a standard catalyst.

As the Ziegler-Natta catalyst, for example, a triethyl aluminum-titanium tetrachloride solid composite is used. The Ziegler-Natta catalyst may be, for example, a combination of a titanium trichloride composition obtained by reducing titanium tetrachloride with an organic aluminum compound and further treating the reduced product with various electron donors and electron acceptors, an organic aluminum compound, and an aromatic carboxylate, or may be a supported catalyst obtained by bringing titanium tetrachloride and various electron donors into contact with magnesium halide.

Examples of the metallocene catalyst include a compound such as bis(cyclopentadienyl) metal complex having a structure in which a transition metal is sandwiched between π electron unsaturated compounds. More specific examples thereof include a compound in which one or more cyclopentadienyl rings or analogs thereof are present as ligands in a tetravalent transition metal such as titanium, zirconium, nickel, palladium, hafnium, or platinum.

The Ziegler-Natta catalyst and the metallocene catalyst may be each used in combination with a specific co-catalyst. Specific examples of the co-catalyst include methylaluminoxane (MAO) and a boron-based compound.

The Phillips catalyst is, for example, a catalyst system containing a chromium compound such as chromium oxide, and specific examples thereof include a catalyst in which a chromium compound such as chromium trioxide or a chromate is supported on a solid oxide such as silica, alumina, silica-alumina, or silica-titania.

The standard catalyst is a known catalyst using molybdenum oxide, and examples thereof include gamma-alumina/molybdenum oxide. As a polymerization catalyst for producing long-chain branched polyethylene, polymerization catalysts described in JP-A-2004-292772, JP-A-2012-214780, JP-A-2015-189973, and JP-A-2017-20019 can be used.

Examples of a polymerization method include: a solvent polymerization method or a slurry polymerization method using an aliphatic hydrocarbyl such as butane, pentane, hexane, heptane, or octane, an aromatic hydrocarbyl such as benzene or toluene, or a halogenated hydrocarbyl such as methylene dichloride as a solvent; and a gas phase polymerization method in which polymerization is performed in a gaseous monomer. In addition, a method in which these are combined, for example, a method in which polymerization is performed by a solution polymerization method and then polymerization is performed by a gas phase polymerization method, or a method in which polymerization is performed by a slurry polymerization method and then polymerization is performed by a gas phase polymerization method may be used.

A polymerization pressure is usually normal pressure to 5 MPa. A polymerization temperature can be in a range of 0 to 220°C. The polymerization temperature is preferably 20°C or higher, more preferably 40°C or higher, and still more preferably 50°C or higher. In addition, the polymerization temperature is preferably 130°C or lower, and more preferably 100°C or lower. A polymerization time is generally appropriately determined depending on the type of a target polymer and a reaction device, but is one minute to 20 hours. In addition, a chain transfer agent such as hydrogen can be added in order to adjust the molecular weight of the ethylene-based polymer.

In addition, either a continuous polymerization method or a batch polymerization method can be used. Furthermore, a single-stage polymerization method or a multi-stage polymerization method may be used. When the polymerization is performed by the multi-stage polymerization method, a plurality of polymerization reactors may be connected in series or connected in parallel, or both polymerization reactors connected in series and polymerization reactors connected in parallel may be used.

Various known resins, rubbers, and additives may be blended in the ethylene-based polymer for use. The resin and the rubber may be an olefin-based polymer (fossil resource-derived polyolefin) such as an ethylene-based polymer (fossil resource-derived polyethylene) or a propylene-based polymer (fossil resource-derived polypropylene) produced using a fossil resource-derived olefin as a raw material, or may be an olefin-based polymer (biomass-derived polyolefin) such as an ethylene-based polymer (biomass-derived polyethylene) or a propylene-based polymer (biomass-derived polypropylene) produced using a biomass-derived olefin as a raw material. In addition, the resin and the rubber may be an olefin-based polymer (regenerated polyolefin) such as an ethylene-based polymer (regenerated polyethylene) or a propylene-based polymer (regenerated polypropylene) that has been material-recycled. Furthermore, two or more types thereof may be combined.

As the additive, one or more known additives such as an antioxidant (phenol-based, phosphor-based, or sulfur-based), an ultraviolet absorber, a light stabilizer, a lubricant, an antistatic agent, an antifogging agent, an antiblocking agent, a mold release agent, a processing aid, an inorganic pigment, an organic pigment, a content dispersant, a dye, a crosslinking agent, a foaming agent, an organic peroxide, a neutralizing agent, an adsorbent, a weather resistance stabilizer, a heat resistance stabilizer, a copper inhibitor, a crystal nucleating agent, a plasticizer, an inorganic or organic filler, a flame retardant, an antibacterial agent, and a light diffusing agent can be blended. Examples of these known additives include compounds described in "Handbook Rubber/Plastic Blending Chemicals" (published on April 27, 2001 by Rubber Digest Co., Ltd.), "Plastic Data book" (jointly edited by Asahi Kasei Amidas Co., Ltd. and "Plastics" editorial department, published by Industrial Research Committee on December 1, 1999).

Examples of the organic filler include carbon fibers, carbon black, carbon nanotubes, cellulose nanofibers, and wood flour. Examples of the inorganic filler include silica, diatomaceous earth, alumina, titanium oxide, magnesium oxide, pumice powder, pumice balloon, aluminum hydroxide, magnesium hydroxide, dolomite, calcium sulfate, potassium titanate, barium sulfate, talc, clay, mica, glass flakes, glass beads, glass fibers, aluminum silicate, calcium silicate, montmorillonite, bentonite, molybdenum sulfide, graphite, calcium carbonate, metal powder (aluminum, copper, iron, lead, and the like), and silica stone.

The ethylene-based polymer can be molded into various compacts (film, sheet, container (bottle, tray, or the like), and the like) by a known molding method such as injection molding, extrusion molding, blow molding, compression molding, vacuum molding, calender molding, or foam molding. Examples of a method for molding a film include extrusion laminate molding, T-die film molding, and inflation molding (air cooling, water cooling, multi-stage cooling, or high-speed processing).

The compact using the ethylene-based polymer may be a single-layer compact containing the ethylene-based polymer, or may be a multilayer compact having a layer other than the layer containing the ethylene-based polymer. Examples of the layer other than the layer containing the ethylene-based polymer include: a vinyl-based polymer such as polyvinyl chloride, polyvinylidene chloride, polystyrene, polyacrylate, or polyacrylonitrile; a polyamide-based polymer such as nylon 6, nylon 66, nylon 10, nylon 11, nylon 12, nylon 610, or polymethaxylylene adipamide; a polyester-based polymer such as polyethylene terephthalate, polyethylene terephthalate/isophthalate, or polybutylene terephthalate; polyvinyl alcohol; an ethylene-vinyl alcohol copolymer; a polycarbonate-based polymer; paper; and a barrier film (such as aluminum foil, a vapor deposition film, or a coating film).

The compact can be applied to various known applications. Examples of specific applications include various packaging films for food packaging, a wrap film, a standard bag, a heavy bag, a sugar bag, a liquid paper container, a laminated raw fabric, a special shape liquid packaging bag (such as standing pouch), an infusion bag, a bag-in-box, a clean film used for packaging semiconductor materials, pharmaceuticals, and the like, a protective film, a blow bottle, a squeeze bottle, a cap, a label, a tube, a pipe, a gasoline tank, an agricultural material, daily goods, and fibers.

<Propylene-based polymer>

By polymerizing a monomer containing propylene produced in the present invention, a propylene-based polymer such as a homopolypropylene, a random polypropylene, or a block polypropylene (heterophasic polypropylene) can be produced.

Examples of the monomer copolymerizable with propylene include: a C2 to C20 olefin such as ethylene, 1-butene, 2-butene, 1-pentene, 1-hexene, 1-heptene, 1-octene, 1-nonene, 1-decene, 1-dodecene, 4-methyl-1-pentene, or 4-methyl-1-hexene; a cyclic olefin such as norbornene; an alkenyl aromatic hydrocarbyl such as styrene; an unsaturated carboxylic acid such as acrylic acid or methacrylic acid; an unsaturated carboxylate such as methyl acrylate, ethyl acrylate, propyl acrylate, butyl acrylate, methyl methacrylate, ethyl methacrylate, propyl methacrylate, or butyl methacrylate; a vinyl ester compound such as vinyl acetate; a polyene compound such as 5-ethylidene-2-norbornene or dicyclopentadie; vinyl fluoride, vinyl chloride, vinyl bromide, tetrafluoroethylene, diethyl maleate, and diethyl fumarate.

As propylene, in addition to propylene produced in the present invention, fossil resource-derived propylene may be used, biomass-derived propylene may be used, or both fossil resource-derived propylene and biomass-derived propylene may be used. In addition, as a monomer other than propylene, a monomer produced in the present invention (for example, ethylene, 1-butene, 2-butene, 1-pentene, 1-hexene, or 1-heptene) may be used, a fossil resource-derived monomer (for example, ethylene, 1-butene, 2-butene, 1-pentene, 1-hexene, or 1-heptene) may be used, and a biomass-derived monomer (for example, ethylene, 1-butene, 2-butene, 1-pentene, 1-hexene, or 1-heptene) may be used. Two or more of these monomers, such as monomer produced in the present invention/fossil resource-derived monomer, monomer produced in the present invention/biomass-derived monomer, fossil resource-derived monomer/biomass-derived monomer, or monomer produced in the present invention/fossil resource-derived monomer/biomass-derived monomer, may be used in combination.

Specific examples of the polymer include a propylene homopolymer, a propylene-ethylene copolymer, a propylene-1-butene copolymer, a propylene-1-hexene copolymer, a propylene-ethylene-1-butene copolymer, a propylene-ethylene-1-hexene copolymer, and a propylene-ethylene-1-butene-1-hexene copolymer. These copolymers may be each a random polypropylene or a block polypropylene (heterophasic polypropylene).

The content of a propylene unit in the propylene-based polymer is usually 50 mass% or more, and preferably 70 mass% or more with respect to 100 mass% of the propylene-based polymer. Note that the content of the propylene unit is measured by an IR method or a $^{13}$C-NMR method.

A melt mass flow rate (MFR) of the propylene-based polymer is usually 0.01 to 1000 g/10 min, and preferably 0.05 to 100 g/10 min. Note that the MFR is measured at a temperature of 230°C under a load of 2.16 kg defined in JIS K7210-1-2014.

A molecular weight distribution (mass average molecular weight (Mw)/number average molecular weight (Mn)) of the propylene-based polymer is usually 2 to 15, and preferably 3 to 10.

When the propylene-based polymer is a homopolypropylene, an isotactic pentad fraction of the homopolypropylene is preferably 0.9 to 0.999, and more preferably 0.95 to 0.998. The isotactic pentad fraction is measured by a method described in Macromolecules, volume 6, page 925 (published in 1973). The isotactic pentad fraction refers to an isotactic fraction in continuous pentad units in a polymer chain. In measurement using a $^{13}$C-NMR spectrum, an intensity fraction of a mmmm peak in a total absorption peak of a methyl carbon region of the $^{13}$C-NMR spectrum is taken as the isotactic pentad fraction. Note that peak attribution in the NMR spectrum is performed on the basis of description of Macromolecules, volume 8, page 687 (published in 1975).

When the propylene-based polymer is a random polypropylene obtained by random copolymerization of propylene and an olefin, the content of a propylene unit in the random polypropylene is preferably 90 to 99.9 mass%, and more preferably 92 to 99 mass%, and the content of an olefin unit is preferably 0.1 to 10 mass%, and more preferably 1 to 8 mass% with respect to 100 mass% of the random polypropylene. Note that the content of the monomer unit is measured by an IR method or a $^{13}$C-NMR method.

A melting point of the random polypropylene is usually 100 to 160°C, and preferably 115 to 155°C. When the melting point is within this range, transparency and sealability are excellent. Note that the melting point is measured using a differential scanning calorimeter in accordance with JIS-K7121.

When the propylene-based polymer is a block polypropylene, the block polypropylene is preferably a propylene-ethylene block copolymer containing a polymer component (a) formed of a propylene homopolymer or a propylene-ethylene random copolymer containing 95 to 100 mass% of propylene units and 0 to 5 mass% of ethylene units (Note that the total amount of the propylene units and the ethylene units is 100 mass%.), and a propylene-ethylene random copolymer component (b) containing 20 to 80 mass% of propylene units and 20 to 80 mass% of ethylene units.

A limiting viscosity [η] of the polymer component (a) contained in the propylene-ethylene block copolymer is preferably 0.8 to 3 dl/g. A limiting viscosity [η] of the propylene-ethylene random copolymer component (b) contained in the propylene-ethylene block copolymer is preferably 1 to 10 dl/g. Note that the limiting viscosity is measured in a tetralin solution at a temperature of 135°C.

The content of the polymer component (a) contained in the propylene-ethylene block copolymer is preferably 55 to 95 mass%, and more preferably 65 to 90 mass%. The content of the propylene-ethylene random copolymer component (b) contained in the propylene-ethylene block copolymer is preferably 5 to 45 mass%, and more preferably 10 to 35 mass%. Note that the total amount of the polymer component (a) and the propylene-ethylene random copolymer component (b) contained in the propylene-ethylene block copolymer is 100 mass%.

Examples of a method for producing the propylene-based polymer include a method for polymerizing a monomer containing propylene using a stereoregular olefin polymerization catalyst. As the polymerization catalyst, a known stereoregular olefin polymerization catalyst, for example, a polymerization catalyst described in "New Edition Polypropylene Handbook" (edited by Nero Pasquini, published by the Nikkan Kogyo Shimbun, Ltd. on September 28, 2012) can be used, and examples thereof include a Ziegler-Natta catalyst system, a metallocene catalyst system, and a catalyst system in which these catalyst systems are combined. Examples of the polymerization method include a bulk polymerization method, a solution polymerization method, a slurry polymerization method, a gas phase polymerization method, and a polymerization method in which these polymerization methods are arbitrarily combined, and each of these polymerization methods can be either a continuous polymerization method or a batch polymerization method. Furthermore, the method for producing the propylene-based polymer may be a single-stage polymerization method or a multi-stage polymerization method. When the polymerization is performed by the multi-stage polymerization method, a plurality of polymerization reactors may be connected in series or connected in parallel, or both polymerization reactors connected in series and polymerization reactors connected in parallel may be used. A process described in "New Edition Polypropylene Handbook" (published by Nikkan Kogyo Shimbun, Ltd. in 2012) can be used. The method for producing the propylene-based polymer is preferably a continuous gas phase polymerization method. Polymerization conditions such as a polymerization temperature, a polymerization pressure, a monomer concentration, a catalyst putting amount, and a polymerization time can be appropriately determined according to a composition, a structure, or the like of an intended propylene-based polymer.

Various known resins, rubbers, and additives may be blended in the propylene-based polymer for use. Examples of the resin and the rubber include the polymers exemplified as a resin and a rubber that may be blended in the ethylene-based polymer. Examples of the resin and rubber further include an ethylene-1-butene copolymer, an ethylene-1-hexene copolymer, an ethylene-1-octene copolymer, an ethylene-propylene-5-ethylidene-2-norbornene copolymer, a styrene-butadiene-styrene block copolymer, a hydrogenated styrene-butadiene-styrene block copolymer, a styrene-isoprene-styrene block copolymer, and a hydrogenated styrene-isoprene-styrene block copolymer.

Examples of the additive include the additives exemplified as an additive that may be blended in the ethylene-based polymer. Examples of the additive further include additives and formulations described in "New Edition Polypropylene Handbook" (edited by Nero Pasquini, published by the Nikkan Kogyo Shimbun, Ltd. on September 28, 2012) can be mentioned.

The propylene-based polymer can be molded into various compacts by a known molding method. Examples of the molding method include the molding methods exemplified for the ethylene-based polymer.

The compact using the propylene-based polymer may be a single-layer compact containing the propylene-based polymer, or may be a multilayer compact having a layer other than the layer containing the propylene-based polymer. Examples of the layer other than the layer containing the propylene-based polymer include the layers exemplified in the compact using the ethylene-based polymer.

The compact can be applied to various known applications such as automobile parts such as automobile interior parts and exterior parts, motorcycle parts, food and medical containers, parts of furniture and electrical products, and civil engineering and construction materials. Examples of the automobile exterior parts include a bumper, a fender, and a wheel cover, examples of the automobile interior parts include an instrumental panel, a door trim, a door panel, a pillar, a side protector, a console box, and a column cover, and examples of the motorcycle parts include a cowling and a muffler cover. Examples of the food and medical containers include a retort pouch, a retort container, a microwave oven heat-resistant container, a frozen food container, a special shape liquid packaging bag (such as a standing pouch), a wrap film, a blow bottle, a cap, a label, an infusion bag, an infusion bottle, a medical product container, and a cosmetic product container. Examples of parts of furniture and electrical products include wall paper, a floor material, a decorative sheet, and a drain hose for a washing machine. Examples of the civil engineering and construction materials include a waterproof sheet, a water shielding sheet, a hose, a duct, and a gasket.

<Butadiene-based polymer>

By polymerizing a monomer containing butadiene produced in the present invention, a butadiene homopolymer and a copolymer of butadiene and a monomer other than butadiene can be produced.

Examples of the butadiene include 1,3-butadiene and 1,2-butadiene. In addition, in addition to the butadiene produced in the present invention, butadiene produced using fossil resources such as petroleum, coal, and natural gas as raw materials (fossil resource-derived butadiene) may be used, butadiene produced using plants such as sugar cane and corn as raw materials (biomass-derived butadiene) may be used, or both fossil resource-derived butadiene and biomass-derived butadiene may be used.

Examples of the monomer copolymerizable with butadiene include an aromatic vinyl compound, a non-conjugated diene, a vinyl cyanide compound, an unsaturated carboxylic acid, an unsaturated carboxylic acid alkyl ester, and an unsaturated carboxylic acid amide.

Examples of the aromatic vinyl compound include styrene, p-methylstyrene, α-methylstyrene, vinylethylbenzene, vinylxylene, vinylnaphthalene, and diphenylethylene. Examples of the non-conjugated diene include dicyclopentadiene, 5-ethylidene-2-norbornene, and 1,5-hexadiene. Examples of the vinyl cyanide compound include acrylonitrile and methacrylonitrile. Examples of the unsaturated carboxylic acid include acrylic acid, methacrylic acid, crotonic acid, maleic acid, fumaric acid, and itaconic acid. Examples of the unsaturated carboxylic acid alkyl ester include methyl acrylate, ethyl acrylate, butyl acrylate,-2-ethylhexyl acrylate, methyl methacrylate, ethyl methacrylate, hexyl methacrylate, octyl methacrylate,-2-ethylhexyl methacrylate, lauryl methacrylate, and glycidyl methacrylate. Examples of the unsaturated carboxylic acid amide include acrylamide, methacrylamide, N-methylolacrylamide, N-methylolmethacrylamide, N,N-dimethylacrylamide, N,N-dimethylaminopropylacrylamide, and N,N-dimethylaminopropylmethacrylamide.

In addition, these copolymerizable monomers may be each a compound produced in the present invention or a monomer derived therefrom, may be a fossil resource-derived monomer, may be a biomass-derived monomer, or may be a combination thereof.

Specific examples of the polymer include a 1,3-butadiene homopolymer, a 1,3-butadiene-styrene copolymer, a 1,3-butadiene-acrylonitrile copolymer, a 1,3-butadiene-methyl methacrylate copolymer, a 1,3-butadiene-styrene-acrylonitrile copolymer, a 1,3-butadiene-acrylonitrile-acrylic acid copolymer, a 1,3-butadiene-styrene-acrylonitrile-methyl methacrylate copolymer, a 1,3-butadiene-styrene-acrylonitrile-acrylic acid copolymer, and a 1,3-butadiene-styrene-acrylonitrile-methyl methacrylate-acrylic acid copolymer.

The content of a butadiene unit in the butadiene-based polymer is preferably 20 mass% or more with respect to 100 mass% of the butadiene-based polymer.

Among the butadiene-based polymers, a copolymer of butadiene and another monomer copolymerizable with an unsaturated carboxylic acid is preferable for latex for paper coating or an adhesive. The content of a butadiene unit in the copolymer is preferably 20 to 80 mass%, the content of an unsaturated carboxylic acid unit is preferably 0.5 to 15 mass%, and the content of another copolymerizable monomer unit is preferably 5 to 79.5 mass%. Note that the amount of the copolymer is 100 mass%.

There are preferably at least two glass transition points of the butadiene-based polymer preferable for latex between -100 to 50°C. More preferably, there is at least one point at -100 to 0°C as a low glass transition point, and there is at least one point at -5 to 50°C as a high glass transition point. Note that the glass transition point is measured using a differential scanning calorimeter.

Examples of the method for producing the butadiene-based polymer include a method in which a monomer containing butadiene is polymerized by an emulsion polymerization method using a known radical polymerization initiator. In the emulsion polymerization method, a batch polymerization method is preferable, and a multi-stage polymerization method or a seed polymerization method may be used. Examples of the method for producing a butadiene-based polymer include a method in which a monomer containing butadiene is polymerized by a solution polymerization method using an organic alkali metal catalyst or a metallocene catalyst. The solution polymerization method can be either a continuous polymerization method or a batch polymerization method, and may be either a single-stage polymerization method or a multi-stage polymerization method.

The butadiene-based polymer preferable for latex is useful as a binder for particles, powder, or a filler made of metal, an inorganic compound, ceramics, a pigment, a phosphor, glass, or the like. In particular, the butadiene-based polymer can be preferably used as a binder of a paper coating composition and a binder of an electrochemical device electrode forming composition such as a battery, a capacitor, or a lithium ion capacitor. In addition, the butadiene-based polymer can be used for various rubber applications, for example, an industrial product such as a tire, a vibration-proof rubber, a belt, a hose, or a vibration-proof rubber, and footwear such as men's shoes, women's shoes, or sport shoes.

[Method for producing compound and method for producing polymer]

A method for producing a compound according to the present embodiment includes a step of synthesizing at least one compound selected from the compound group consisting of methyl methacrylate, propylene oxide, ethylene oxide, ethylene glycol, phenol, acetone, and isopropyl alcohol using, as a raw material, a C2-C8 unsaturated hydrocarbon obtained by the method for producing a C2-C8 unsaturated hydrocarbon according to any one of the first to seventh embodiments or a C2-C8 unsaturated hydrocarbon mixture obtained by the above-described method for producing a C2-C8 unsaturated hydrocarbon mixture.

A method for producing a polymer according to the present embodiment includes a step of polymerizing a monomer containing at least one compound selected from the compound group consisting of methyl methacrylate, propylene oxide, ethylene oxide, and ethylene glycol, the monomer being produced by using, as a raw material, a C2-C8 unsaturated hydrocarbon obtained by the method for producing a C2-C8 unsaturated hydrocarbon according to any one of the first to seventh embodiments or a C2-C8 unsaturated hydrocarbon mixture obtained by the above-described method for producing a C2-C8 unsaturated hydrocarbon mixture.

<Method for producing methyl methacrylate>

Methyl methacrylate which is a monomer of a methyl methacrylate-based polymer can be produced using, as raw materials, a C2-C8 unsaturated hydrocarbon and a C2-C8 unsaturated hydrocarbon mixture produced in the present invention by a known method such as a production method described on pages 4 to 14 of "Sumitomo Chemical 2004 II". Examples of a method for producing methyl methacrylate from ethylene include a BASF method and an alpha process. Examples of a method for producing methyl methacrylate from isobutylene include a direct oxidation method, a methacrylonitrile method (MAN), and a direct oxidation esterification method. This isobutylene may be produced by dehydrogenation of isobutane, may be produced from tert-butyl alcohol produced using isobutane, isobutylene, or the like as a starting material, or may be produced from methyl-tert-butyl ether (MTBE) produced using isobutane, isobutylene, or the like as a starting material. Examples of a method for producing methyl methacrylate from acetone include an acetone cyanhydrin (ACH) method. This acetone is produced using propylene, benzene, toluene, or the like as a starting material.

Among these methods, the method for producing methyl methacrylate is preferably a method for producing methyl methacrylate using, as a raw material, at least one compound selected from the compound group consisting of ethylene, propylene, and isobutylene obtained by the production method of the present invention.

The method for producing methyl methacrylate using ethylene as a raw material is preferably an alpha process. The alpha process is a method in which ethylene, carbon monoxide, and methanol are caused to react with each other to synthesize methyl propionate, and then methyl propionate and formaldehyde are caused to react with each other to produce methyl methacrylate. Examples of a catalyst for the reaction of ethylene, carbon monoxide, and methanol include a palladium complex catalyst, and examples of a catalyst for the reaction of methyl propionate and formaldehyde include a $Cs/SiO_2$ solid base catalyst.

The method for producing methyl methacrylate using propylene as a raw material is preferably an acetone cyanhydrin (ACH) method using acetone synthesized using propylene as a raw material. Acetone can be synthesized by a known method using propylene as a raw material. Examples thereof include a method in which propylene and oxygen are caused to react with each other to directly oxidize propylene. Examples of a catalyst include a palladium chloride-copper chloride-based catalyst. Examples of the method for synthesizing acetone include a method in which propylene is caused to react with benzene to synthesize cumene (isopropylbenzene), then the cumene is oxidized to synthesize cumene hydroperoxide, and the cumene hydroperoxide is decomposed to produce acetone and phenol. Examples of the ACH method include: a method in which acetone is caused to react with hydrogen cyanide to synthesize acetone cyanhydrin, acetone cyanhydrin is caused to react with sulfuric acid to synthesize a methacrylamide sulfate, and the methacrylamide sulfate is caused to react with methanol to produce methyl methacrylate; and a method in which acetone is caused to react with hydrogen cyanide to synthesize acetone cyanhydrin, acetone cyanhydrin is hydrated to synthesize $\alpha$-hydroxyiso-butyramide, $\alpha$-hydroxyisobutyramide is caused to react with methyl formate to synthesize methyl $\alpha$-hydroxyisobutyrate, and methyl $\alpha$-hydroxyisobutyrate is dehydrated to produce methyl methacrylate.

The method for producing methyl methacrylate using isobutylene as a raw material is preferably a direct oxidation method. Examples of the direct oxidation method include: a method in which isobutylene is oxidized with oxygen to synthesize methacrolein, methacrolein is oxidized with oxygen to synthesize methacrylic acid, and then methacrylic acid is caused to react with methanol to produce methyl methacrylate; a method in which isobutylene is hydrated to synthesize tert-butyl alcohol, tert-butyl alcohol is oxidized with oxygen to synthesize methacrolein, methacrolein is oxidized with oxygen to synthesize methacrylic acid, and then methacrylic acid is caused to react with methanol to produce methyl methacrylate; and a method in which isobutylene is hydrated to synthesize tert-butyl alcohol, then tert-butyl alcohol is dehydrated to synthesize isobutylene, then isobutylene is oxidized with oxygen to synthesize methacrolein, methacrolein is oxidized with oxygen to synthesize methacrylic acid, and methacrylic acid is caused to react with methanol to produce methyl methacrylate.

A catalyst used in the oxidation reaction of isobutylene and tert-butyl alcohol is preferably a catalyst containing molybdenum and bismuth, and more preferably a Mo-Bi-Fe-X-A-O-based catalyst (X represents at least one element selected from the group consisting of cobalt and nickel, and A represents at least one element selected from the group consisting of an alkali metal, an alkaline earth metal, and thallium.). A catalyst used in the oxidation reaction of methacrolein is preferably a catalyst containing molybdenum and phosphorus, and more preferably a P-Mo-V-Cu-A-O-based catalyst (A represents at least one element selected from the group consisting of an alkali metal, an alkaline earth metal, and thallium.). As these catalysts, catalysts described in JP-A-5-23596, WO-A-2019/013116, WO-A-2020/196853, and WO-A-2021/200689 can be used.

Examples of a catalyst used for the reaction between methacrylic acid and methanol include acid catalysts such as sulfuric acid and a strongly acidic cation exchange resin. Examples of a catalyst used in the hydration reaction of isobutylene include acid catalysts such as a strongly acidic cation exchange resin and a heteropoly acid. Examples of a catalyst used in the dehydration reaction of tert-butyl alcohol include: solid acids such as solid phosphoric acid, active alumina, and silica-alumina; a strong acid such as sulfuric acid; and an acid catalyst such as a strongly acidic ion exchange resin containing a sulfonic acid group.

<Method for producing methyl methacrylate-based polymer>

A method for producing a methyl methacrylate-based polymer includes a step of polymerizing a monomer containing methyl methacrylate produced using, as a raw material, a C2-C8 unsaturated hydrocarbon obtained by the method for producing a C2-C8 unsaturated hydrocarbon according to any one of the first to seventh embodiments or a C2-C8 unsaturated hydrocarbon mixture obtained by the above-described method for producing a C2-C8 unsaturated hydrocarbon mixture.

Examples of the method for producing a methyl methacrylate-based polymer include a method in which a monomer containing methyl methacrylate is polymerized with a known radical polymerization initiator. Examples of the polymerization method include a suspension polymerization method, a solution polymerization method, and a bulk polymerization method, and the bulk polymerization method is preferable. The method for producing a methyl methacrylate-based polymer can be either a continuous polymerization method or a batch polymerization method, and may be either a single-stage polymerization method or a multi-stage polymerization method. For example, production methods described in JP-A-2011-168683, JP-A-2014-108988, WO-A-2017/010323, and JP-A-2021-155698 can be used.

(Methyl methacrylate-based polymer)

By polymerizing a monomer containing methyl methacrylate obtained by the above production method, a methyl methacrylate homopolymer and a copolymer of methyl methacrylate and a monomer other than methyl methacrylate can be produced.

As the methyl methacrylate, in addition to methyl methacrylate obtained by the above production method, methyl methacrylate produced using fossil resources such as petroleum, coal, and natural gas as a raw material (fossil resource-derived methyl methacrylate) may be used, methyl methacrylate produced using plants such as sugar cane and corn as a raw material (biomass-derived methyl methacrylate) may be used, or both a fossil resource-derived methyl methacrylate and a biomass-derived methyl methacrylate may be used.

Examples of the monomer copolymerizable with methyl methacrylate include an acrylate, an unsaturated carboxylic acid, an unsaturated carboxylic acid amide, a vinyl cyanide compound, and an aromatic vinyl compound.

Examples of the acrylate include methyl acrylate, ethyl acrylate, propyl acrylate, butyl acrylate, cyclohexyl acrylate, benzyl acrylate, 2-ethylhexyl acrylate, 2-hydroxyethyl acrylate, and cyclopentadiene acrylate. Examples of the unsaturated carboxylic acid include acrylic acid, methacrylic acid, maleic acid, itaconic acid, maleic anhydride, and itaconic anhydride. Examples of the vinyl cyanide compound include acrylonitrile and methacrylonitrile. Examples of the unsaturated carboxylic acid amide include acrylamide and methacrylamide. Examples of the aromatic vinyl compound include styrene and $\alpha$-methylstyrene.

In addition, these copolymerizable monomers may be each a compound produced in the present invention or a monomer derived therefrom, may be a fossil resource-derived monomer, may be a biomass-derived monomer, or may be a combination thereof.

Specific examples of the polymer include a methyl methacrylate homopolymer, a methyl methacrylate-methyl acrylate copolymer, a methyl methacrylate-ethyl acrylate copolymer, a methyl methacrylate-methyl acrylate-butyl acrylate copolymer, a methyl methacrylate-butyl acrylatestyrene copolymer, and a methyl methacrylate-styrene-maleic anhydride copolymer.

The content of a methyl methacrylate unit in the methyl methacrylate-based polymer is preferably 80 mass% or more, and more preferably 90 mass% or more with respect to 100 mass% of the methyl methacrylate-based polymer. Note that the content of the methyl methacrylate unit is measured by thermal decomposition gas chromatography.

A mass average molecular weight of the methyl methacrylate-based polymer is preferably 50,000 to 300,000, and a molecular weight distribution (mass average molecular weight (Mw)/number average molecular weight (Mn)) thereof is usually 2 to 10.

Various known resins, rubbers, and additives may be blended in the methyl methacrylate-based polymer for use. The resin and the rubber may be each a methyl methacrylate-based polymer produced using fossil resource-derived methyl methacrylate as a raw material (fossil resource-derived polymethyl methacrylate), or may be a methyl methacrylate-based polymer produced using biomass-derived methyl methacrylate as a raw material (biomass-derived polymethyl methacrylate). In addition, the resin and the rubber may be each a recycled methyl methacrylate-based polymer (regenerated polymethyl methacrylate). Furthermore, two or more types thereof may be combined.

Examples of the additive include the additives exemplified as an additive that may be blended in the ethylene-based polymer.

The methyl methacrylate-based polymer can be molded into various compacts by a known molding method such as injection molding, extrusion molding, or pressure molding. In addition, an obtained compact may be further secondarily molded by a method such as pressure molding or vacuum molding.

The compact can be applied to various known applications such as optical materials, vehicle parts, lighting materials, and building materials. Examples of the vehicle parts of an automobile include a rear lamp outer cover, an optical member inside a rear lamp, an inner lens for a headlight (also referred to as a projector lens or a PES lens), a meter cover, a door mirror housing, a pillar cover (sash cover), a license garnish, a front grille, a fog garnish, and an emblem.

<Method for producing propylene oxide>

Propylene oxide can be produced using propylene as a raw material by a known method such as a production method described in "Sumitomo Chemical 2006-I" on pages 4 to 10 and "Sumitomo Chemical 2019" on pages 4 to 11. Examples thereof include a chlorine method (chlorohydrin method), a cumene method (POC method), a hydrogen peroxide method (HPPO method), an improved hydrogen peroxide method (improved HPPO method), a direct oxidation method, a halcone method (ethylbenzene method (propylene oxide/styrene monomer (PO/SM) co-production method), and an isobutane method (propylene oxide/tert-butanol (PO/TBA) co-production method).

The chlorine method (chlorohydrin method) is a method in which propylene chlorohydrin is synthesized from propylene, chlorine, and water, and then propylene chlorohydrin is dehydrochlorinated with calcium hydroxide to produce propylene oxide. The hydrogen peroxide method (HPPO method) and the improved hydrogen peroxide method (improved HPPO method) are methods for producing propylene oxide from propylene and hydrogen peroxide. The cumene method is a propylene oxide producing method in which cumene is oxidized to synthesize cumene hydroperoxide, then propylene is epoxidized with the cumene hydroperoxide to synthesize propylene oxide, and $\alpha$-cumyl alcohol by-produced by the epoxidation is hydrogenated to synthesize cumene.

<Method for producing propylene oxide-based polymer>

The method for producing a propylene oxide-based polymer includes a step of polymerizing a monomer containing propylene oxide produced using propylene obtained by the production method of the present invention as a raw material.

(Propylene oxide-based polymer)

Examples of the propylene oxide-based polymer include polypropylene glycol and a copolymer of polypropylene glycol and polyisocyanate. These polymers can be produced by a known method. For example, polypropylene glycol can be produced by homopolymerization of propylene oxide or copolymerization of propylene oxide and ethylene oxide using a polyhydric alcohol (ethylene glycol, dipropylene glycol, glycerin, sorbitol, sucrose, or the like), an amine, a polyamine, or the like as a polymerization initiator. Examples of a catalyst include a basic catalyst such as potassium hydroxide. Examples of a catalyst for the copolymerization of polypropylene glycol and polyisocyanate include an amine-based catalyst (triethylenediamine, bis(2-dimethylaminoethyl) ether, N,N,N',N'-tetramethylhexamethylenediamine, or the like) and a metal-based catalyst (dibutyltin dilaurate, stannous octoate, or the like).

Here, examples of the polyisocyanate include tolylene diisocyanate (TDI), diphenylmethane diisocyanate (MDI), polymethylene polyphenyl polyisocyanate (polymeric MDI), xylylene diisocyanate (XDI), naphthylene diisocyanate (NDI), paraphenylene diisocyanate (PPDI), tetramethylxylylene diisocyanate (TMXDI), hexamethylene diisocyanate (HDI), dicyclohexylmethane diisocyanate (HMDI), and isophorone diisocyanate (IPDI).

<Method for producing ethylene oxide>

Ethylene oxide can be produced by a known method using ethylene produced in the present invention as a raw material. For example, ethylene oxide can be produced by a method for performing catalytic gas phase oxidation of ethylene oxide with a molecular oxygen-containing gas. Examples of a catalyst include a silver catalyst. More specific examples thereof include methods described in JP-A-62-103072, JP-A-2010-36104, JP-A-2014-198680, and WO-A-2020/032279.

<Method for producing ethylene oxide-based polymer>

A method for producing an ethylene oxide-based polymer includes a step of polymerizing a monomer containing ethylene oxide produced using ethylene obtained by the production method of the present invention as a raw material.

(Ethylene oxide-based polymer)

The ethylene oxide-based polymer can be produced by a known method. For example, the ethylene oxide-based polymer can be produced by a method for ring-opening and polymerizing ethylene oxide. Examples of a catalyst include a metal alkoxide catalyst. In addition, examples of the method for producing the ethylene oxide-based polymer include a method in which ethylene oxide is polymerized using a polyhydric alcohol (ethylene glycol, glycerin, sorbitol, sucrose, or the like), an amine, a polyamine, or the like as a polymerization initiator. Examples of a catalyst include a basic catalyst such as potassium hydroxide.

<Method for producing ethylene glycol>

Ethylene glycol can be produced by a known method using the ethylene oxide as a raw material. For example, ethylene glycol can be produced by a method for causing ethylene oxide to react with water. This is performed in the presence or absence of an acid catalyst. In addition, examples of the method for producing ethylene glycol include a method for causing ethylene oxide to react with carbon dioxide to generate ethylene carbonate, and then causing ethylene carbonate to react with water. Examples of a catalyst include a bromide of an alkali metal, an iodide of an alkali metal, and a fourth phosphonium halide. More specific examples thereof include methods described in JP-A-54-16416, JP-A-2001-316308, JP-A-2000-128814, and JP-A-2002-201148.

<Method for producing ethylene glycol-based polymer>

A method for producing an ethylene glycol-based polymer includes a step of polymerizing a monomer containing ethylene oxide produced using ethylene obtained by the production method of the present invention as a raw material.

(Ethylene glycol-based polymer)

Examples of the ethylene glycol-based polymer include a copolymer of ethylene glycol and a dicarboxylic acid or a dimethyl dicarboxylate. Here, examples of the dicarboxylic acid include terephthalic acid, isophthalic acid, and 2,6-naphthalenedicarboxylic acid, and examples of the dimethyl dicarboxylate include dimethyl terephthalate, dimethyl isophthalate, and dimethyl 2,6-naphthalenedicarboxylate. These polymers can be produced, for example, by a method in which a dicarboxylic acid and ethylene glycol are directly esterified or a dimethyl dicarboxylate and ethylene glycol are subjected to transesterification to synthesize a bishydroxyethyl compound (for example, bishydroxyethyl terephthalate, bishydroxyethyl isophthalate, or bishydroxyethyl 2,6-naphthalenedicarboxylate), and the bishydroxyethyl compound is polycondensed.

The direct esterification reaction is usually performed without a catalyst, and if necessary, an alkali metal, an amine compound, or the like is used as a diethylene glycol generation inhibitor. Examples of a catalyst for the transesterification reaction include weak acid salts such as calcium, manganese, magnesium, zinc, and lithium; and a titanate, and specifically, calcium acetate, magnesium acetate, lithium acetate, and the like can be used. Examples of a catalyst for the polycondensation reaction include an antimony compound such as antimony trioxide, a germanium compound such as germanium dioxide, titanium compounds such as tetra-n-propyl titanate, tetra-i-propyl titanate, and tetra-n-butyl titanate, and aluminum compounds such as aluminum acetate, aluminum chloride, aluminum hydroxide, aluminum hydroxychloride, and aluminum acetylacetonate.

<Method for producing phenol>

Phenol can be produced by a known method using propylene obtained by the production method of the present invention as a raw material. For example, by causing propylene to react with benzene to synthesize cumene (isopropylbenzene), then oxidizing the cumene with a mixed gas containing oxygen and an inert gas or an oxygen-containing gas such as air to synthesize cumene hydroperoxide, and decomposing the cumene hydroperoxide, phenol and acetone can be produced. Here, examples of a catalyst for the reaction between propylene and benzene include an aluminum chloride catalyst, a zeolite catalyst, and a phosphoric acid catalyst. The oxidation reaction of cumene is performed by autoxidation. This oxidation reaction may be performed without using an additive, or an additive such as an alkali may be used. Examples of the additive include an alkali metal compound such as NaOH or KOH, an alkaline earth metal compound, an alkali metal carbonate such as $Na_2CO_3$ or $NaHCO_3$, ammonia, $(NH_4)_2CO_3$, and an alkali metal ammonium carbonate. Examples of a catalyst for the decomposition reaction of cumene hydroperoxide include an acid catalyst such as sulfuric acid.

<Method for producing acetone>

Acetone can be produced by a known method using propylene obtained by the production method of the present invention as a raw material. For example, acetone can be produced by a method for causing propylene to react with oxygen to directly oxidize propylene. Examples of a catalyst include a palladium chloride-copper chloride-based catalyst. Examples of the method for producing acetone include a method in which propylene is caused to react with benzene to synthesize cumene (isopropylbenzene), then the cumene is oxidized to synthesize cumene hydroperoxide, and the cumene hydroperoxide is decomposed to produce acetone and phenol as in the above-described method for producing phenol.

<Method for producing isopropyl alcohol>

Isopropyl alcohol can be produced by a known method using propylene obtained by the production method of the present invention as a raw material. For example, isopropyl alcohol can be produced by a method for causing propylene to react with water. Examples of a catalyst include a strongly acidic cation exchange resin; and an acid catalyst, for example, a heteropolyacid such as phosphotungstic acid, silicotungstic acid, or silicomolybdic acid. Specific examples thereof include methods described in JP-A-8-165259, JP-A-8-165260, JP-A-8-291092, and WO-A-2017/217279. In addition, examples of the method for producing isopropyl alcohol include a method in which propylene is caused to react with benzene to synthesize cumene (isopropylbenzene), then the cumene is oxidized to synthesize cumene hydroperoxide, the cumene hydroperoxide is decomposed to produce acetone and phenol, and then the acetone is caused to react with hydrogen as in the above method for producing acetone. Examples of a catalyst for the reaction between acetone and hydrogen include hydrogenation catalysts such as a copper oxide-chromium oxide catalyst, a Raney nickel catalyst, and a noble metal catalyst such as platinum, palladium, or ruthenium. Specific examples thereof include methods described in JP-A-2002-121160 and JP-A-2002-128716. The catalyst is preferably a copper oxide-chromium oxide catalyst or a Raney nickel catalyst.

[Plant-derived carbon concentration]

In the production method of the present invention, a raw material based on fossil resources such as oil, coal, and natural gas (fossil resource-derived raw material) and a raw material based on plants such as sugar cane and corn (biomass-derived raw material) can be used in combination. Therefore, when a biomass-derived raw material is used in the production method of the present invention, a compound produced in the present invention and a derivative produced using the compound as a starting material (for example, the above compounds and the compounds described in Figs. 1 to 5) have plant-derived carbon. In addition, by applying mass balance approach to a biomass degree, the biomass degree can be allocated to all the compounds produced in the present invention and derivatives produced using the compounds as starting materials. Therefore, any product among these compounds and derivatives can be a biomass product depending on the biobass degree of the whole of these compounds and derivatives, and therefore the production method of the present invention is useful.

A plant-derived carbon concentration of the compound produced in the present invention and the derivative produced using the compound as a starting material (for example, the above compounds and the compounds described in Figs. 1 to 5) can be changed by adjusting, for example, an amount ratio between a fossil resource-derived raw material and a biomass-derived raw material, and is preferably 0.1 to 99.9 pMC (%), and more preferably 0.3 to 70 pMC (%). The plant-derived carbon concentration is more preferably 0.5 pMC (%) or more, still more preferably 1 pMC (%) or more, and particularly preferably 5 pMC (%) or more from a viewpoint of reducing an environmental load. In addition, the plant-derived carbon concentration is more preferably 60 pMC (%) or less, still more preferably 50 pMC (%) or less, and particularly preferably 40 pMC (%) or less from a viewpoint of cost.

As one aspect, a plant-derived carbon concentration of an olefin-based polymer produced using a C2-C8 unsaturated hydrocarbon or a C2-C8 unsaturated hydrocarbon mixture produced in the present invention is 0.1 to 99.9 pMC (%).

As another aspect, a plant-derived carbon concentration of a polymer produced using a C2-C8 unsaturated hydrocarbon or a C2-C8 unsaturated hydrocarbon mixture produced in the present invention is 0.1 to 99.9 pMC (%).

The plant-derived carbon concentration can be determined by measuring radioactive carbon ($^{14}C$). Since carbon dioxide in the atmosphere contains $^{14}C$ at a certain ratio (105.5 pMC), it is known that the content of $^{14}C$ in a plant that grows by taking in carbon dioxide in the atmosphere, for example, corn, is also about 105.5 pMC. It is also known that fossil resources contain almost no $^{14}C$. Therefore, the plant-derived carbon concentration of the compound and the derivative can be calculated by measuring a ratio of $^{14}C$ contained in all carbon atoms in the compound and the derivative. When the content of $^{14}C$ in the compound and the derivative is represented by $[^{14}C]$, a plant-derived carbon concentration $X_{bio}$ of the compound and the derivative can be determined as follows.

$$X_{bio}(\%)=[^{14}C]/105.5\times100$$

Theoretically, when plant-derived raw materials are used as all of raw materials of the compound and the derivative, the plant-derived carbon concentration of the compound and the derivative is 100%. In addition, the plant-derived carbon concentration of the compound and derivative produced only from fossil resource-derived raw materials is 0%.

The plant-derived carbon concentration is determined as a percentage of mordern carbon (pMC: unit%) by an accelerator mass spectrometry (AMS) method defined in ISO 16620-2: 2019.

## Claims

1. A method for producing a C2-C8 unsaturated hydrocarbon, comprising:

   step (1) of sorting, from a waste plastic, a plastic mixture (A) having a polyolefin-based plastic content of 50 mass% or more and a residue (B);
   step (2) of decomposing the plastic mixture (A) to obtain a reaction product containing a C2-C8 unsaturated hydrocarbon; and
   step (3) of cleaning and purifying the reaction product to separate the C2-C8 unsaturated hydrocarbon.

2. A method for producing a C2-C8 unsaturated hydrocarbon, comprising:

   step (1) of sorting, from a waste plastic, a plastic mixture (A) having a polyolefin-based plastic content of 50 mass% or more and a residue (B);
   step (12) of heating the residue (B) to obtain a mixed gas containing carbon monoxide, carbon dioxide, and hydrogen;
   step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0;
   step (14) of causing carbon monoxide, carbon dioxide, and hydrogen contained in the adjustment gas to react with each other to obtain an alcohol; and
   step (15) of obtaining a C2-C8 unsaturated hydrocarbon using the alcohol as a raw material.

3. A method for producing a C2-C8 unsaturated hydrocarbon, comprising:

   step (1) of sorting, from a waste plastic, a plastic mixture (A) having a polyolefin-based plastic content of 50 mass% or more and a residue (B);
   step (2) of decomposing the plastic mixture (A) to obtain a reaction product containing a C2-C8 unsaturated hydrocarbon;
   step (3) of cleaning and purifying the reaction product to separate the C2-C8 unsaturated hydrocarbon;
   step (12) of heating the residue (B) to obtain a mixed gas containing carbon monoxide, carbon dioxide, and hydrogen;

step (13) of mixing the mixed gas with at least one gas selected from the group consisting of carbon monoxide, carbon dioxide, and hydrogen or removing a part of at least one gas selected from the group consisting of carbon monoxide, carbon dioxide, and hydrogen from the mixed gas to obtain an adjustment gas in which a volume amount of hydrogen with respect to a total volume amount of carbon monoxide and carbon dioxide is 1.5 to 4.0; step (14) of causing carbon monoxide, carbon dioxide, and hydrogen contained in the adjustment gas to react with each other to obtain an alcohol; and

step (15) of obtaining a C2-C8 unsaturated hydrocarbon using the alcohol as a raw material.

4. The method for producing a C2-C8 unsaturated hydrocarbon according to claim 1 or 3, wherein the reaction product obtained in the step (2) further contains a paraffin.

5. A method for producing a C2-C8 unsaturated hydrocarbon, comprising:

step (21) of causing carbon dioxide and hydrogen to react with each other to obtain a reaction mixture containing an alcohol, carbon dioxide, and carbon monoxide; and
step (22) of obtaining a C2-C8 unsaturated hydrocarbon from the alcohol and/or carbon monoxide contained in the reaction mixture.

6. A method for producing a C2-C8 unsaturated hydrocarbon, comprising:

the method for producing a C2-C8 unsaturated hydrocarbon according to any one of claims 1 to 4; and
the method for producing a C2-C8 unsaturated hydrocarbon according to claim 5.

7. A method for producing a C2-C8 unsaturated hydrocarbon, comprising:

the method for producing a C2-C8 unsaturated hydrocarbon according to any one of claims 1 to 5; and
a method for producing a C2-C8 unsaturated hydrocarbon, comprising step (31) of subjecting at least one alcohol selected from the group consisting of ethanol, propanol, and butanol to a dehydration reaction to obtain a C2-C8 unsaturated hydrocarbon.

8. A method for producing a C2-C8 unsaturated hydrocarbon, comprising:

the method for producing a C2-C8 unsaturated hydrocarbon according to claim 6; and
a method for producing a C2-C8 unsaturated hydrocarbon, comprising step (31) of subjecting at least one alcohol selected from the group consisting of ethanol, propanol, and butanol to a dehydration reaction to obtain a C2-C8 unsaturated hydrocarbon.

9. A method for producing a C2-C8 unsaturated hydrocarbon mixture, comprising step (41) of bringing a C2-C8 unsaturated hydrocarbon obtained by the method for producing a C2-C8 unsaturated hydrocarbon according to any one of claims 1 to 8 into contact with a C2-C8 unsaturated hydrocarbon derived from fossil resources to obtain a mixture.

10. The method for producing a C2-C8 unsaturated hydrocarbon mixture according to claim 9, wherein a contact mass of the fossil resource-derived C2-C8 unsaturated hydrocarbon to a contact mass of the C2-C8 unsaturated hydrocarbon is 0.001 to 10,000.

11. A method for producing an olefin-based polymer, comprising a step of polymerizing a monomer containing a C2-C8 unsaturated hydrocarbon obtained by the method for producing a C2-C8 unsaturated hydrocarbon according to any one of claims 1 to 8 or a C2-C8 unsaturated hydrocarbon mixture obtained by the method for producing a C2-C8 unsaturated hydrocarbon mixture according to claim 9 or 10.

12. The method for producing an olefin-based polymer according to claim 11, wherein the olefin-based polymer is a butadiene-based polymer.

13. A method for producing a compound, comprising

a step of synthesizing at least one compound selected from the compound group consisting of methyl methacrylate, propylene oxide, ethylene oxide, ethylene glycol, phenol, acetone, and isopropyl alcohol

using, as a raw material, a C2-C8 unsaturated hydrocarbon obtained by the method for producing a C2-C8 unsaturated hydrocarbon according to any one of claims 1 to 8 or a C2-C8 unsaturated hydrocarbon mixture obtained by the method for producing a C2-C8 unsaturated hydrocarbon mixture according to claim 9 or 10.

14. A method for producing a polymer, comprising a step of polymerizing a monomer containing at least one compound selected from the compound group consisting of methyl methacrylate, propylene oxide, ethylene oxide, and ethylene glycol, the monomer being produced by using, as a raw material, a C2-C8 unsaturated hydrocarbon obtained by the method for producing a C2-C8 unsaturated hydrocarbon according to any one of claims 1 to 8 or a C2-C8 unsaturated hydrocarbon mixture obtained by the method for producing a C2-C8 unsaturated hydrocarbon mixture according to claim 9 or 10.

15. An olefin-based polymer obtained by the method for producing an olefin-based polymer according to claim 11 or 12, having a plant-derived carbon concentration of 0.1 to 99.9 pMC.

16. A polymer obtained by the method for producing a polymer according to claim 14, having a plant-derived carbon concentration of 0.1 to 99.9 pMC.

[Fig. 1]

EP 4 549 418 A1

49

```
Ethylene ──► Ethylene-based polymer
              Ethylene homopolymer, Ethylene-1-butene copolymer, Ethylene-1-hexene copolymer, Ethylene-vinyl acetate copolymer, Ethylene-methyl
              methacrylate copolymer, Ethylene-methyl acrylate copolymer, Ethylene-glycidyl methacrylate copolymer, and the like

          ├─► Ethanol ──► Ethyl acetate
          │
          ├─► Ethylene oxide ──► Ethylene glycol ──► Polyester, Polyethylene terephthalate
          │
          │                 Polyol HO-(CH2CH2O)n-H  (n is an integer)
          │                 Diethyl glycol, Polyethylene glycol, and the like
          │
          │                 Glycol ether
          │                 R-O-(CH2CH2O)n-H, R-O-(CH2CH2O)n-R' (R and R' are hydrocarbon groups, and n is an integer)
          │
          │                 Ethanol amine ──► Aziridine ──► Polyethylene imine
          │                 Ethylene carbonate
          ├─► Acetaldehyde ──► Pyridine
          │
          ├─► 1,2-Dichloroethane ──► Tetrachloroethylene
          │                          Trichloroethylene
          │                          Vinyl chloride ──► Vinyl chloride resin
          │                          Ethylene amine
          │
          ├─► Vinyl acetate ──► Vinyl acetate resin ──► Polyvinyl alcohol
          │
          ├─► Ethyl acetate
          │
          ├─► Ethyl benzene ──► Styrene ──► Styrene-based polymer
          │                                  Styrene homopolymer, Styrene-butadiene copolymer, Acrylonitrile-
          │                                  butadiene-styrene copolymer, Methyl methacrylate-butadiene-
          │                                  styrene copolymer, and the like
          ├─► 1-Butene ──► Polybutene
          │
          ├─► 1-Hexene ──► Polyhexene
          │
          └─► Methyl methacrylate ──► Methyl methacrylate-based polymer (Methacrylic resin)
```

【Ｆｉｇ．２】

Propylene

Propylene-based polymer
Propylene homopolymer, Propylene-ethylene copolymer, Propylene-1-butene copolymer, Propylene-ethylene-1-butene copolymer, and the like

Isopropyl alcohol → Acetone → Methyl isobutyl ketone

Acetone → Acetone cyanohydrin → Methyl methacrylate → Methyl methacrylate-based polymer (Methacrylic resin)

Acrylonitrile → Acrylamide

Propylene oxide → Propylene glycol

Polyol HO-(CH2CH2CH2O)n-H (n is an integer)
Dipropylene glycol, Polypropylene glycol, and the like

Glycol ether
R-O-(CH2CH2CH2O)n-H, R-O-(CH2CH2CH2O)n-R' (R and R' are hydrocarbon groups, and n is an integer)

Epichlorohydrin → Propylene oxide → Propylene glycol

Acetone → Methyl isobutyl ketone

Acetone cyanohydrin → Methyl methacrylate → Methyl methacrylate-based polymer (Methacrylic resin)

Acrolein → Acrylic acid → Acrylate → Acrylic resin

Allyl chloride → Epichlorohydrin → Glycerin

Epoxy resin

Butyl aldehyde → Butanol → Butyl acetate

Octanol → Dioctyl phtalate

Isobutyl aldehyde → Isobutyl alcohol → Isobutyl acetate

Cumene → Phenol → Bisphenol A → Polycarbonate, Epoxy resin

Acetone → Isopropyl alcohol

α-Methylstyrene

Ally acetate → n-Propyl acetate

Allyl alcohol

1,3-Isopropyl benzene → Resorcin

Acetone → m-Aminophenol

【Fig. 3】

Butadiene → Butadiene-based polymer, Butadiene rubber, Styrene-butadiene copolymer, Acrylonitrile-butadiene copolymer, Acrylonitrile-butadiene-styrene copolymer, Methyl methacrylate-butadiene-styrene copolymer, and the like

1,4-Butanediol → Polybutylene terephtalate
Tetrahydrofuran → Polytetramethylene glycol

1-Butene
2-Butene
2-Butanol → Methyl ethyl ketone

1-Butene → Polybutene

Isobutylene → Isobutylene-based polymer, Polyisobutylene, Butyl rubber, and the like

Diisobutylene → Isooctane
p-Octylphenol

tert-Butyl alcohol → Methyl methacrylate
Methyl-tert-butyl ether → Epichlorohydrin → Glycerin
Ethyl-tert-butyl ether

Methyl methacrylate → Methyl methacrylate-based polymer (Methacrylic resin)
Methyl methacrylate-based polymer (Methacrylic resin)

Butadiene
Maleic anhydride → γ-Butyrolactone → N-Methyl-2-pyrrolidone

n-Butane

Isobutylene → Isooctene

Isobutane → tert-Butyl alcohol
Methyl-tert-butyl ether
Ethyl-tert-butyl ether

Methyl methacrylate-based polymer (Methacrylic resin)

【Ｆｉｇ.4】

[Fig. 5]

EP 4 549 418 A1

Benzene
- Ethylbenzene → Styrene → Styrene-based polymer
  Styrene homopolymer, Styrene-butadiene copolymer, Acrylonitrile-butadiene-styrene copolymer, Methyl methacrylate-butadiene-styrene copolymer, and the like
- Cumene
  - Phenol → Bisphenol A → Polycarbonate, Epoxy resin
  - Acetone → Isopropyl alcohol
  - α-Methylstyrene
- 1,3-Isopropyl benzene
  - Resorcin → m-Aminophenol
  - Acetone
- Cyclohexane
  - Cyclohexanone → Caprolactam → Polyamide
  - Cyclohexanol
- Maleic anhydride
- Nitrobenzene → Aniline → Diphenylmethane diisocyanate
- Chlorobenzene

Toluene
- Benzene
- Phenol
- Dinitrotoluene → Toluene diamine → Toluene diisocyanate → Polyurethane
- Cymene
  - Acetone → Methyl isobutyl ketone
  - Cresol
- Benzoic acid

o-Xylene
- Maleic anhydride

m-Xylene
- Isophthalic acid → Polyamide, Polyester
- Isophthalonitrile → m-Xylene diamine

p-Xylene
- Terephthalic acid → Polyester, Polyethylene terephthalate
- Dimethyl terephthalate

53

[Fig. 6]

Plastic mixture (A) having a polyolefin-based plastic content of 50 mass% or more

Waste plastic → Step (1) Sorting → Step (2) Decomposition → Step (3) Cleaning/Purification → **C2-C8 unsaturated hydrocarbon**

Residue (B)

【Ｆｉｇ.7】

【Ｆ ｉ ｇ．８】

Alcohol, Carbon dioxide, Carbon monoxide

Step (21)

Carbon dioxide, Hydrogen → | Alcohol synthesis |

Step (22)

| C2-C8 unsaturated hydrocarbon synthesis | → **C2-C8 unsaturated hydrocarbon**

[Fig. 10]

EP 4 549 418 A1

58

Plastic mixture (A) having a polyolefin-based plastic
content of 50 mass% or more

Waste plastic →

Step (1)
Sorting

Step (2)
Decomposition

Step (3)
Cleaning/Purification → **C2-C8 unsaturated
hydrocarbon**

Residue (B)

Carbon monoxide,
Carbon dioxide,Hydrogen

Step (12)
Heating

Step (13)
Gas adjustment

Step (14)
Alcohol synthesis

Step (15)
C2-C8 unsaturated
hydrocarbon synthesis → **C2-C8 unsaturated
hydrocarbon**

Carbon monoxide,
Carbon

Alcohol, Carbon dioxide, Carbon monoxide

Carbon dioxide,
Hydrogen →

Step (21)
Alcohol synthesis

Step (22)
C2-C8 unsaturated
hydrocarbon synthesis → **C2-C8 unsaturated
hydrocarbon**

【Ｆｉｇ．１１】

Plastic mixture (A) having a polyolefin-based plastic content of 50 mass% or more

Waste plastic →

Step (1)
Sorting

Step (2)
Decomposition

Step (3)
Cleaning/Purification

→ **C2-C8 unsaturated hydrocarbon**

Residue (B)

Carbon monoxide, Carbon dioxide, Hydrogen

Step (12)
Heating

Step (13)
Gas adjustment

Step (14)
Alcohol synthesis

Step (15)
C2-C8 unsaturated hydrocarbon synthesis

→ **C2-C8 unsaturated hydrocarbon**

Carbon monoxide, Carbon dioxide, Hydrogen

Alcohol, Carbon dioxide, Carbon monoxide

Carbon dioxide, Hydrogen →

Step (21)
Alcohol synthesis

Step (22)
C2-C8 unsaturated hydrocarbon synthesis

→ **C2-C8 unsaturated hydrocarbon**

Ethanol, Propanol, Butanol →

Step (31)
Dehydration reaction

**C2-C8 unsaturated hydrocarbon**

## INTERNATIONAL SEARCH REPORT

| International application No. |
| --- |
| **PCT/JP2023/023215** |

**A.    CLASSIFICATION OF SUBJECT MATTER**

*C07C 4/22*(2006.01)i; *B29B 17/02*(2006.01)i; *C07C 1/20*(2006.01)i; *C07C 11/02*(2006.01)i; *C07C 11/12*(2006.01)i; *C08F 10/00*(2006.01)i; *C08F 22/00*(2006.01)i; *C08F 36/00*(2006.01)i; *C08J 11/12*(2006.01)i; *C08J 11/16*(2006.01)i

FI:    C07C4/22; C07C11/02; C07C11/12; C07C1/20; C08F10/00 510; C08F36/00 510; C08F22/00 510; B29B17/02; C08J11/12; C08J11/16

According to International Patent Classification (IPC) or to both national classification and IPC

**B.    FIELDS SEARCHED**

Minimum documentation searched (classification system followed by classification symbols)

C07C4/22; B29B17/02; C07C1/20; C07C11/02; C07C11/12; C08F10/00; C08F22/00; C08F36/00; C08J11/12; C08J11/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2002-121318 A (ISHIKAWAJIMA HARIMA HEAVY IND. CO., LTD.) 23 April 2002 (2002-04-23) claims 1, 7, fig. 1, paragraph [0012] | 1, 4, 9-14 |
| Y | | 1-4, 6-16 |
| Y | JP 2009-240920 A (TOSHIBA PLANT SYSTEMS & SERVICES CORP.) 22 October 2009 (2009-10-22) claims 1, 3, paragraph [0010] | 1-4, 6-16 |
| X | WO 2021/133887 A1 (CHEVRON U.S.A. INC.) 01 July 2021 (2021-07-01) claim 1, fig. 2 | 1, 4, 9-11 |
| Y | | 2-4, 6-11, 15-16 |
| Y | JP 2009-517448 A (BP CHEMICALS LTD.) 30 April 2009 (2009-04-30) claim 2, paragraphs [0008]-[0011], [0041] | 2-4, 6-16 |

☑ Further documents are listed in the continuation of Box C.     ☑ See patent family annex.

| | |
| --- | --- |
| *      Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **04 September 2023** | **12 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2023/023215** |

**C.** **DOCUMENTS CONSIDERED TO BE RELEVANT**

| Category* | Citation of document, with indication, where appropriate, of the relevant passages | Relevant to claim No. |
| --- | --- | --- |
| Y | JP 2012-201610 A (HIROSHIMA UNIVERSITY) 22 October 2012 (2012-10-22)<br>    claims 1-2, paragraph [0001] | 2-4, 6-16 |
| Y | JP 51-122003 A (MOBIL OIL CORP.) 25 October 1976 (1976-10-25)<br>    claim 1, example 1 | 2-4, 6-16 |
| X<br><br>Y | JP 2010-500362 A (UNIVERSITY OF SOUTHERN CALIFORNIA) 07 January 2010<br>(2010-01-07)<br>    claim 1, paragraphs [0010], [0021] | 5<br><br>2-4, 6-16 |
| A<br><br>Y | JP 2019-506458 A (FUNDACIÓ INSTITUT CATALÀ D'INVESTIGACIÓ QUÍMICA<br>(ICIQ)) 07 March 2019 (2019-03-07)<br>    tables 2-5, paragraph [0003] | 5<br><br>6-16 |
| Y | JP 4-300840 A (MITSUI SEKIYU KAGAKU KOGYO KK) 23 October 1992 (1992-10-23)<br>    claim 1 | 7-16 |
| Y | JP 2-172925 A (MITSUI SEKIYU KAGAKU KOGYO KK) 04 July 1990 (1990-07-04)<br>    claim 1 | 7-16 |
| X<br><br>Y | JP 2020-185802 A (DAINIPPON PRINTING CO., LTD.) 19 November 2020 (2020-11-19)<br>    example 2, paragraphs [0035], [0053] | 15<br><br>15-16 |
| X | JP 2012-514590 A (ARKEMA FRANCE) 28 June 2012 (2012-06-28)<br>    claim 9, paragraph [0045] | 16 |
| A | WO 2014/098229 A1 (AASU RECYCLE KK) 26 June 2014 (2014-06-26)<br>    entire text | 1-16 |
| A | JP 48-36273 A (KOGYO GIJUTSU INCHO) 28 May 1973 (1973-05-28)<br>    entire text | 1-16 |
| A | WO 2021/006245 A1 (SEKISUI CHEMICAL CO., LTD.) 14 January 2021 (2021-01-14)<br>    entire text | 1-16 |

Form PCT/ISA/210 (second sheet) (January 2015)

**INTERNATIONAL SEARCH REPORT**
Information on patent family members

International application No.

**PCT/JP2023/023215**

| Patent document cited in search report | | | Publication date (day/month/year) | Patent family member(s) | | | Publication date (day/month/year) |
|---|---|---|---|---|---|---|---|
| JP | 2002-121318 | A | 23 April 2002 | (Family: none) | | | |
| JP | 2009-240920 | A | 22 October 2009 | (Family: none) | | | |
| WO | 2021/133887 | A1 | 01 July 2021 | JP | 2023-508352 | A | |
| | | | | US | 2021/0189252 | A1 | |
| | | | | EP | 4093838 | A1 | |
| | | | | KR | 10-2022-0117901 | A | |
| | | | | CN | 114901781 | A | |
| JP | 2009-517448 | A | 30 April 2009 | US | 2009/0082605 | A1 | |
| | | | | claim 2, paragraphs [0008]-[0011], [0052] | | | |
| | | | | WO | 2007/063281 | A1 | |
| | | | | EP | 1792885 | A1 | |
| | | | | KR | 10-2008-0071163 | A | |
| | | | | CN | 101336218 | A | |
| JP | 2012-201610 | A | 22 October 2012 | (Family: none) | | | |
| JP | 51-122003 | A | 25 October 1976 | US | 4025575 | A | |
| | | | | claim 1 | | | |
| JP | 2010-500362 | A | 07 January 2010 | US | 2008/0039538 | A1 | |
| | | | | claim 1, paragraphs [0010], [0020] | | | |
| | | | | WO | 2008/021698 | A2 | |
| | | | | EP | 2054364 | A2 | |
| | | | | CN | 101500975 | A | |
| | | | | KR | 10-2009-0057240 | A | |
| JP | 2019-506458 | A | 07 March 2019 | US | 2020/0207689 | A1 | |
| | | | | tables 2-5, paragraph [0003] | | | |
| | | | | WO | 2017/140800 | A1 | |
| | | | | EP | 3208258 | A1 | |
| | | | | CN | 108779051 | A | |
| JP | 4-300840 | A | 23 October 1992 | US | 5475183 | A | |
| | | | | claim 1 | | | |
| | | | | EP | 498573 | A1 | |
| | | | | KR | 10-1992-0016389 | A | |
| | | | | CN | 1063863 | A | |
| JP | 2-172925 | A | 04 July 1990 | US | 5227563 | A | |
| | | | | claim 1 | | | |
| | | | | EP | 379803 | A1 | |
| | | | | KR | 10-1990-0009515 | A | |
| | | | | CN | 1043694 | A | |
| JP | 2020-185802 | A | 19 November 2020 | (Family: none) | | | |
| JP | 2012-514590 | A | 28 June 2012 | US | 2011/0301316 | A1 | |
| | | | | claims 10-11, paragraph [0064] | | | |
| | | | | WO | 2010/079293 | A1 | |
| | | | | EP | 2379485 | A1 | |
| | | | | CN | 102341365 | A | |
| WO | 2014/098229 | A1 | 26 June 2014 | (Family: none) | | | |
| JP | 48-36273 | A | 28 May 1973 | (Family: none) | | | |
| WO | 2021/006245 | A1 | 14 January 2021 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022106346 A
- JP 2022002833 A
- JP 2017170653 A
- US 62369379
- JP 2010194421 A
- JP 2000121 A
- JP 3844734 B
- JP 2014046273 A
- WO 2007083684 A
- WO 2012077723 A
- JP 2012136516 A
- WO 2012077724 A
- JP 2013043794 A
- JP 2010018556 A
- JP 2014055126 A
- WO 2014024774 A
- JP 2022030433 A
- WO 2007117157 A
- WO 2008115080 A
- US 6340581 B2
- US 6136577 B2
- US 5593886 B2
- US 5807722 B2
- US 5821111 B2
- WO 200208438 A
- WO 2009008377 A
- WO 2011034031 A
- WO 2011111638 A
- WO 2012020833 A
- JP 2021185862 A
- WO 2008028055 A
- US 4539293 B2
- US 5753474 B2
- US 1315585 B2
- US 2010330633 A1
- US 8119844 B2
- JP 2012214780 A
- JP 2004292772 A
- JP 2015189973 A
- JP 2017020019 A
- JP 5023596 A
- WO 2019013116 A
- WO 2020196853 A
- WO 2021200689 A
- JP 2011168683 A
- JP 2014108988 A
- WO 2017010323 A
- JP 2021155698 A
- JP 62103072 A
- JP 2010036104 A
- JP 2014198680 A
- WO 2020032279 A
- JP 54016416 A
- JP 2001316308 A
- JP 2000128814 A
- JP 2002201148 A
- JP 8165259 A
- JP 8165260 A
- JP 8291092 A
- WO 2017217279 A
- JP 2002121160 A
- JP 2002128716 A

**Non-patent literature cited in the description**

- *Monthly fine chemicals*, 2017, vol. 46 (12), 44-50
- *Mitsubishi Heavy Industries, Ltd. Technical Method*, 1996, vol. 33 (5)
- *Catalyst*, 2022, 64, 33
- *Catalyst*, 2020, 62, 133
- *Chem. Soc. Rev.*, 2017, vol. 46, 1358
- *Journal of the Japan Petroleum Institute*, 1990, vol. 33, 1
- Carbon Recycling Technology in Catalyst 2021. *CMC Research*, 20 April 2021, 210-214
- **DEMLER, M.** ; **WEUSTER-BOTZ**. Reaction Engineering Analysis of Hydrogenotrophic Production of Acetic Acid by Acetobacterum Woodii. *Biotechnology and Bioengineering*, 02 February 2011, vol. 108
- **LIOU et al.** *International Journal of Systematic and Evolutionary Microbiology*, vol. 33, 2085-2091
- **ABRINI et al.** *Archives of Microbiology*, vol. 161, 345-351
- **SAKAI et al.** *Biotechnology Letters*, vol. 29, 1607-1612
- **SVETLICHNY, V.A.** ; **SOKOLOVA, T.G. et al.** *Systematic and Applied Microbiology*, 1991, vol. 14, 254-260

- **SIMPA**. *Critical Reviews in Biotechnology*, 2006, vol. 26, 41-65
- Handbook Rubber/Plastic Blending Chemicals. Rubber Digest Co., Ltd., 27 April 2001
- Plastic Data book
- Plastics. Industrial Research Committee, 01 December 1999
- Macromolecules. 1973, vol. 6, 925
- Macromolecules. 1975, vol. 8, 687
- New Edition Polypropylene Handbook. Nikkan Kogyo Shimbun, Ltd., 28 September 2012
- New Edition Polypropylene Handbook. Nikkan Kogyo Shimbun, Ltd., 2012
- *Sumitomo Chemical*, 2004, vol. II, 4-14
- *Sumitomo Chemical*, 2006, vol. I, 4-10
- *Sumitomo Chemical*, 2019, 4-11